# EUROPEAN PATENT SPECIFICATION

(11) **EP 1 742 923 B1**
(45) Date of publication and mention of the grant of the patent: **27.05.2009**
(21) Application number: 05735100.9
(22) Date of filing: 20.04.2005
(51) Int. Cl.: C07D 231/12, A61K 31/41, A61P 3/00

(54) **PYRAZOLE PHENYL DERIVATIVES AS PPAR ACTIVATORS**
PYRAZOLPHENYLDERIVATE ALS PPAR-AKTIVATOREN
DERIVES DE PHENYL PYRAZOLE UTILISES EN TANT QU'ACTIVATEURS DES PPAR

(30) Priority: 28.04.2004 EP 04101792
(43) Date of publication of application: 17.01.2007
(73) Proprietor: F.HOFFMANN-LA ROCHE AG, 4070 Basel (CH)
(72) Inventor: ACKERMANN, Jean, CH-4125 Riehen (CH); AEBI, Johannes, CH-4102 Binningen (CH); BINGGELI, Alfred, CH-4102 Binningen (CH); GRETHER, Uwe, 79588 Efringen-Kirchen (DE); HIRTH, Georges, F-68000 Colmar (FR); KUHN, Bernd, CH-4410 Liestal (CH); MAERKI, Hans-Peter, CH-4059 Basel (CH); MEYER, Markus, 79395 Neuenburg (DE); MOHR, Peter, CH-4054 Basel (CH); WRIGHT, Matthew, Blake, CH-4053 Basel (CH)
(74) Representative: Klostermeyer, Dörte
(86) International application number: PCT/EP2005/004199
(87) International publication number: WO 2005/105754

(56) References cited:
- WO-A-01/16120
- SHINKAI H ET AL: "Isoxazolidine-3,5-dione and noncyclic 1,3-dicarbonyl compounds as hypoglycemic agents" JOURNAL OF MEDICINAL CHEMISTRY, AMERICAN CHEMICAL SOCIETY. WASHINGTON, US, vol. 41, no. 11, 21 May 1998 (1998-05-21), pages 1927-1933, XP002155273 ISSN: 0022-2623

## Description

The present invention is concerned with novel phenyl derivatives of the formula 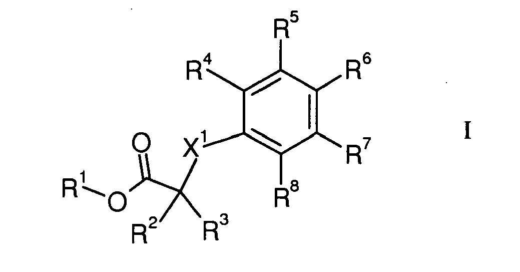 and pharmaceutically acceptable salts thereof, wherein
- X¹: is selected from the group consisting of O, S, and CH₂;
- R¹: is hydrogen or C₁₋₇-alkyl;
- R²: is hydrogen or C₁₋₇-alkyl,
or, if X¹ is CH₂, R² is selected from the group consisting of hydrogen, C₁₋₇-alkyl and C₁₋₇-alkoxy;
- R³: is hydrogen or C₁₋₇-alkyl;
- R⁴ and R⁸: independently from each other are selected from the group consisting of hydrogen, C₁₋₇-alkyl, C₁₋₇-alkoxy, C₃₋₇-cycloalkyl, halogen, . C₁₋₇-alkoxy-C₁₋₇-alkyl, C₂₋₇-alkenyl, C₂₋₇-alkinyl, fluoro-C₁₋₇-alkyl, fluoro-C₁₋₇-alkoxy, cyano-C₁₋₇-alkyl and cyano;
- R⁵, R⁶ and R⁷: independently from each other are selected from the group consisting of hydrogen, C₁₋₇-alkyl, C₁₋₇-alkoxy, C₃₋₇-cycloalkyl, halogen, C₁₋₇-alkoxy-C₁₋₇-alkyl, C₂₋₇-alkenyl, C₂₋₇-alkinyl, fluoro-C₁₋₇-alkyl, fluoro-C₁₋₇-alkoxy, cyano-C₁₋₇-alkyl and cyano;
- and one of R⁵, R⁶ and R⁷: is 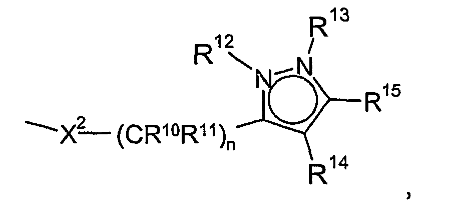 wherein
X² is selected from the group consisting of S, O, and NR⁹,
R⁹ is selected from the group consisting of hydrogen, C₁₋₇-alkyl, C₃₋₇-cycloalkyl, fluoro-C₁₋₇-alkyl, hydroxy-C₂₋₇-alkyl and C₁₋₇-alkoxy-C₂₋₇-alkyl;
R¹⁰ is selected from the group consisting of hydrogen, C₁₋₇-alkyl, C₃₋₇-cycloalkyl and fluoro-C₁₋₇-alkyl;
R¹¹ is selected from the group consisting of hydrogen, C₁₋₇-alkyl and C₁₋₇-alkoxy-C₁₋₇-alkyl;
one of R¹² or R¹³ is selected from the group consisting of hydrogen, C₁₋₇-alkyl, C₃₋₇-cycloalkyl, C₂₋₇-alkoxy-C₁₋₇-alkyl, C₂₋₇-alkenyl, C₂₋₇-alkinyl and fluoro-C₁₋₇-alkyl; and the other one is a lone pair;
R¹⁴ is selected from the group consisting of hydrogen, C₁₋₇-alkyl, C₃₋₇-cycloalkyl, halogen, C₁₋₇-alkoxy-C₁₋₇-alkyl, C₂₋₇-alkenyl, C₂₋₇-alkinyl and fluoro-C₁₋₇-alkyl;
R¹⁵ is 4-trifluoromethoxyphenyl; and
n is 1, 2 or 3.

It has been found that compounds of formula I are useful as lipid modulators and insulin sensitizers. In particular, compounds of formula I are PPAR activators.

Peroxisome Proliferator Activated Receptors (PPARs) are members of the nuclear hormone receptor superfamily. The PPARs are ligand-activated transcription factors that regulate gene expression and control multiple metabolic pathways. Three subtypes have been described which are PPARα, PPARδ (also known as PPARβ, and PPAγ. PPARδ is ubiquitously expressed. PPARα is predominantly expressed in the liver, kidney and heart. There are at least two major isoforms of PPARγ. PPARγ1 is expressed in most tissues, and the longer isoform, PPARγ2 is almost exclusively expressed in adipose tissue. The PPARs modulate a variety of physiological responses including regulation of glucose- and lipid- homeostasis and metabolism, energy balance, cell differentiation, inflammation and cardiovascular events.

Approximately half of all patients with coronary artery disease have low concentrations of plasma HDL cholesterol. The atheroprotective function of HDL was first highlighted almost 25 years ago and stimulated exploration of the genetic and environmental factors that influence HDL levels. The protective function of HDL comes from its role in a process termed reverse cholesterol transport. HDL mediates the removal of cholesterol from cells in peripheral tissues including those in the atherosclerotic lesions of the arterial wall. HDL then delivers its cholesterol to the liver and sterol-metabolizing organs for conversion to bile and elimination. Data from the Framingham study showed that HDL-C levels are predictive of coronary artery disease risk independently of LDL-C levels. The estimated age-adjusted prevalence among. Americans age 20 and older who have HDL-C of less than 35 mg/dl is 16% (males) and 5.7% (females). A substantial increase of HDL-C is currently achieved by treatment with niacin in various formulations. However, the substantial side-effects limit the therapeutic potential of this approach.

As many as 90% of the 14 million diagnosed type 2 diabetic patients in the US are overweight or obese, and a high proportion of type 2 diabetic patients have abnormal concentrations of lipoproteins. The prevalence of total cholesterol > 240 mg/dl is 37% in diabetic men and 44% in women. The respective rates for LDL-C > 160 mg/dl are 31% and 44%, respectively, and for HDL-C < 35 mg/dl 28% and 11%, respectively. Diabetes is a disease in which a patient's ability to control glucose levels in blood is decreased because of partial impairment in response to the action of insulin. Type II diabetes (T2D) is also called non-insulin dependent diabetes mellitus (NIDDM) and afflicts 80-90 % of all diabetic patients in developed countries. In T2D, the pancreatic Islets of Langerhans continue to produce insulin. However, the target organs for insulin action, mainly muscle, liver and adipose tissue, exhibit a profound resistance to insulin stimulation. The body continues to compensate by producing unphysiologically high levels of insulin, which ultimately decreases in later stage of disease, due to exhaustion and failure of pancreatic insulin-producing capacity. Thus T2D is a cardiovascular-metabolic syndrome associated with multiple comorbidities including insulin resistance, dyslipidemia, hypertension, endothelial dysfunction and inflammatory atherosclerosis.

First line treatment for dyslipidemia and diabetes generally involves a low-fat and low-glucose diet, exercise and weight loss. However, compliance can be moderate, and as the disease progresses, treatment of the various metabolic deficiencies becomes necessary with e.g. lipid-modulating agents such as statins and fibrates for dyslipidemia and hypoglycemic drugs, e.g. sulfonylureas or metformin for insulin resistance. A promising new class of drugs has recently been introduced that resensitizes patients to their own insulin (insulin sensitizers), thereby restoring blood glucose and triglyceride levels to normal, and in many cases, obviating or reducing the requirement for exogenous insulin. Pioglitazone (Actos^{™}) and rosiglitazone (Avandia^{™}) belong to the thiazolidinedione (TZD) class of PPARγ-agonists and were the first in their class to be approved for NIDDM in several countries. These compounds, however, suffer from side effects, including rare but severe liver toxicity (as seen with troglitazone). They also increase body weight in patients. Therefore, new, more efficacious drugs with greater safety and lower side effects are urgently needed. Recent studies provide evidence that agonism of PPARδ would result in compounds with enhanced therapeutic potential, i.e. such compounds should improve the lipid profile, with a superior effect on HDL-C raising compared to current treatments and with additional positive effects on normalization of insulin-levels (Oliver et al; Proc Nat Acad Sci USA 2001; 98: 5306-11). Recent observations also suggest that there is a independent PPARα mediated effect on insulin-sensitzation in addition to its well known role in reducing triglycerides (Guerre-Millo et al; J Biol Chem 2000; 275: 16638-16642). Thus selective PPARδ agonists or PPARδ agonists with additional PPARα activity may show superior therapeutic efficacy without the side-effects such as the weight gain seen with PPARγ agonists.

PCT Publ. No. WO 01/16120 discloses biaryl-oxy(thia)zole derivatives as PPAR modulators.

The novel compounds of the present invention exceed the compounds known in the art, inasmuch as they bind to and selectively activate PPARδ or coactivate PPARδ and PPARα simultaneously and very efficiently, and with much improved pharmacokinetic properties. Therefore, these compounds combine the anti-dyslipidemic and antiglycemic effects of PPARδ and PPARα activation with no effect on PPARγ. Consequently, HDL cholesterol is increased, triglycerides lowered (=improved lipid profile) and plasma glucose and insulin are reduced (=insulin sensitization). In addition, such compounds may also lower LDL cholesterol, decrease blood pressure and counteract inflammatory atherosclerosis. Furthermore, such compounds may also be useful for treating inflammatory diseases such as rheumatoid arthritis, osteoarthritis, and psoriasis. Since multiple facets of combined dyslipidemia and the T2D disease syndrome are addressed by PPARδ-selective agonists and PPARδ and α coagonists, they are expected to have an enhanced therapeutic potential compared to the compounds already known in the art.

The compounds of the present invention further exhibit improved pharmacological properties compared to known compounds.

Unless otherwise indicated the following definitions are set forth to illustrate and define the meaning and scope of the various terms used to describe the invention herein.

The term "alkyl", alone or in combination with other groups, refers to a branched or straight-chain monovalent saturated aliphatic hydrocarbon radical of one to twenty carbon atoms, preferably one to sixteen carbon atoms, more preferably one to ten carbon atoms.

The term "lower alkyl" or "C₁₋₇-alkyl", alone or in combination with other groups, refers to a branched or straight-chain monovalent alkyl radical of one to seven carbon atoms, preferably one to four carbon atoms. This term is further exemplified by such radicals as methyl, ethyl, n-propyl, isopropyl, n-butyl, s-butyl, t-butyl and the groups specifically exemplified herein.

The term "halogen" refers to fluorine, chlorine, bromine and iodine.

The term "fluoro-lower alkyl" or "fluoro-C₁₋₇-alkyl" refers to to lower alkyl groups which are mono- or multiply substituted with fluorine. Examples of fluoro-lower alkyl groups are e.g. -CF₃, -CH₂CF₃, -CH(CF₃)₂ and the groups specifically exemplified herein.

The term "alkoxy" refers to the group R'-O-, wherein R' is alkyl. The term "lower-alkoxy" or "C₁₋₇-alkoxy" refers to the group R'-O-, wherein R' is lower-alkyl. Examples of lower-alkoxy groups are e.g. methoxy, ethoxy, propoxy, isopropoxy, butoxy, isobutoxy and hexyloxy. Preferred are the lower-alkoxy groups specifically exemplified herein.

The term "lower fluoroalkoxy" or "fluoro-C₁₋₇-alkoxy" refers to lower alkoxy groups as defined above which are mono- or multiply substituted with fluorine. Examples of lower fluoroalkoxy groups are e.g. -OCF₃, and -OCH₂CF₃.

The term "lower alkenyl" or "C₂₋₇-alkenyl", alone or in combination, signifies a straight-chain or branched hydrocarbon residue comprising an olefinic bond and up to 7, preferably up to 6, particularly preferred up to 4 carbon atoms. Examples of alkenyl groups are ethenyl, 1-propenyl, 2-propenyl, isopropenyl, 1-butenyl, 2-butenyl, 3-butenyl and isobutenyl. A preferred example is 2-propenyl.

The term "lower alkinyl" or "C₂₋₇-alkinyl", alone or in combination, signifies a straight-chain or branched hydrocarbon residue comprising a triple bond and up to 7, preferably up to 6, particularly preferred up to 4 carbon atoms. Examples of alkinyl groups are ethinyl, 1-propinyl, or 2-propinyl.

The term "cycloalkyl" or "C₃₋₇-cycloalkyl" denotes a saturated carbocyclic group containing from 3 to 7 carbon atoms, such as cyclopropyl, cyclobutyl, cyclopentyl, cyclohexyl or cycloheptyl.

A "lone pair" is a pair of electrons in the outermost shell of an atom, in particular a nitrogen atom, that are not used in bonding.

The term "protecting group" refers to groups such as e.g. acyl, alkoxycarbonyl, aryloxycarbonyl, silyl, or imine-derivatives, which are used to temporarily block the reactivity of functional groups. Well known protecting groups are e.g. t-butyloxycarbonyl, benzyloxycarbonyl, fluorenylmethyloxycarbonyl or diphenylmethylene which can be used for the protection of amino groups, or lower-alkyl-, β-trimethylsilylethyl- and β-trichloroethyl-esters, which can be used for the protection of carboxy groups.

"Isomers" are compounds that have identical molecular formulae but that differ in the nature or the sequence of bonding of their atoms or in the arrangement of their atoms in space. Isomers that differ in the arrangement of their atoms in space are termed "stereoisomers". Stereoisomers that are not mirror images of one another are termed "diastereoisomers", and stereoisomers that are non-superimposable mirror images are termed "enantiomers"; or sometimes optical isomers. A carbon atom bonded to four nonidentical substituents is termed a "chiral center".

The term "pharmaceutically acceptable salts" embraces salts of the compounds of formula (I) with pharmaceutically acceptable bases such as alkali salts, e.g. Na- and K-salts, alkaline earth salts, e.g. Ca- and Mg-salts, and ammonium or substituted ammonium salts, such as e.g. trimethylammonium salts. The term "pharmaceutically acceptable salts" also relates to such salts.

The compounds of formula (I) can also be solvated, e.g. hydrated. The solvation can be effected in the course of the manufacturing process or can take place e.g. as a consequence of hygroscopic properties of an initially anhydrous compound of formula (I) (hydration). The term pharmaceutically acceptable salts also includes pharmaceutically acceptable solvates.

In detail, the present invention relates to compounds of formula (I) 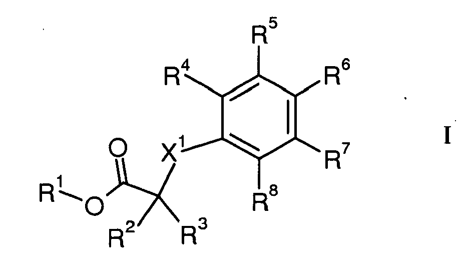 wherein
- X¹: is selected from the group consisting of O, S and CH₂;
- R¹: is hydrogen or C₁₋₇-alkyl;
- R²: is hydrogen or C₁₋₇-alkyl,
or, if X¹ is CH₂, R² is selected from the group consisting of hydrogen, C₁₋₇-alkyl and C₁₋₇-alkoxy;
- R³: is hydrogen or C₁₋₇-alkyl;
- R⁴ and R⁸: independently from each other are selected from the group consisting of hydrogen, C₁₋₇-alkyl, C₁₋₇-alkoxy, C₃₋₇-cycloalkyl, halogen, C₁₋₇-alkoxy- C₁₋₇-alkyl, C₂₋₇-alkenyl, C₂₋₇-alkinyl, fluoro-C₁₋₇-alkyl, fluoro-C₁₋₇-alkoxy, cyano-C₁₋₇-alkyl and cyano;
- R⁵, R⁶ and R⁷: independently from each other are selected from the group consisting of hydrogen, C₁₋₇-alkyl, C₁₋₇-alkoxy, C₃₋₇-cycloalkyl, halogen, C₁₋₇-alkoxy- C₁₋₇-alkyl, C₂₋₇-alkenyl, C₂₋₇-alkinyl, fluoro-C₁₋₇-alkyl, fluoro-C₁₋₇-alkoxy, cyano-C₁₋₇-alkyl and cyano;
- and one of R⁵, R⁶ and R⁷: is 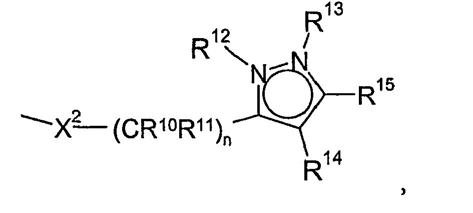 wherein
X² is selected from the group consisting of S, O and NR⁹;
R⁹ is selected from the group consisting of hydrogen, C₁₋₇-alkyl, C₃₋₇-cycloalkyl, fluoro-C₁₋₇-alkyl, hydroxy-C₂₋₇-alkyl and C₁₋₇-alkoxy-C₂₋₇-alkyl;
R¹⁰ is selected from the group consisting of hydrogen, C₁₋₇-alkyl, C₃₋₇-cycloalkyl and fluoro-C₁₋₇-alkyl;
R¹¹ is selected from the group consisting of hydrogen, C₁₋₇-alkyl and C₁₋₇-alkoxy-C₁₋₇-alkyl;
one of R¹² or R¹³ is selected from the group consisting of hydrogen, C₁₋₇-alkyl, C₃₋₇-cycloalkyl, C₂₋₇-alkoxy-C₁₋₇-alkyl, C₂₋₇-alkenyl, C₂₋₇-alkinyl and fluoro-C₁₋₇-alkyl; and the other one is a lone pair;
R¹⁴ is selected from the group consisting of hydrogen, C₁₋₇-alkyl, C₃₋₇-cycloalkyl, halogen, C₁₋₇-alkoxy-C₁₋₇-alkyl, C₂₋₇-alkenyl, C₂₋₇-alkinyl and fluoro-C₁₋₇-alkyl;
R¹⁵ is 4-trifluoromethoxyphenyl;
n is 1, 2 or 3; and
pharmaceutically acceptable salts thereof.

Preferred compounds of formula I of the present invention are compounds of formula 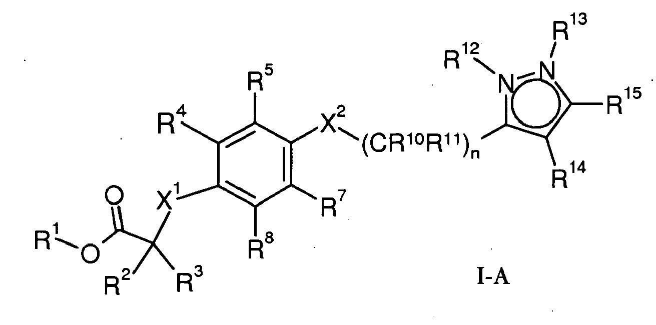 wherein
X¹, X², R¹ to R⁴, R⁸, R¹⁰ to R¹⁵ and n are as defined herein before;
R⁵ and R⁷ independently from each other are selected from the group consisting of hydrogen, C₁₋₇-alkyl, C₁₋₇-alkoxy, C₃₋₇-cycloalkyl, halogen, C₁₋₇-alkoxy- C₁₋₇-alkyl, C₂₋₇-alkenyl, C₂₋₇-alkinyl, fluoro-C₁₋₇-alkyl, fluoro-C₁₋₇-alkoxy, cyano-C₁₋₇-alkyl and cyano; and
pharmaceutically acceptable salts thereof.

More preferred are those compounds of formula I-A in accordance with the present invention, wherein at least one of R⁴, R⁵, R⁷ and R⁸ is C₁₋₇-alkyl or C₁₋₇-alkoxy, with those compounds of formula I-A wherein R⁴ is C₁₋₇-alkyl or C₁₋₇-alkoxy being especially preferred. Even more preferred are those compounds of formula I-A, wherein one of R⁴ and R⁸ is methyl and the other one hydrogen.

Also preferred are compounds of formula I having the formula 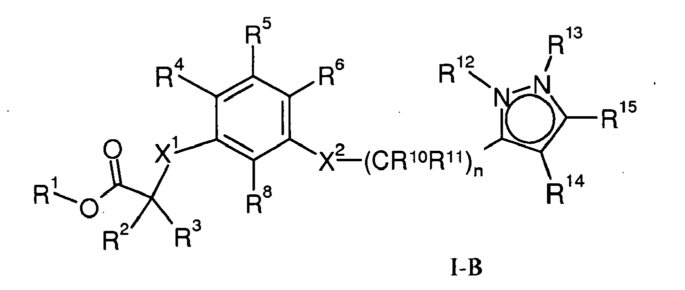 wherein
X¹, X², R¹ to R⁴, R⁸, R¹⁰ to R¹⁵ and n are as defined herein before;
R⁵ and R⁶ independently from each other are selected from the group consisting of hydrogen, C₁₋₇-alkyl, C₁₋₇-alkoxy, C₃₋₇-cycloalkyl, halogen, C₁₋₇-alkoxy- C₁₋₇-alkyl, C₂₋₇-alkenyl, C₂₋₇-alkinyl, fluoro-C₁₋₇-alkyl, fluoro-C₁₋₇-alkoxy, cyano-C₁₋₇-alkyl and cyano; and
pharmaceutically acceptable salts thereof.

More preferred are those compounds of formula I-B in accordance with the present invention, wherein at least one of R⁴, R⁵, R⁶ and R⁸ is C₁₋₇-alkyl or C₁₋₇-alkoxy, with those compounds of formula I-B wherein R⁴ is C₁₋₇-alkyl or C₁₋₇-alkoxy being especially preferred. Even more preferred are those compounds of formula I-B, wherein one of R⁴ and R⁸ is methyl and the other one hydrogen.

Further preferred compounds of formula I have the formula 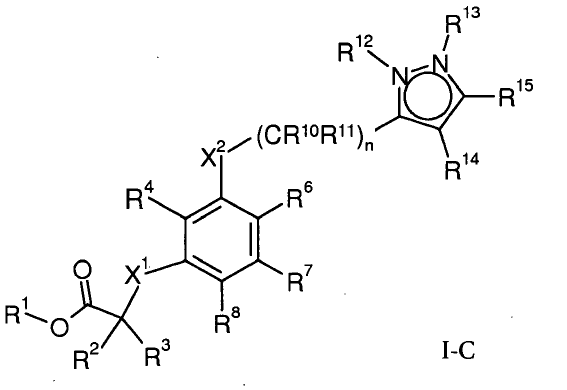 wherein
X¹ X², R¹ to R⁴, R⁸, R¹⁰ to R¹⁵ and n are as defined herein before;
R⁶ and R⁷ independently from each other are selected from the group consisting of hydrogen, C₁₋₇-alkyl, C₁₋₇-alkoxy, C₃₋₇-cycloalkyl, halogen, C₁₋₇-alkoxy- C₁₋₇-alkyl, C₂₋₇-alkenyl, C₂₋₇-alkinyl, fluoro-C₁₋₇-alkyl, fluoro-C₁₋₇-alkoxy, cyano-C₁₋₇-alkyl and cyano; and
pharmaceutically acceptable salts thereof.

More preferred are those compounds of formula I-C in accordance with the present invention, wherein at least one of R⁴, R⁶, R⁷ and R⁸ is C₁₋₇-alkyl or C₁₋₇-alkoxy, with those compounds of formula I-C wherein R⁴ is C₁₋₇-alkyl or C₁₋₇-alkoxy being especially preferred. Even more preferred are those compounds of formula I-C, wherein one of R⁴ and R⁸ is methyl and the other one hydrogen.

Preferred compounds of formula I are those, wherein R¹ is hydrogen.

X¹ is from the group consisting of O, S and CH₂. Compounds of formula I, wherein X¹ is O are preferred. More preferred are those compounds of formula I, wherein X¹ is O and at least one of R² and R³ is C₁₋₇-alkyl with those compounds of formula I wherein X¹ is O and R² and R³ are C₁₋₇-alkyl being especially preferred.

Also preferred are compounds of formula I, wherein X¹ is CH₂.

X² is selected from the group consisting of S, O and NR⁹. Preferred are compounds of formula I, wherein X² is O.

R¹⁰ is selected from hydrogen, C₁₋₇-alkyl, C₃₋₇-cycloalkyl or fluoro-C₁₋₇-alkyl and R¹¹ is selected from hydrogen, C₁₋₇-alkyl or C₁₋₇-alkoxy-C₁₋₇-alkyl. Preferred are compounds of formula I, wherein R¹⁰ and R¹¹ are hydrogen.

The integer n is 1, 2 or 3. Preferred are compounds of formula I, wherein n is 1. Further preferred are compounds of formula I, wherein n is 2.

Furthermore, compounds of formula I are also preferred, wherein n is 3.

Further preferred compounds are those compounds of formula I, wherein one of R⁵, R⁶ and R⁷ is 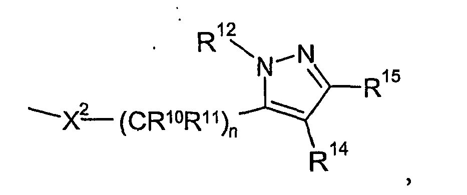 and X², R¹⁰ to R¹², R¹⁴, R¹⁵ and n are as defined herein before.

Especially preferred are those compounds, wherein R¹² is C₁₋₇-alkyl or fluoro-C₁₋₇-alkyle.

Also especially preferred are compounds of formula I, wherein one of R⁵, R⁶ and R⁷ is 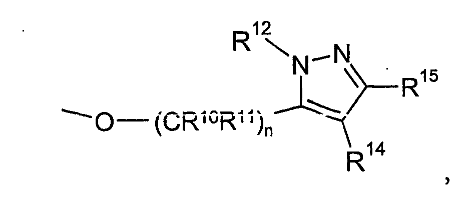 and R¹⁰ to R¹², R¹⁴ , R¹⁵ and n are as defined herein above.

Also preferred are compounds of formula I, wherein one of R⁵, R⁶ and R⁷ is 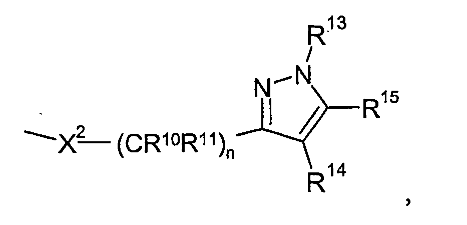 and X², R¹⁰, R¹¹, R¹³ to R¹⁵ and n are as defined herein before.

Examples of preferred compounds of formula I are the following:
2-methyl-2-{2-methyl-4-[2-methyl-5-(4-trifluoromethoxy-phenyl)-2H-pyrazol-3-ylmethoxy]-phenoxy}-propionic acid,
2-methyl-2-(2-methyl-4-{2-[2-methyl-5-(4-trifluoromethoxy-phenyl)-2H-pyrazol-3-yl]-ethoxy}-phenoxy)-propionic acid,
2-methyl-2-(3-{2-[2-methyl-5-(4-trifluoromethoxy-phenyl)-2H-pyrazol-3-yl]-ethoxy}phenoxy)-propionic acid,
3-{2-methoxy-4-[2-methyl-5-(4-trifluoromethoxy-phenyl)-2H-pyrazol-3-ylmethoxy] - phenyl}-propionic acid,
2-{2,3-dimethyl-4-[2-methyl-5-(4-trifluoromethoxy-phenyl)-2H-pyrazol-3-ylmethoxy]-phenoxy}-2-methyl-propionic acid,
2-methyl-2-{2-methyl-4-[2-(2,2,2-trifluoro-ethyl)-5-(4-trifluoromethoxy-phenyl)-2H-pyrazol-3-ylmethoxy] -phenoxy}-propionic acid,
2-methyl-2-{2-methyl-4-[1-methyl-5-(4-trifluoromethoxy-phenyl)-1H-pyrazol-3-ylmethoxy]-phenoxy}-propionic acid,
2-{2,5-dichloro-4-[2-methyl-5-(4-trifluoromethoxy-phenyl)-2H-pyrazol-3-ylmethoxy]-phenoxy}-2-methyl-propionic acid,
2-{4- [1-difluoromethyl-5-(4-trifluoromethoxy-phenyl)-1H-pyrazol-3-ylmethoxy] -2-methyl-phenoxy}-2-methyl-propionic acid,
2-methyl-2-(2-methyl-4-{3-[2-methyl-5-(4-trifluoromethoxy-phenyl)-2H-pyrazol-3-yl]-propoxy}-phenoxy)-propionic acid,
2-(2,5-dichloro-4-{2-[2-methyl-5-(4-trifluoromethoxy-phenyl)-2H-pyrazol-3-yl]-ethoxy}-phenoxy)-2-methyl-propionic acid,
2-methyl-2-(2-methyl-4-{2-[2-(2,2,2-trifluoro-ethyl)-5-(4-trifluoromethoxy-phenyl)-2H-pyrazol-3-yl]-ethoxy}-phenoxy)-propionic acid,
2-{5-methoxy-2-methyl-4-[2-methyl-5-(4-trifluoromethoxy-phenyl)-2H-pyrazol-3-ylmethoxy]-phenoxy}-2-methyl-propionic acid,
2-(5-methoxy-2-methyl-4-{3-[2-methyl-5-(4-trifluoromethoxy-phenyl)-2H-pyrazol-3-yl]-propoxy}-phenoxy)-2-methyl-propionic acid, and
2-{4-[2-difluoromethyl-5-(4-trifluoromethoxy-phenyl)-2H-pyrazol-3-ylmethoxy]-2-methyl-phenoxy}-2-methyl-propionic acid.

Particularly preferred compounds of formula I of the present invention are the following:
2-methyl-2-{2-methyl-4- [2-methyl-5-(4-trifluoromethoxy-phenyl)-2H-pyrazol-3-ylmethoxy]-phenoxy}-propionic acid,
2-methyl-2-(2-methyl-4-{2-[2-methyl-5-(4-trifluoromethoxy-phenyl)-2H-pyrazol-3-yl]-ethoxy}-phenoxy)-propionic acid,
2-methyl-2-(3-{2- [2-methyl-5-(4-trifluoromethoxy-phenyl)-2H-pyrazol-3-yl] -ethoxy}phenoxy)-propionic acid,
2-{2,3-dimethyl-4-[2-methyl-5-(4-trifluoromethoxy-phenyl)-2H-pyrazol-3-ylmethoxy]-phenoxy}-2-methyl-propionic acid,
2-methyl-2-(2-methyl-4-{3-[2-methyl-5-(4-trifluoromethoxy-phenyl)-2H-pyrazol-3-yl]-propoxy}-phenoxy)-propionic acid,
2-(2,5-dichloro-4-{2-[2-methyl-5-(4-trifluoromethoxy-phenyl)-2H-pyrazol-3-yl]-ethoxy}-phenoxy)-2-methyl-propionic acid,
2-methyl-2-(2-methyl-4-{2-[2-(2,2,2-trifluoro-ethyl)-5-(4-trifluoromethoxy-phenyl)-2H-pyrazol-3-yl]-ethoxy}-phenoxy)-propionic acid,
2-(5-methoxy-2-methyl-4-{3-[2-methyl-5-(4-trifluoromethoxy-phenyl)-2H-pyrazol-3-yl]-propoxy}-phenoxy)-2-methyl-propionic acid, and
2-{4-[2-difluoromethyl-5-(4-trifluoromethoxy-phenyl)-2H-pyrazol-3-ylmethoxy]-2-methyl-phenoxy}-2-methyl-propionic acid.

Especially preferred are also the following compounds of formula I of the present invention:
2-methyl-2-{2-methyl-4-[2-methyl-5-(4-trifluoromethoxy-phenyl)-2H-pyrazol-3-ylmethoxy]-phenoxy}-propionic acid,
2-methyl-2-(2-methyl-4-{2-[2-methyl-5-(4-trifluoromethoxy-phenyl)-2H-pyrazol-3-yl]-ethoxy}-phenoxy)-propionic acid,
2-methyl-2-(2-methyl-4-{3-[2-methyl-5-(4-trifluoromethoxy-phenyl)-2H-pyrazol-3-yl]-propoxy}-phenoxy)-propionic acid, and
2-(5-methoxy-2-methyl-4-{3- [2-methyl-5-(4-trifluoromethoxy-phenyl)-2H-pyrazol-3-yl]-propoxy}-phenoxy)-2-methyl-propionic acid.

Furthermore, the pharmaceutically acceptable salts of the compounds of formula I individually constitute preferred embodiments of the present invention.

Compounds of formula I can have one or more asymmetric carbon atoms and can exist in the form of optically pure enantiomers, mixtures of enantiomers such as, for example, racemates, optically pure diastereoisomers, mixtures of diastereoisomers, diastereoisomeric racemates or mixtures of diastereoisomeric racemates. The optically active forms can be obtained for example by resolution of the racemates, by asymmetric synthesis or asymmetric chromatography (chromatography with a chiral adsorbens or eluant). The invention embraces all of these forms.

Compounds of formula I may also contain C₁₋₇-alkenyl groups. All forms of cis- and trans-isomers are embraced by the present invention.

It will be appreciated, that the compounds of general formula I in this invention may be derivatised at functional groups to provide derivatives which are capable of conversion back to the parent compound in vivo.

A further aspect of the present invention is the process for the manufacture of compounds of formula (I) as defined above, which process comprises
reacting a compound of formula 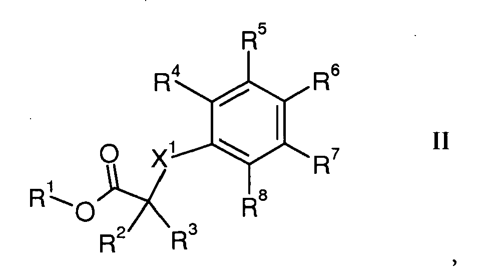 wherein R¹ is C₁₋₇-alkyl, R², R³, R⁴ and R⁸ are as defined as above and R⁵, R⁶ and R⁷ are selected from hydrogen, C₁₋₇-alkyl, C₁₋₇-alkoxy, C₃₋₇-cycloalkyl, halogen, C₁₋₇-alkoxy-C₁₋₇-alkyl, C₂₋₇-alkenyl, C₂₋₇-alkinyl, fluoro-C₁₋₇-alkyl, fluoro-C₁₋₇-alkoxy, cyano-C₁₋₇-alkyl, and cyano with the proviso that one of R⁵, R⁶ or R⁷ is -OH, -SH or -NHR⁹, wherein R⁹ is as defined above,
with a compound of formula 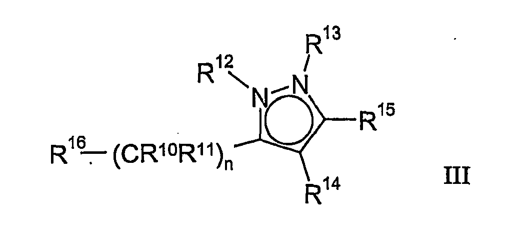 wherein R¹⁰ to R¹⁵ and n are as defined as above and R¹⁶ is -OH, -Cl, -Br, -I or another leaving group, to obtain a compound of formula 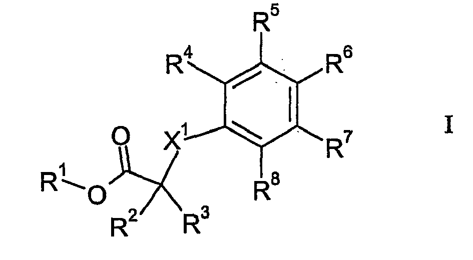 wherein R¹ is C₁₋₇-alkyl, X¹ and R² to R⁸ are as defined as above,
and optionally hydrolysing the ester group to obtain a compound of formula I, wherein R¹ is hydrogen.

As described above, the compounds of formula (I) of the present invention can be used as medicaments for the treatment and/or prevention of diseases which are modulated by PPARδ and/or PPARα agonists. Examples of such diseases are diabetes, particularly non-insulin dependent diabetes mellitus, increased lipid and cholesterol levels, particularly low HDL-cholesterol, high LDL-cholesterol, or high triglyceride levels, atherosclerotic diseases, metabolic syndrome, syndrome X, elevated blood pressure, endothelial dysfunction, procoagulant state, dyslipidemia, polycystic ovary syndrome, inflammatory diseases (such as e.g. Crohn's disease, inflammatory bowel disease, colitis, pancreatitis, cholestasis/fibrosis of the liver, rheumatoid arthritis, osteoarthritis, psoriasis and other skin disorders, and diseases that have an inflammatory component such as e.g. Alzheimer's disease or impaired/improvable cognitive function) and proliferative diseases (cancers such as e.g. liposarcoma, colon cancer, prostate cancer, pancreatic cancer and breast cancer). The use as medicament for the treatment of low HDL cholesterol levels, high LDL cholesterol levels, high triglyceride levels, metabolic syndrome and syndrome X is preferred.

The invention therefore also relates to pharmaceutical compositions comprising a compound as defined above and a pharmaceutically acceptable carrier and/or adjuvant.

Further, the invention relates to compounds as defined above for use as therapeutically active substances, particularly as therapeutic active substances for the treatment and/or prevention of diseases which are modulated by PPARδ and/or PPARα agonists. Examples of such diseases are diabetes, particularly non-insulin dependent diabetes mellitus, increased lipid and cholesterol levels, particularly low HDL-cholesterol, high LDL-cholesterol, or high triglyceride levels, atherosclerotic diseases, metabolic syndrome, syndrome X, elevated blood pressure, endothelial dysfunction, procoagulant state, dyslipidemia, polycystic ovary syndrome, inflammatory diseases such as rheumatoid arthritis, osteoarthritis, psoriasis and other skin disorder, and proliferative diseases.

In addition, the invention relates to the use of compounds as defined above for the preparation of medicaments for the treatment and/or prevention of diseases which are modulated by PPARδ and/or PPARα agonists. Preferred examples of such diseases are diabetes, particularly non-insulin dependent diabetes mellitus, increased lipid and cholesterol levels, particularly low HDL-cholesterol, high LDL-cholesterol, or high triglyceride levels, atherosclerotic diseases, metabolic syndrome, syndrome X, elevated blood pressure, endothelial dysfunction, procoagulant state, dyslipidemia, polycystic ovary syndrome, inflammatory diseases such as rheumatoid arthritis, osteoarthritis, psoriasis and other skin disorder, and proliferative diseases. Such medicaments comprise a compound as defined above.

The compounds of formula (I) can be manufactured by the methods given below, by the methods given in the examples or by analogous methods. Appropriate reaction conditions for the individual reaction steps are known to a person skilled in the art. Starting materials are either commercially available or can be prepared by methods analogous to the methods given below, by methods described in references cited in the text or in the examples, or by methods known in the art.

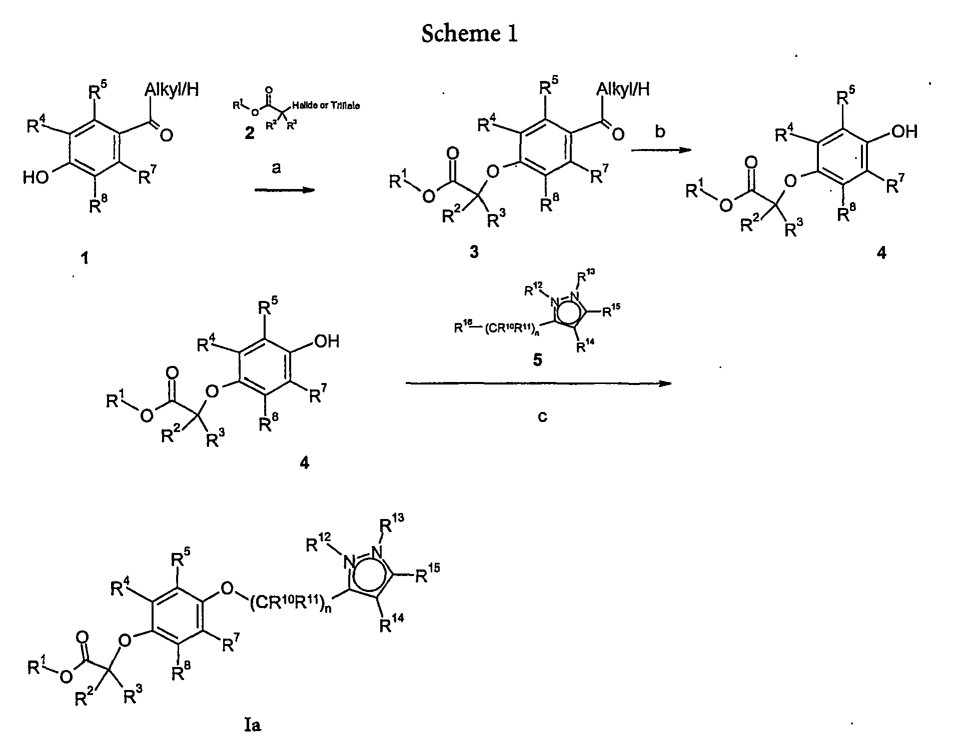

Hydroxy aldehydes or hydroxy aryl alkyl ketones 1 are known or can be prepared by methods known in the art. Reaction of phenols 1 with alpha halo esters of formula 2 in the presence of a base like potassium or cesium carbonate in solvents like acetone, methyl-ethyl ketone, acetonitrile or N,N-dimethylformamide in a temperature range between room temperature and 140°C leads to the corresponding ether compounds 3 (steps a). *Baeyer* Villiger oxidation e. g. with meta chloro perbenzoic acid in a solvent like dichloromethane, leads to compounds 4 (step b). Pyrazoles 5 (prepared as outlined in schemes 6 to 9) are condensed with phenols 4 according to well known procedures (step c): if R¹⁶ represents a hydroxy group e.g. via *Mitsunobu-reaction,* with triphenylphosphine and di-tert-butyl-, diisopropyl- or diethyl-azodicarboxylate as reagents; this transformation is preferably carried out in a solvent like toluene, dichloromethane or tetrahydrofuran at ambient temperature. Alternatively, if R¹⁶ represents a halide; mesylate, tosylate or triflate moiety, pyrazoles 5 can be reacted with phenols 4 in solvents like N,N-dimethylformamide, dimethylsulfoxide, acetonitrile, acetone or methyl-ethyl ketone in the presence of a weak base like cesium or potassium carbonate at a temperature ranging from room temperature to 140 °C, preferably around 50 °C to yield ether compounds Ia (step c). Those can optionally be hydrolyzed according to standard procedures, e. g. by treatment with an alkali hydroxide like LiOH or NaOH in a polar solvent mixture like tetrahydrofuran/ethanol/water leading to carboxylic acids Ia.

An analogous reaction scheme with the same reaction sequences applies for the isomeric compound series leading to compounds of general formula I, particularly compounds according to formula Ib:

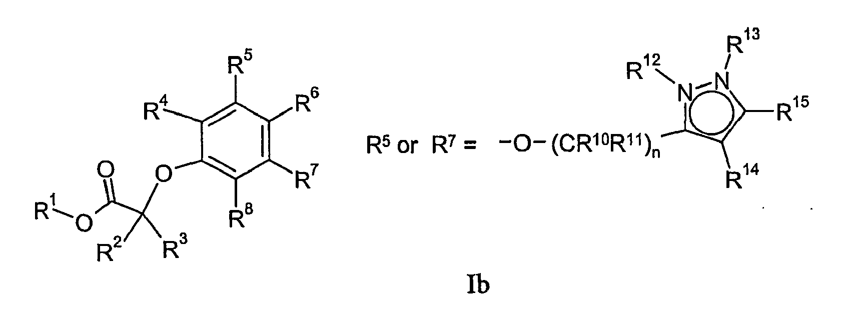

The synthesis of compounds with the general structure I, particularly compounds according to formula Ic, with X¹ equal to O and X² equal to nitrogen can be accomplished according to schemes 2 and 3.

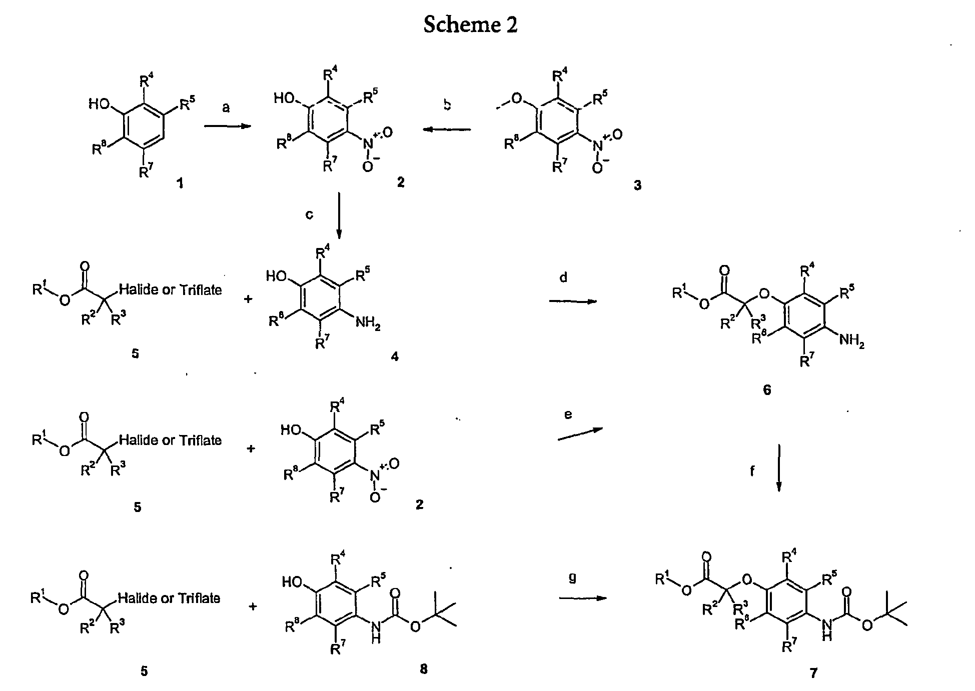

Nitro-phenols 2 of scheme 2 are commercially available, known or can be synthesized from anisols 3 by demethylation with aqueous 62% HBr in acetic acid between RT and 120 °C (step b). Alternatively, phenols 1 can be nitrated in para-position according to well established methods, e. g. with a solution of NaNO₃ in water/concentrated hydrochloric acid in a solvent like Et₂O, followed by the addition of acetic acid anhydride at RT [following a procedure of P. Keller, Bull. Soc. Fr. 1994,131, 27-29] leading to phenols 2 (step a). Nitro-phenols 2 are then reduced in an alcohol like EtOH or MeOH with hydrogen in the presence of Pd/C and optionally an acid like HCI or AcOH at RT to give anilines 4 (step c). Intermediates 4 are then O-alkylated with electrophile 5, e.g. a bromo-acetate 5, in the presence of K₂CO₃ or Cs₂CO₃ in a solvent like acetonitrile or acetone between 10 °C and RT to give intermediates 6 (step d). Electrophiles 5 are commercially available or can be synthesized by methods known in the art. Triflates 5 can be prepared from the corresponding alcohols. Anilines 6 can alternatively be synthesized from compounds 5 and nitrophenols 2 in a two step procedure: first by O-alkylation as described above, followed by hydrogenation with Pd/C in an alcohol like MeOH or EtOH optionally in the presence of AcOH or HCI (step e). BOC-protection with di-tert-butyl dicarbonate in tetrahydrofuran at RT to reflux yields compounds 7 (step f). Compounds 7 can also be synthesized directly from electrophiles 5 and BOC-protected anilines 8 with K₂CO₃ or Cs₂CO₃ as described for the synthesis of compounds 6 (step g).

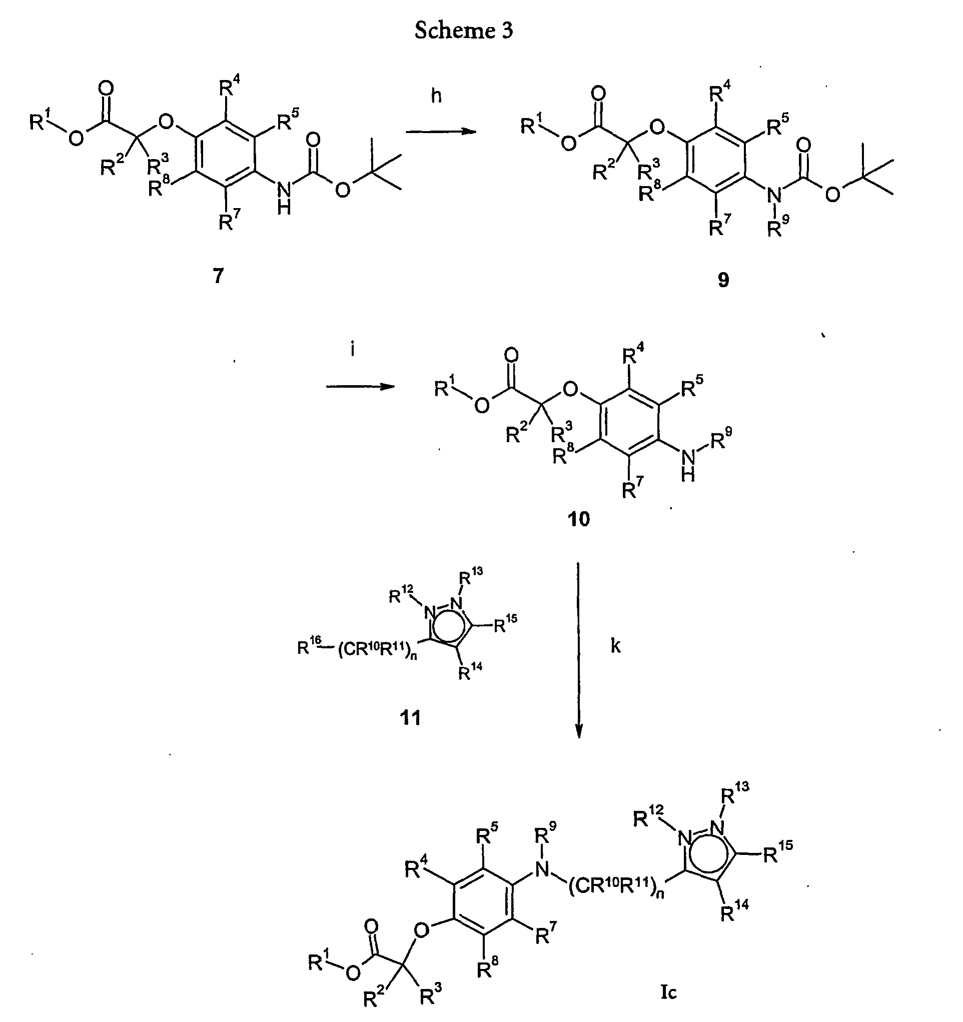

Intermediates 7 of scheme 3 can optionally be alkylated at nitrogen using sodium hydride and a reactive alkyl halogenide/mesylate or triflate to give compounds 9 (step h). Standard BOC-deprotection (TFA/CH₂Cl₂, or HCl in dioxane) at 0 °C to RT affords anilines 10 (step i). Reaction with activated pyrazoles 11 (R¹⁶ being a halide or a methanesulfonate) using sodium hydride or sodium, potassium or cesium carbonate in N,N-dimethylformamide, dimethylsulfoxide, dimethylacetamide or tetrahydrofuran, at 0 °C to RT, leads to compounds Ic (step k). Alternatively, pyrazoles 11 with R¹⁶=OH can be transformed in situ to the corresponding triflates by treatment with trifluoromethanesulfonic anhydride/2,6-di-tert-butylpyridine in CH₂Cl₂ at 0 °C. These triflates are then reacted with anilines 10 in the presence of 2,6-di-tert-butylpyridine as base in nitromethane between RT and 60 °C to yield compounds Ic [following a procedure of Belostotskii, anatomy M., Hassner, A., Tetrahedron Lett. 1994, 35(28), 5075-6] (step k). Secondary aniline compounds Ic (R⁹=H) can be reductively methylated with an aqueous solution of NaH₂PO₃ and formaldehyde between RT and 65 °C [Loibner, H., Pruckner, A., Stuetz, A., Tetrahedron Lett. 1984, 25, 2535-2536] to give compounds Ic with R⁹=Me. Ensuing hydrolysis with aqueous LiOH, NaOH or KOH in tetrahydrofuran/EtOH or another suitable solvent produces compounds Ic of scheme 3 in the form of the free acid.

An analogous reaction scheme with the same reaction sequences applies for the isomeric compound series leading to compounds of general formula I, particularly compounds according to formula Id:

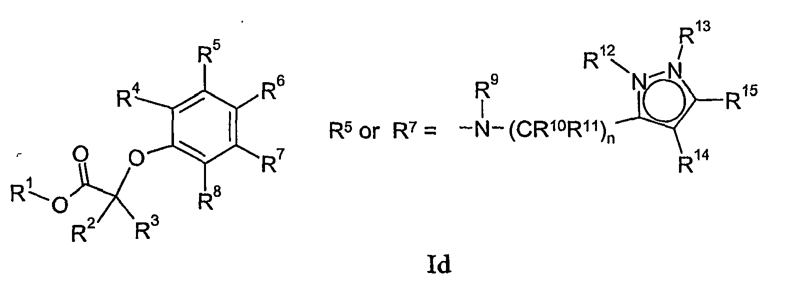

As alternative to the sequences described in scheme 2, the nitrogen containing intermediates can be prepared from suitable intermediates carrying a phenolic hydroxyl moiety. In such intermediates, optionally carrying one or more protective functions, the phenolic OH group can be replaced by the corresponding aromatic NH₂ function by methods known in the art. For example by a three step sequence as described in Tetrahedron Letters 43(42), 7617-7619 (2002): i) transformation of the phenol moiety into its trifluoromethanesulfonate (triflic anhydride, 2,6-lutidine, 4-dimethylaminopyridine, dichloromethane, 0 °C to room temperature; ii) treatment of the triflate with benzophenone imine, di-palladium-tris(dibenzylideneacetone) complex, S-(-)-2,2'-bis(diphenylphosphino)-1,1'-binaphthyl, cesium carbonate, toluene, in a Schlenk tube at temperatures around 120 °C; iii) treatment with catalytic amounts of hydrochloric acid in wet tetrahydrofuran preferably at room temperature to liberate the aromatic NH₂ moiety.

The synthesis of compounds with the general structure I, particularly compounds according to formula Ie, with X¹ equal to CH₂ and X² equal to oxygen can be accomplished according to scheme 4.

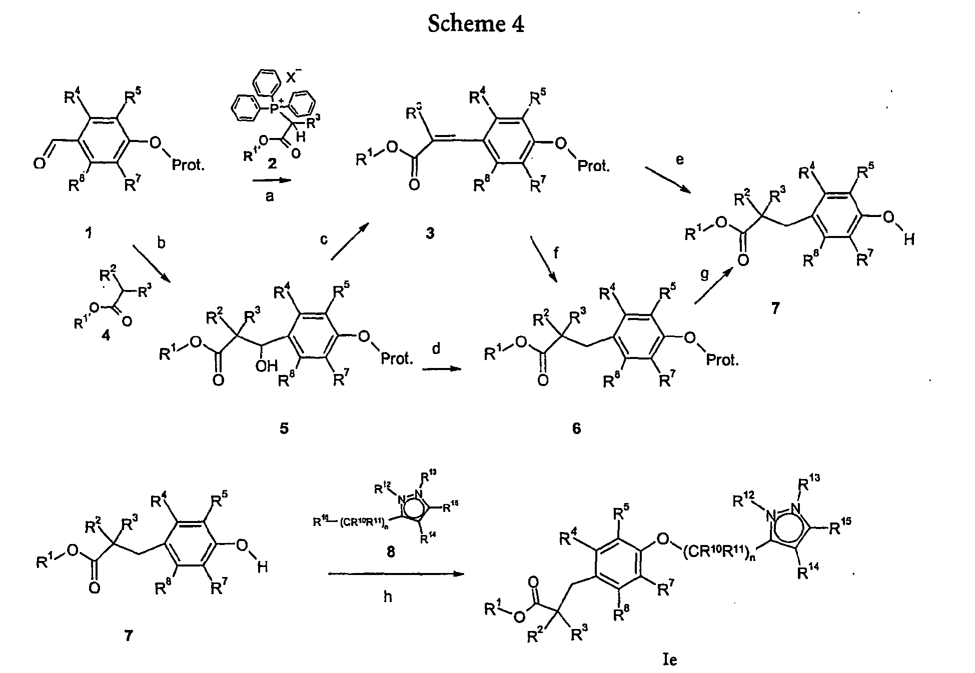

Aldehydes 1 are known, commercially available or can be prepared by methods known in the art. Aldehydes 1 can be reacted with a *Wittig* salt 2 such as (1,2-diethoxy-2-oxoethyl)triphenyl phosphonium chloride or (1,2-dimethoxy-2-oxoethyl)triphenyl phosphonium bromide in solvents like isopropanol, dichloromethane or tetrahydrofuran or mixtures thereof in the presence of a base like potassium carbonate, 1,8-diazabicydo[5.4.0]undec-7-ene (DBU), 1,1,3,3-tetramethyl-guanidine or sodium tert butylate, preferably between 0 °C and the reflux temperature of the solvents, giving acrylic esters 3 as E and/or Z isomers (step a). Alternatively, a *Horner-Wadsworth-Emmons* reaction can be used for the transformation of compounds 1 into unsaturated esters 3, e. g. using dimethyl(methoxycarbonyl)methyl phosphonate, optionally substituted at the methylene group, and a base like sodium hydride in a solvent like tetrahydrofuran. Hydrogenation of acrylic esters 3 using palladium on charcoal as catalyst, preferably at room temperature and 1 atm. pressure of hydrogen, in solvents like methanol, ethanol, tetrahydrofuran, acetic acid, dichloromethane and mixtures thereof, affords esters 7, provided that the protecting group can be cleaved reductively (step e).

Alternatively, aldehydes 1 are reacted with the enolate of an acetic acid ester 4 (preferably the lithium-enolate, prepared at -78 °C by treatment of 4 with a strong, non-nucleophilic base like lithium diisopropylamide in an inert solvent like tetrahydrofuran), preferably at temperatures around -78 °C, in solvents like tetrahydrofuran giving the aldol product 5 as a mixture of diastereomers (step b). Removal of the benzylic hydroxy group in compounds 5 can be performed with a reducing agent like e. g. triethylsilane in the presence of a Lewis acid, like boron-trifluoride, or a protic acid, like trifluoroacetic acid, in a suitable solvent like trifluoroacetic acid itself or dichloromethane between 0 °C and 60 °C to yield protected phenol compounds 6 (step d). Subsequent removal of the protecting group, e. g. a benzyl group, by standard technology, e. g. catalytic hydrogenation using hydrogen and a catalyst like palladium or by using dimethyl sulfide and boron trifluoride diethyl etherate in a solvent like dichloromethane between room temperature and the reflux temperature of the solvent gives phenolic compounds 7 (step g). Catalytic hydrogenation can be used to transform unsaturated esters 3 into compounds 6 (step f). In case the protective group in compounds 3 is a benzyl group, then a one step hydrogenation procedure directly gives phenolic compounds 7. Catalytic hydrogenation can also be used for the simultaneous removal of the benzylic hydroxy function and a benzyl protecting group, preferably using palladium on charcoal as catalyst in the presence of an acid like oxalic acid in solvents like alcohols at temperatures around room temperature and a hydrogen pressure up to 100 bar, thus giving the transformation of compounds 5 into compounds 7 in one step (step d and g). As an alternative method, compounds 5 can be treated with catalytic amounts of an acid like p-toluene sulfonic acid in a solvent like benzene or toluene, preferably under conditions allowing the removal of the water formed (e. g. with a Dean Stark trap or in the presence of molecular sieves) at temperatures between room temperature and the reflux temperature of the solvents to yield acrylic esters 3 (step c). The condensation of phenols 7 with pyrazoles 8 to form compounds Ie can be performed as outlined in scheme l.

An analogous reaction scheme with the same reaction sequences applies for the isomeric compound series leading to compounds of general formula I, particularly compounds according to formula If

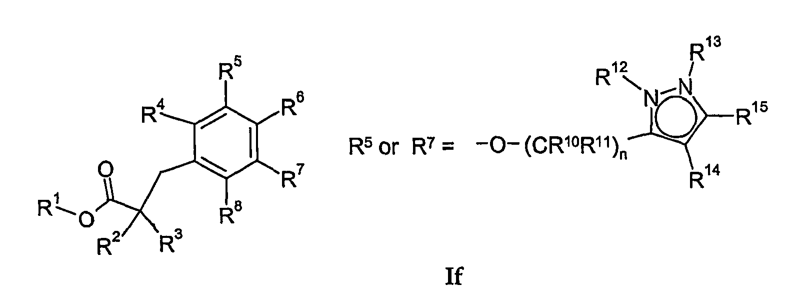

The synthesis of compounds with the general structure I, particularly compounds according to formula Ig, with X¹ equal to CH₂ and X² equal to nitrogen can be accomplished according to scheme 5.

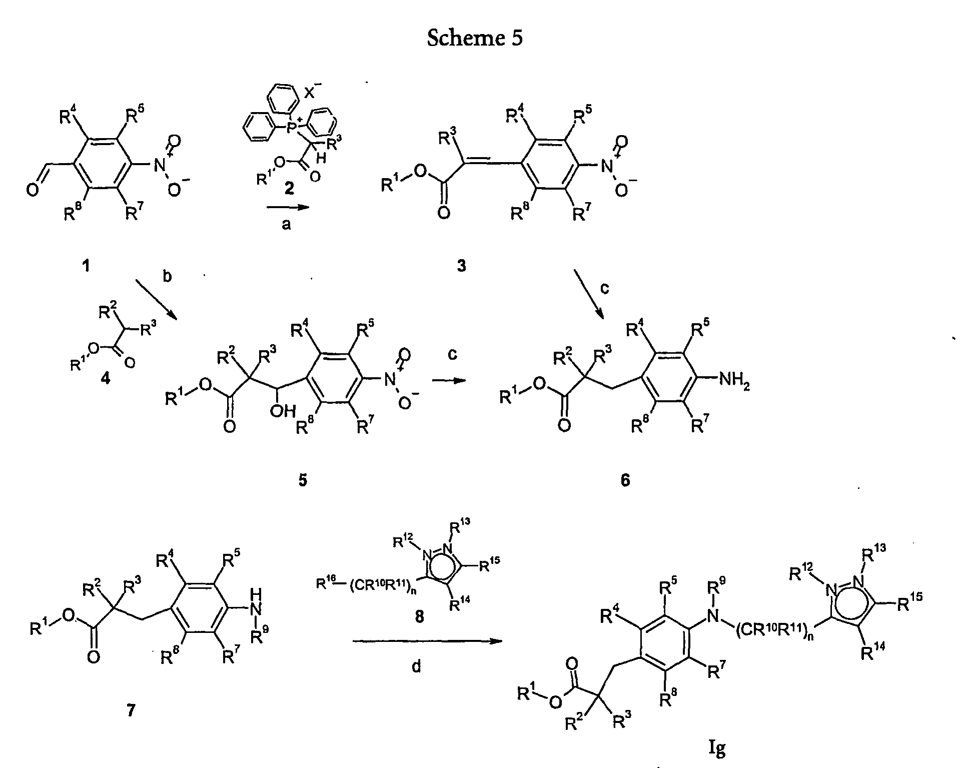

Nitro-phenyl compounds 3 and 5 are prepared from nitro aldehydes 1, which are known, commercially available or can be prepared by methods known in the art, e.g. by *WittiglHorner-Wadsworth-Emmons* or aldol reactions analogous to the reactions described for the synthesis of compounds 3 and 5 in scheme 4 (steps a and b). Catalytic hydrogenation can be used for the simultaneous removal of the benzylic hydroxy function (compounds 5) or the reduction of the double bond (compounds 3) and the reduction of the nitro group; preferably using palladium on charcoal as catalyst optionally in the presence of an acid like oxalic acid in solvents like alcohols at temperatures around room temperature and a hydrogen pressure up to 100 bar (step c). Compounds 7 with R⁹ substituents different from hydrogen are obtained by first introduction of a BOC group, alkylation and removal of the BOC protective function as described in schemes 2 and 3. The condensation of anilines 7 with pyrazoles 8 to form compounds Ig can be performed as outlined in scheme 3.

An analogous reaction scheme with the same reaction sequences applies for the isomeric compound series leading to compounds of general formula I, particularly compounds according to formula Ih:

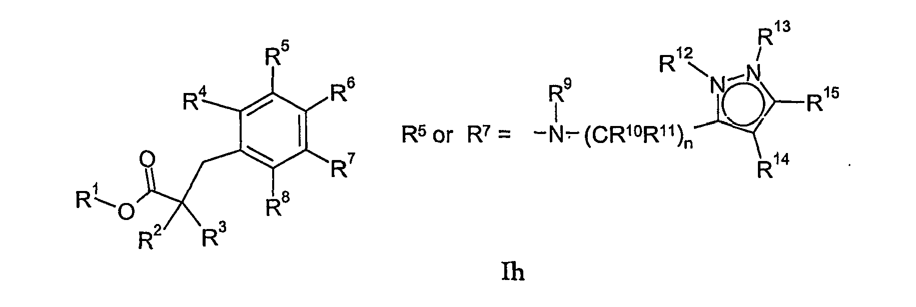

As alternative to the sequences described in scheme 5, the nitrogen containing intermediates can be prepared from suitable intermediates carrying a phenolic hydroxy function. In such intermediates, optionally carrying one or more protective functions, the phenolic OH group can be replaced by the corresponding aromatic NH₂ function by methods known in the art. For example by a three step sequence as described in Tetrahedron Letters 43(42), 7617-7619 (2002) and discussed in the context of schemes 2 and 3.

The synthesis of compounds with the general structure I, particularly compounds with X¹ and / or X² equal to S can be accomplished in close analogy to the synthesis of the corresponding analogues with X¹ and / or X² equal to oxygen. Suitable sulfur containing intermediates are known, can be prepared by methods known in the art or are prepared from phenolic intermediates as described by W Zhi-Liang and AP Kozikowski (J. Org. Chem. 2003, 68, 9116-9118): treatment of a phenolic intermediate with sodium thiocyanate, sodium bromide and bromine in a solvent like methanol preferably between 0 °C and room temperature gives the corresponding 4-thiocyanato-phenols; subsequent reduction with lithium aluminium hydride in a solvent like tetrahydrofuran at temperatures around 0 °C then liberates the corresponding 4-mercapto-phenol. Alternatively, intermediates carrying an aromatic SH moiety can be prepared from suitable intermediates carrying a phenolic hydroxy function. In such intermediates, optionally carrying one or more protective functions, the phenolic OH group can be replaced by the corresponding aromatic SH function by methods known in the art. For example by a three step sequence as described in J. Labelled Compounds & Radiopharmaceuticals 43(7), 683-691, (2000): i) transformation of the phenol moiety into its trifluoromethanesulfonate (triflic anhydride, triethylamine, dichloromethane, at low temperature, preferably around -30 °C); ii) treatment of the triflate with triisopropylsilanethiolate, tetrakis(triphenylphosphine)-palladium(0) in solvent mixtures like toluene and tetrahydrofuran in a temperature range between 60 °C and 150 °C; iii) treatment of the silyl sulfide with hydrogen chloride in methanol preferably around 0 °C to liberate the phenolic SH moiety.

Compounds of the general formula I may be obtained in the form of racemates. Racemic compounds can be separated into their antipodes by methods known in the art, such as separation of the antipodes via diastereomeric salts by crystallization with optically pure amines such as e. g. (R) or (S)-1-phenyl-ethylamine, (R) or (S)-1-naphthalen-1-yl-ethylamine, brucine, quinine or quinidine or by separation of the antipodes by specific chromatographic methods using either a chiral adsorbens or a chiral eluent.

Pyrazoles 5 (scheme 1), identical to 11 (scheme 3), 8 (scheme 4) and 8 (scheme 5) are commercially available, known or can be synthesized by methods known in the art. Representative examples of possible syntheses of these key intermediates are given in schemes 6-9.

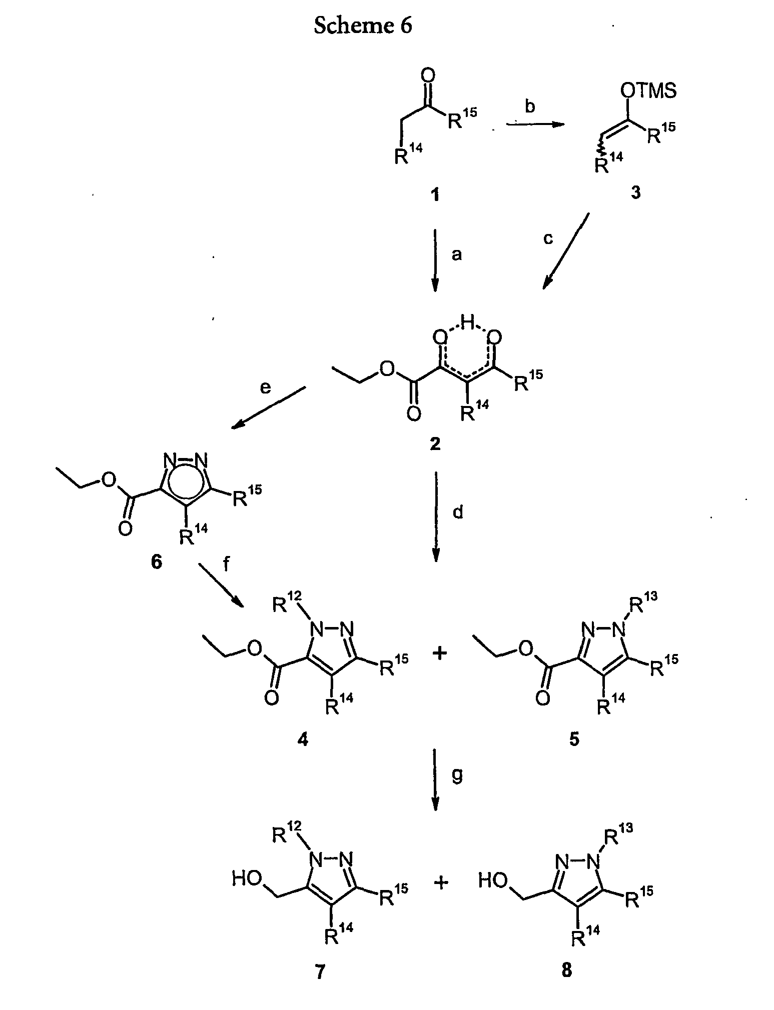

Substituted acetophenones and heteroaryl ketones 1 are commercially available, known or can be prepared by methods known in the art. Acylation of compounds 1 with oxalate derivatives can be performed under standard conditions, e. g. with diethyl oxalate in the presence of a base like sodium ethoxide at temperatures between -78 °C and 50 °C in solvents like ethanol, or with lithium hexamethyldisilazide at temperatures between -78 °C and ambient temperature in solvents like ether, to form after subsequent acidification free ethyl pyruvates 2 (step a). Alternatively, pyruvates 2 can be synthesized via i) transforming ketones 1 into the corresponding silyl enol ethers 3, e. g. through treatment with trimethylsilyl chloride in the presence of a base like triethylamine at temperatures between 0 °C and 40 °C in a solvent like acetonitrile (step b); ii) in situ formation of a metal enol ether, e. g. with zinc chloride and subsequent acylation with an acylation reagent like ethyl oxalyl chloride at temperatures between 0 °C and 50 °C in a solvent like toluene or dichloromethane (step c). Pyruvates 2 can be converted to regioisomeric pyrazoles 4 and 5 through condensation with monosubstituted hydrazines H₂NNHR^{12/13} which are commercially available, known or can be prepared by methods known in the art, e. g. at temperatures between ambient temperature and the reflux temperature of the solvent in solvents like ethanol (step d). Alternatively, pyrazoles 4 and 5 can be synthesized via i) reacting pyruvates 2 with hydrazine, preferably at reflux temperature in ethanol (step e); ii) conversion of the obtained pyrazole 6 into regioisomeres 4 and 5 under standard conditions, e. g. through alkylation with an alkyl halogenide in the presence of a base like potassium hydroxide at temperatures between -20 °C and the reflux temperature of the solvent in solvents like ethanol (step f). Regioisomeric pyrazoles 4 and 5 can easily be separated by standard techniques, e. g. through column chromatography on silica. Reduction of esters 4 and 5 can be performed by methods well known in the art, e. g. with lithium aluminium hydride at temperatures between 0 °C and the reflux temperature of the solvents in solvents like tetrahydrofuran or dimethyl ether (step g).

The alcohol compounds 7 and 8 correspond to or can be converted into compounds of general formula 5 (scheme 1), identical to 11 (scheme 3), 8 (scheme 4) and 8 (scheme 5), e. g. by treatment with methanesulfonyl chloride in dichloromethane in the presence of a base like triethylamine preferably in a temperature range between - 20 °C and room temperature, or e. g. by reaction with carbon tetrachloride or carbon tetrabromide and triphenylphosphine in solvents like tetrahydrofuran, preferably in a temperature range between room temperature and the reflux temperature of the solvents.

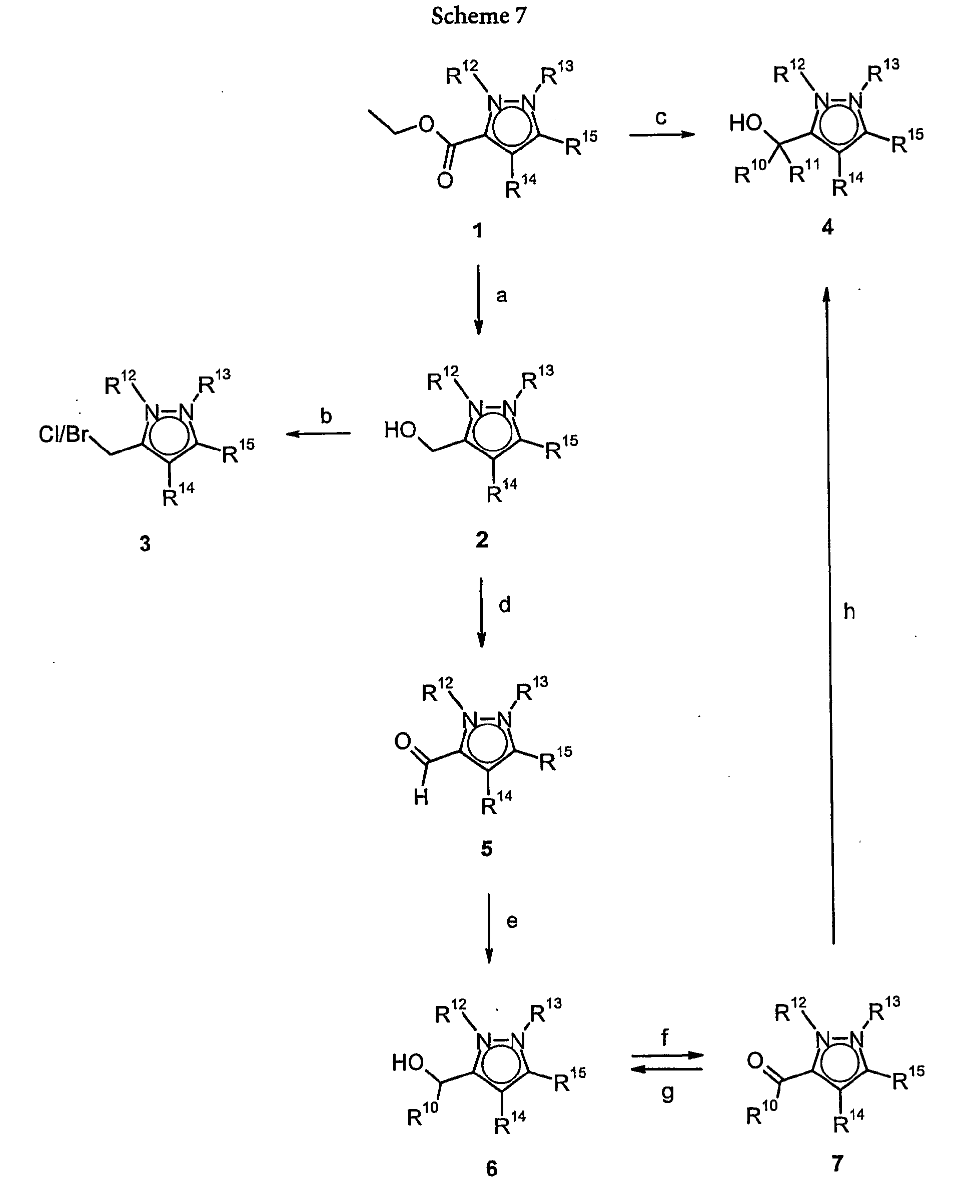

Reduction of pyrazole esters 1 (compounds 4, 5 and 6 in scheme 6), preferably using lithium aluminum hydride in a solvent like ether or tetrahydrofuran, preferably between 0 °C and room temperature, gives primary alohols 2 (step a), which can be used as such or can be converted into the corresponding halides 3, e. g. by treatment with methanesulfonyl chloride in dichloromethane in the presence of 2,6-lutidine, preferably between -20 °C and the reflux temperature of dichloromethane, by treatment with thionyl chloride in a solvent like dichloromethane or chloroform, preferably at temperatures between -20 °C and +50 °C, or by treatment with tetrabromomethane and triphenylphosphine in solvents like tetrahydrofuran at temperatures between 0 °C and the reflux temperature of tetrahydrofuran (step b). Esters 1 can further be converted into tertiary alcohols 4 with R¹⁰ = R¹¹ through reaction with alkyl organometallic reagents, preferably using alkyl Grignard compounds in a solvent like tetrahydrofuran or ether, preferably between -15 °C and the reflux temperature of the solvent (step c). Alkohols 4 with R¹⁰ not equal to R¹¹ can be prepared by a sequential procedure: i) saponification to the acid; ii) treatment with R¹⁰Li, optionally in the presence of a Cu(I) salt, in ether or tetrahydrofuran to yield the alkyl ketones -COR¹⁰; iii) subsequent reaction with R¹¹Li or lithium aluminium hydride in ether or tetrahydrofuran (step c). Primary alcohols 2 can be oxidized to aldehydes 5 by methods known in the art, e. g. by treatment with pyridinium chlorochromate in dichloromethane, preferably at temperatures between room temperature and the reflux temperature of dichloromethane, or by treatment with manganese dioxide in solvents like dichloromethane, preferably at room temperature (step d). These aldehydes 5 can be converted to the corresponding secondary alcohols 6 through reaction with alkyl organometallic compounds, preferably under the conditions, given for the transformation of esters 1 to tertiary alcohols 4 (step e). Ketones 7 can be obtained from secondary alcohols 6 by methods known in the art, e. g. by treatment with Cr(VI) reagents like the Jones reagent (Jones et al., J. Chem. Soc. 1953, 2548.) (step f). These ketones 7 can be reduced back to the corresponding secondary alcohols 6 in an enantioselective fashion leading to the (R)- or (S)-alcohols 6, e. g. by treatment with borane-dimethylsulfide complex and (S)- or (R)-2-methyl-CBS-oxazaborolidine as chiral catalyst in tetrahydrofuran, preferably at temperatures between -78 °C and ambient temperature, according to Corey et al. (E. J. Corey, R. K. Bakshi, S. Shibata, J. Am. Chem. Soc. 1987, 109, 5551-5553)*,* or by treatment with (+)- or (-)-B-chlorodiisopinocampheylborane (DIP-Cl), according to Brown et al. (P. V. Ramachandran, B. Gong, A. V. Teodorovic, H. C. Brown, Tetrahedron: Asymmetry 1994, 5, 1061-1074) (step g). Ketones 7 can in addition be converted to the corresponding tertiary alcohols 4 through reaction with alkyl organometallic compounds, preferably under the conditions given for the transformation of esters 1 to tertiary alcohols 4 in step c (step h). If the alcohol compounds 2, 4, or 6 contain one or more chiral centers and are not optically pure, they can optionally be separated into optically pure antipodes by methods well known in the art, e. g. chromatography on a chiral HPLC column, or by derivatization with an optically pure acid to form esters, which can then be separated by conventional HPLC chromatography and converted back to the original alcohol.

The alcohol compounds 2, 4, and 6, and the halide compound 3, correspond to or can be converted into compounds of general formula 5 (scheme 1), identical to 11 (scheme 3), 8 (scheme 4) and 8 (scheme 5), e. g. by treatment with methanesulfonyl chloride in dichloromethane in the presence of a base like triethylamine preferably in a temperature range between -20 °C and room temperature, or e. g. by reaction with carbon tetrachloride or carbon tetrabromide and triphenylphosphine in solvents like tetrahydrofuran, preferably in a temperature range between room temperature and the reflux temperature of the solvents.

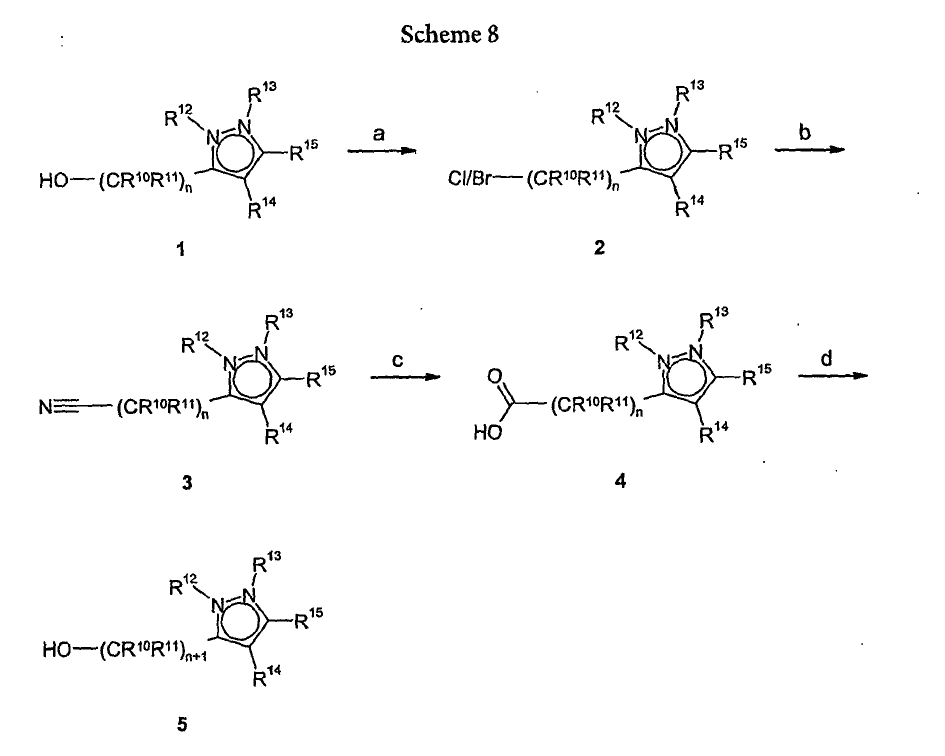

Pyrazole alkanols 1 with a chain length of n carbon atoms can be converted into analogues with a chain length of n+1 carbon atoms by methods well known in the art, e. g. by conversion of the primary alcohol function into a suitable leaving group, e. g. a halide (step a), reaction with cyanide ion (step b), saponification (step c) followed by reduction of the acid formed (compounds 4) to the primary alcohols 5, e.g. by using diborane in tetrahydrofuran (step d). In order to introduce substituents R¹⁰ and/or R¹¹ different from hydrogen, cyano intermediates 3 of this elongation process can be reacted with alkyl Grignard reagents R¹⁰MgX in solvents like ether or tetrahydrofuran between 0 °C and the reflux temperature of the solvent to form the corresponding R¹⁰CO- alkyl ketones, which upon treatment with an alkyllithium reagent R¹¹Li or lithium aluminum hydride in solvents like ether or tetrahydrofuran give alcohols 5. R¹⁰CO- alkyl ketones can also be reduced, e. g. by treatment with sodium borohydride in alcohol, preferably at temperatures between -15 °C. and 40 °C. This reaction can also be carried out in an enantioselective fashion leading to the (R)- or (S)-alcohols 5, e. g. by treatment with borane-dimethylsulfide complex and (S)- or (R)-2-methyl-CBS-oxazaborolidine as chiral catalyst in tetrahydrofuran, preferably at temperatures between -78 °C and ambient temperature according to Corey et al. (E. J. Corey, R. K. Bakshi, S. Shibata, J. Am. Chem. Soc. 1987, 109, 5551-5553), or by treatment with (+)- or (-)-B-chlorodiisopinocampheylborane (DIP-Cl), according to Brown et al. (P. V. Ramachandran, B. Gong, A. V. Teodorovic, H. C. Brown, Tetrahedron: Symmetry 1994, 5,1061-1074). Alternatively, alcohol compounds 5 which contain one or more chiral centers can optionally be separated into optically pure antipodes by methods well known in the art, e. g. chromatography on a chiral HPLC column, or by derivatization with an optically pure acid to form esters, which can then be separated by conventional HPLC and converted back to the original alcohol. The alcohol compounds 5 correspond to or can be transformed into compounds of general formula 5 (scheme 1), identical to 11 (scheme 3), 8 (scheme 4) and 8 (scheme 5), e. g. by treatment with methanesulfonyl chloride in dichloromethane in the presence of a base like triethylamine, preferably in a temperature range between -20 °C and room temperature, or e. g. by reaction with carbon tetrachloride or carbon tetrabromide and triphenylphosphine in solvents like tetrahydrofuran, preferably in a temperature range between room temperature and the reflux temperature of the solvents. 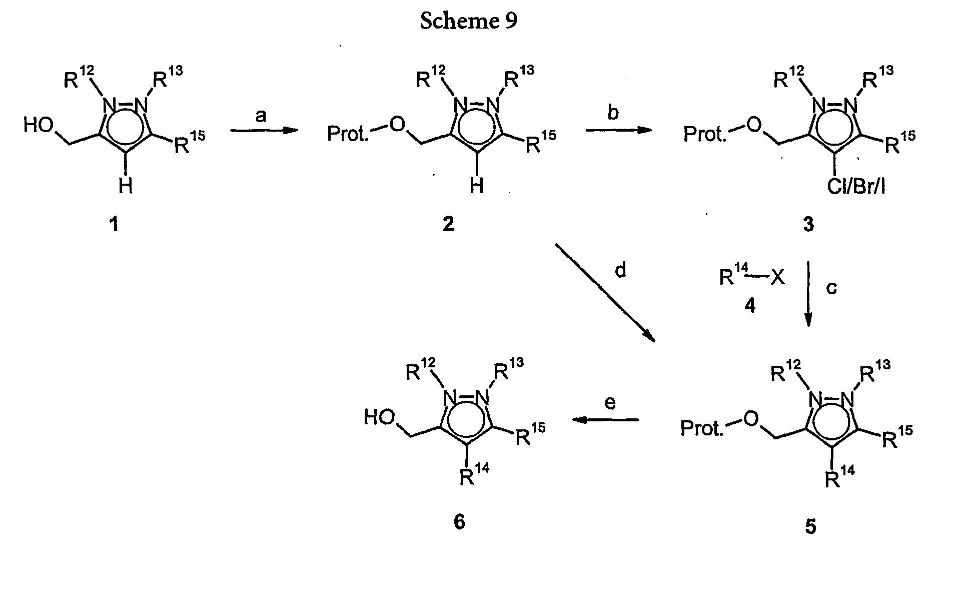 Alcohols 1 (compounds 5 with R¹⁴ = H and R¹⁶= OH in scheme 1, compounds 11 with R¹⁴ = H and R¹⁶= OH in scheme 3, compounds 8 with R¹⁴ = H and R¹⁶= OH in schemes 4 and 5, compounds 7 and 8 with R¹⁴ = H in scheme 6, compounds 2, 4 and 6 with R¹⁴ = H in scheme 7, compounds 1 and 5 with R¹⁴ = H in scheme 8), can be protected by methods known in the literature, e. g. by treating them with tert-butyldimethylsilyl chloride in the presence of imidazole, preferably at room temperature in solvents like N,N-dimethylformamide, to obtain the corresponding tert-butyldimethylsilyl ethers 2 (step a). Halogenation of protected pyrazoles 2, e. g. through reaction with bromine preferably at temperatures between 0 °C and ambient temperature in solvents like dichloromethane delivers 4-halo pyrazoles 3 (step b). Compounds 3 can - following halogen metal exchange, preferably with tert-butyllithium at -78 °C in solvents like tetrahydrofuran - be reacted with alkylating reagents 4 with X e. g. being a chlorine, bromine or iodine atom, preferably with alkyl iodides, at temperatures between -78 °C and ambient temperature in solvents like tetrahydrofuran, to form pyrazoles 5 bearing a substituent in position 4 (step c). Alternatively, transition metal catalyzed reactions can be used to transform 4-halo pyrazoles 3 into compounds 5, e. g. by treatment with a stannane (X being trialkyl stannyl) in the presence of a Pd(0) catalyst like [Pd₂(dba)₃] and triphenyl arsine at temperatures between 0 °C and the reflux temperature of the solvent in solvents like dioxane. Residues R¹⁴ can further be introduced by i) formylation of pyrazoles 2 through methods well known in the art, e. g. with phosphorus oxychloride and N,N-dimethylformamide preferably at temperatures between 0 °C and 100 °C; ii) subsequent transformation of the intermediate formyl pyrazole to 4-substituted pyrazoles 5, e. g. through reduction with sodium cyano borohydride in the presence of zinc iodide at temperatures between -78 °C and the reflux temperature of the solvent in solvents like diethyl ether (step d). O-Deprotection of compounds 5 leading to building blocks 6 can be performed by methods described in the literature, e. g. by treatment with tetrabutyl ammonium fluoride at temperatures between -15 °C and ambient temperature in a solvent like tetrahydrofuran, if the protecting groups are silyl ethers (step e). The alcohol compounds 6 correspond to or can be transformed into compounds of general formula 5 (scheme 1), identical to 11 (scheme 3), 8 (scheme 4) and 8 (scheme 5), e. g. by treatment with methanesulfonyl chloride in dichloromethane in the presence of a base like triethylamine, preferably in a temperature range between -20 °C and room temperature, or e. g. by reaction with carbon tetrachloride or carbon tetrabromide and triphenylphosphine in solvents like tetrahydrofuran, preferably in a temperature range between room temperature and the reflux temperature of the solvents.

The following tests were carried out in order to determine the activity of the compounds of formula (I).

Background information on the performed assays can be found in: Nichols IS et al. "Development of a scintillation proximity assay for peroxisome proliferator-activated receptor gamma ligand binding domain", (1998) Anal. Biochem. 257:112-119.

Full-length cDNA clones for humans PPARδ and PPARα and mouse PPARγ were obtained by RT-PCR from human adipose and mouse liver cRNA, respectively, cloned into plasmid vectors and verified by DNA sequencing. Bacterial and mammalian expression vectors were constructed to produce glutathione-s-transferase (GST) and Gal4 DNA binding domain proteins fused to the ligand binding domains (LBD) of PPARδ (aa 139 to 442), PPARγ (aa 174 to 476) and PPARα (aa 167 to 469). To accomplish this, the portions of the cloned sequences encoding the LBDs were amplified from the full-length clones by PCR and then sub cloned into the plasmid vectors. Final clones were verified by DNA sequence analysis.

Induction, expression, and purification of GST-LBD fusion proteins were performed in *E. coli* strain BL21(pLysS) cells by standard methods (Ref: Current Protocols in Molecular Biology, Wiley Press, edited by Ausubel et al.).

### Radioligand Binding Assay

PPARδ receptor binding was assayed in HNM10 (50mM Hepes, pH 7.4, 10 mM NaCl, 5mM MgCl₂, 0.15 mg/ml fatty acid-free BSA and 15 mM DTT). For each 96 well reaction a 500 ng equivalent of GST-PPARδ-LBD fusion protein and radioligand, e.g. 20000 dpm {2-methyl-4-[4-methyl-2-(4-trifluoromethyl-phenyl)-thiazol-5-yl-ditritiomethylsulfanyl]-phenoxy}-acetic acid, was bound to 10 µg SPA beads (PharmaciaAmersham) in a final volume of 50 µl by shaking. The resulting slurry was incubated for 1h at RT and centrifuged for 2 min at 1300g. The supernatant containing unbound protein was removed and the semidry pellet containing the receptor-coated beads was resuspended in 50 ul of HNM. Radioligand was added and the reaction incubated at RT for 1h and scintillation proximity counting performed in the presence of test compounds was determined. All binding assays were performed in 96 well plates and the amount of bound ligand was measured on a Packard TopCount using OptiPlates (Packard). Dose response curves were done in triplicates within a range of concentration from 10⁻¹⁰ M to 10⁻⁴ M.

PPARα receptor binding was assayed in TKE50 (50mM Tris-HCl, pH 8, 50 mM KCl, 2mM EDTA, 0.1 mg/ml fatty acid-free BSA and 10 mM DTT). For each 96 well reaction an 140 ng equivalent of GST-PPARα-LBD fusion protein was bound to 10 µg SPA beads (PharmaciaAmersham) in a final volume of 50 µl by shaking. The resulting slurry was incubated for 1h at RT and centrifuged for 2 min at 1300g. The supernatant containing unbound protein was removed and the semidry pellet containing the recptor-coated beads was resolved in 50 µl of TKE. For radioligand binding e.g. 10000 dpm of 2(S)-(2-benzoyl-phenylamino)-3-{4- [1,1-ditritio-2-(5-methyl-2-phenyl-oxazol-4-yl)-ethoxy]-phenyl}-propionic acid or 2,3-ditritio-2(S)-methoxy-3-{4-[2-(5-methyl-2-phenyl-oxazol-4-yl)-ethoxy] -benzo [b]thiophen-7-yl}-propionic acid in 50 ul were added, the reaction incubated at RT for 1h and scintillation proximity counting performed. All binding assays were performed in 96 well plates and the amount of bound ligand measured on a Packard TopCount using OptiPlates (Packard). Nonspecific binding was determined in the presence of 10⁻⁴ M unlabelled compound. Dose response curves were done in triplicates within a range of concentration from 10⁻¹¹ M to 10⁻⁴ M.

PPARγ receptor binding was assayed in TKE50 (50mM Tris-HCl, pH 8, 50 mM KCl, 2mM EDTA, 0.1 mg/ml fatty acid-free BSA and 10 mM DTT). For each 96 well reaction an 140 ng equivalent of GST-PPARγ-LBD fusion protein was bound to 10 µg SPA beads (PharmaciaAmersham) in a final volume of 50 ul by shaking. The resulting slurry was incubated for 1h at RT and centrifuged for 2 min at 1300g. The supernatant containing unbound protein was removed and the semidry pellet containig the receptor-coated beads was resolved in 50 ul of TKE. For radioligand binding e.g. 10000 dpm 2(S)-(2-benzoyl-phenylamino)-3-{4-[1,1-ditritio-2-(5-methyl-2-phenyl-oxazol-4-yl)-ethoxy]-phenyl}-propionic acid in 50 µl were added, the reaction incubated at RT for 1h and scintillation proximity counting performed. All binding assays were performed in 96 well plates and the amount of bound ligand measured on a Packard TopCount using OptiPlates (Packard). Nonspecific binding was determined in the presence of 10⁻⁴ M unlabelled compound. Dose response curves were done in triplicates within a range of concentration from 10⁻¹⁰ M to 10⁻⁴ M.

### Luciferase Transcriptional Reporter Gene Assays (Transactivation Assay)

Baby hamster kidney cells (BHK21 ATCC CCL10) were grown in DMEM medium containing 10% FBS at 37°C in a 95%O2:5%CO₂ atmosphere. Cells were seeded in 6 well plates at a density of 10⁵ cells/well and then batch-transfected with either the pFA-PPARδ-LBD, pFA-PPARγ-LBD or pFA-PPARα-LBD expression plasmids plus a reporter plasmid (to monitor transfection efficiency). Transfection was accomplished with the Fugene 6 reagent (Roche Molecular Biochemicals) according to the suggested protocol. Six hours following transfection, the cells were harvested by trypsinization and seeded in 96 well plates at a density of 10⁴ cells/well. After 24 hours the medium was removed and replaced with 100 ul of phenol red-free medium containing the corresponding test compounds or control ligands (at a final DMSO concentration of 0.1%). Following incubation of the cells for 24 hours with compounds, 50 µl of the supernatant was discarded and then 50 µl of Luciferase Constant-Light Reagent (Roche Molecular Biochemicals) was added to lyse the cells and initiate the luciferase reaction. Luminescence for luciferase was measured in a Packard TopCount. Transcriptional activation in the presence of a test substance was expressed as fold-activation over cells incubated in the absence of the substance. The signals were normalized against plate-specific controls (DMSO alone) and the fold-stimulation of luciferase activity observed with specific and selective PPARα ((2(S)-2-(2-benzoyl-phenylamino)-3-{4-[2-(5-methyl-2-phenyl-oxazol-4-yl)-ethoxy]-phenyl}-propionic acid), PPARγ (Rosiglitazone) and PPARδ ({2-methyl-4-[4-methyl-2-(4-trifluoromethyl-phenyl)-thiazol-5-ylmethylsulfanyl]-phenoxy}-acetic acid) reference compounds was set to 100 %. EC50 values were calculated using the XLfit program (ID Business Solutions Ltd. UK).

The free acids of the compounds of the present invention (R¹ is hydrogen) exhibit IC₅₀ values of 0.1 nM to 10 µM, preferably 1 nM to 500 nM for PPARδ and/or IC₅₀ values of 1 nM to 10 µM , preferably 10 nM to 500 nM for PPARα. Further the free acids of the compounds of the present invention (R¹ is hydrogen) exhibit EC₅₀ values of 1 nM to 10 µM, preferably 10 nM to 1 µM for PPARδ and/or EC₅₀ values of 1 nM to 10 µM , preferably 10 nM to 1 µM for PPARα Compounds, in which R¹ is not hydrogen are converted in vivo to compounds in which R¹ is hydrogen. The following table shows measured values for some selected compounds of the present invention.

| | PPARα IC5₀ (µmol/l) | PPARγ IC₅₀ (µmol/l) | PPARδ IC₅₀ (µmol/l) | PPARα EC₅₀ (µmol/l) (fold activation [%] ) | PPARδ EC₅₀ (µmol/l) (fold activation [%]) |
|---|---|---|---|---|---|
| Example 1 | 0.166 | 10 | 0.144 | 0.38 (100) | 0.2 (59) |
| Example 6 | 0.212 | 10 | 0.222 | 0.16 (120) | 0.096 (140) |

The compounds of formula (I) and their pharmaceutically acceptable salts and esters can be used as medicaments, e.g. in the form of pharmaceutical preparations for enteral, parenteral or topical administration. They can be administered, for example, perorally, e.g. in the form of tablets, coated tablets, dragées, hard and soft gelatine capsules, solutions, emulsions or suspensions, rectally, e.g. in the form of suppositories, parenterally, e.g. in the form of injection solutions or infusion solutions, or topically, e.g. in the form of ointments, creams or oils.

The production of the pharmaceutical preparations can be effected in a manner which will be familiar to any person skilled in the art by bringing the described compounds of formula (I) and their pharmaceutically acceptable, into a galenical administration form together with suitable, non-toxic, inert, therapeutically compatible solid or liquid carrier materials and, if desired, usual pharmaceutical adjuvants.

Suitable carrier materials are not only inorganic carrier materials, but also organic carrier materials. Thus, for example, lactose, corn starch or derivatives thereof, talc, stearic acid or its salts can be used as carrier materials for tablets, coated tablets, dragées and hard gelatine capsules. Suitable carrier materials for soft gelatine capsules are, for example, vegetable oils, waxes, fats and semi-solid and liquid polyols (depending on the nature of the active ingredient no carriers are, however, required in the case of soft gelatine capsules). Suitable carrier materials for the production of solutions and syrups are, for example, water, polyols, sucrose, invert sugar and the like. Suitable carrier materials for injection solutions are, for example, water, alcohols, polyols, glycerol and vegetable oils. Suitable carrier materials for suppositories are, for example, natural or hardened oils, waxes, fats and semi-liquid or liquid polyols. Suitable carrier materials for topical preparations are glycerides, semi-synthetic and synthetic glycerides, hydrogenated oils, liquid waxes, liquid paraffins, liquid fatty alcohols, sterols, polyethylene glycols and cellulose derivatives.

Usual stabilizers, preservatives, wetting and emulsifying agents, consistency-improving agents, flavour-improving agents, salts for varying the osmotic pressure, buffer substances, solubilizers, colorants and masking agents and antioxidants come into consideration as pharmaceutical adjuvants.

The dosage of the compounds of formula (I) can vary within wide limits depending on the disease to be controlled, the age and the individual condition of the patient and the mode of administration, and will, of course, be fitted to the individual requirements in each particular case. For adult patients a daily dosage of about 1 mg to about 1000 mg, especially about 1 mg to about 100 mg, comes into consideration. Depending on the dosage it is convenient to administer the daily dosage in several dosage units.

The pharmaceutical preparations conveniently contain about 0.1-500 mg, preferably 0.5-100 mg, of a compound of formula (I).

The following examples serve to illustrate the present invention in more detail. They are, however, not intended to limit its scope in any manner.

### Examples

### Abbreviations:

AcOEt = ethyl acetate, DMF = N,N-dimethylformamide, DMPU = 1,3-dimethyl-3,4,5,6-tetrahydro-2(1H)-pyrimidinone, MeOH = methanol, quant. = quantitative, RT = room temperature.

### Example 1

### a] (Z)-2-Hydroxy-4-oxo-4-(4-trifluoromethoxy-phenyl)-but-2-enoic acid ethyl ester

A solution of 1-(4-trifluoromethoxy-phenyl)-ethanone (5 g, 24 mmol) and diethyl oxalate (3.25 ml, 24 mmol) in ethanol (5 ml) was added within 20 min to an ice cooled solution of metallic sodium (552 mg, 24 mmol) in ethanol (15 ml) under an argon atmosphere. The cooling bath was removed and the reaction stirred 30 min after reaching ambient temperature. After standing 14 h, the precipitated yellow solid was filtered. The solid was partitioned between 1 M HCl/ice water 1/1 and tert butyl methyl ether. The aqueous layer was extracted two times with tert butyl methyl ether, the combined extracts were washed with brine/ice water 1/1 and dried over sodium sulfate. Evaporation of the solvent under reduced pressure gave 7.2 g (23.8 mmol, 99 %) of the title compound as orange crystals.
MS: 305.0 (M+H)⁺.

### b] 5-(4-Trifluoromethoxy-phenyl)-1H-pyrazole-3-carboxylic acid ethyl ester

Hydrazine monohydrate (0.78 ml, 24 mmol) was added at ambient temperature to a solution of (Z)-2-hydroxy-4-oxo-4-(4-trifluoromethoxy-phenyl)-but-2-enoic acid ethyl ester (7.2 g, 24 mmol) in ethanol (37 ml) under an argon atmosphere. The suspension was stirred for 4 h at reflux temperature, the solvent was removed under reduced pressure and the residue partitioned between 1 M HCl/ice water and ethyl acetate. The aqueous layer was extracted two times with ethyl acetate, the combined extracts were washed with brine (3 times) and dried over sodium sulfate. Removal of the solvent under reduced pressure left yellow crystals which were recrystallized from dichloromethane/heptane to give 4.3 g (14.5 mmol, 61 %) of the title compound as yellow crystals.
MS: 301.0 (M+H)⁺.

### c] 2-Methyl-5-(4-trifluoromethoxy-phenyl)-2H-pyrazole-3-carboxylic acid ethyl ester and 1-methyl-5-(4-trifluoromethoxy-phenyl)-1H-pyrazole-3-carboxylic acid ethyl ester

5-(4-Trifluoromethoxy-phenyl)-1H-pyrazole-3-carboxylic acid ethyl ester (3 g, 10 mmol) was added to a solution of KOH (701 mg, 12 mmol) in absolute ethanol (91 ml). The solution was stirred at ambient temperature for 15 min. Methyl iodide (1.25 ml, 20 mmol) was added and the reaction solution was heated under reflux for 3 h. The solvent was removed under reduced pressure and the residue dissolved in brine/ice water 1/1 and ethyl acetate. The layers were separated and the aqueous layer was extracted two times with ethyl acetate. The combined organic layers were washed with brine and dried over sodium sulfate. The solvent was removed under reduced pressure and the residue purified by column chromatography (silica gel, heptane/AcOEt) to give 2.2 g (7 mmol, 70 %) 2-methyl-5-(4-trifluoromethoxy-phenyl)-2H-pyrazole-3-carboxylic acid ethyl ester as white crystals and 170 mg (0.54 mmol, 5 %) 1-methyl-5-(4-trifluoromethoxyphenyl)-1H-pyrazole-3-carboxylic acid ethyl ester as yellow oil.

2-methyl-5-(4-trifluoromethoxy-phenyl)-2H-pyrazole-3-carboxylic acid ethyl ester: MS: 315.0 (M+H)⁺.

### d] [2-Methyl-5-(4-trifluoromethoxy-phenyl)-2H-pyrazol-3-yl]-methanol

A solution of 2-methyl-5-(4-trifluoromethoxy-phenyl)-2H-pyrazole-3-carboxylic acid ethyl ester (2.2 g, 7 mmol) in diethyl ether (68 ml) was added within 5 min to a suspension of lithium aluminium hydride (584 mg, 15 mmol) in diethyl ether (68 ml) under an argon atmosphere at ambient temperature. The mixture was heated to reflux for 12 h, cooled to 0 °C and treated cautiously with water (20 ml) and 10 % aqueous NaOH (10 ml). The reaction mixture was filtered over celite, ice water/tert butyl methyl ether 1/1 was added and the layers were separated. The aqueous layer was extracted one more time with tert butyl methyl ether, the combined organic layers were washed with ice water/brine 1/1 and dried over sodium sulfate. Removal of the solvent under reduced pressure gave 1.84 g (6.8 mmol, 97 %) of the title compound as white crystals.
MS: 273.1 (M+H)⁺.

### e] 2-Methyl-2-{2-methyl-4-[2-methyl-5-(4-trifluoromethoxy-phenyl)-2H-pyrazol-3-ylmethoxy]-phenoxy}-propionic acid ethyl ester

To an ice cold solution of 2-(4-hydroxy-2-methyl-phenoxy)-2-methyl-propionic acid ethyl ester (50 mg, 210 µmol; PCT Int. Appl. (2002), WO 2002092590 A1), [2-methyl-5-(4-trifluoromethoxy-phenyl)-2H-pyrazol-3-yl]-methanol (57 mg, 210 µmol) and tributylphosphine (70 µl, 250 µmol) in tetrahydrofuran (5 ml) was added N,N,N',N'-tetramethyl azodicarboxamide (43 mg, 250 µmol). The cooling bath was removed and stirring continued for 14 h. The mixture was filtered over celite and the solvent removed under reduced pressure to give a yellow oil which was purified by column chromatography (silica gel, heptane/AcOEt) to obtain 77 mg (160 µmol, 75 %) of the title compound as colorless oil.
MS: 493.5 (M+H)⁺.

### f] 2-Methyl-2-12-methyl-4-[2-methyl-5-(4-trifluoromethoxy-phenyl)-2H-pyrazol-3-ylmethoxy]-phenoxy}-propionic acid

To a solution of 2-methyl-2-{2-methyl-4-[2-methyl-5-(4-trifluoromethoxy-phenyl)-2H-pyrazol-3-ylmethoxy]-phenoxy}-propionic acid ethyl ester (10 mg, 20 µmol) in THF/methanol 2/1 (1.5 ml) was added 1 N aqueous LiOH solution (120 µl). The reaction mixture was stirred for 14 h at ambient temperature and concentrated under reduced pressure. The residue was dissolved in 1 N HCl/ice water 1/1 and ethyl acetate, the layers were separated and the aqueous layer was extracted with ethyl acetate. The combined extracts were washed with ice water/brine 1/1, dried over sodium sulfate and the solvent was evaporated in vacuo to give the title compound (9 mg, 20 µmol, 95 %) as off-white solid.
MS: 463.1 (M-H)⁻.

### Example 2

### a] 5-Chloromethyl-1-methyl-3-(4-trifluoromethoxy-phenyl)-1H-pyrazole

To a solution of [2-methyl-5-(4-trifluoromethoxy-phenyl)-2H-pyrazol-3-yl]-methanol (1 g, 3.7 mmol; example 1 d]) in chloroform (100 ml) was added thionyl chloride (0.53 ml, 7.3 mmol) at 0 °C under an argon atmosphere. The solution was stirred at ambient temperature for 4 h. Additional thionyl chloride (1.6 ml, 22 mmol) was added at 0 °C and the solution was stirred for 12 h at ambient temperature. The mixture was poured onto ice water/aqueous NaHCO₃ 1/1, extracted two times with dichloromethane and the combined extracts were dried over sodium sulfate. Evaporation of the solvent under reduced pressure gave 2.15 g (quant.) of the title compound as colorless oil which was used in the next step without further purification.
MS: 291.0 (M+H)⁺.

### b] [2-Methyl-5-(4-trifluoromethoxy-phenyl)-2H-pyrazol-3-yl]-acetonitrile

Tetrabutylammonium cyanide (1.27 g, 4.7 mmol) was added to a solution of 5-chloromethyl-1-methyl-3-(4-trifluoromethoxy-phenyl)-1H-pyrazole (1.06 g, 3.7 mmol) in acetonitrile (24 ml). The solution was stirred at ambient temperature for 16 h, saturated aqueous sodium bicarbonate solution/ice water 1/1 and ethyl acetate were added and the layers were separated. The aqueous layer was extracted with ethyl acetate, the combined organic layers were washed with ice water/brine 1/1, dried over sodium sulfate and the solvent was evaporated in vacuo to give a red oil which was purified by column chromatography (silica gel, n-heptane/AcOEt) to yield 627 mg (2.2 mmol, 61 %) of the title compound as yellow crystals.
MS: 300.4 (M+NH₄)⁺.

### c] [2-Methyl-5-(4-trifluoromethoxy-phenyl)-2H-pyrazol-3-yl]-acetic acid

A mixture of [2-methyl-5-(4-trifluoromethoxy-phenyl)-2H-pyrazol-3-yl]-acetonitrile (620 mg, 2.2 mmol), sodium hydroxide (882 mg, 22 mmol), water (9 ml) and ethanol (9 ml) was stirred vigorously at 85 °C for 7 h. The reaction mixture was poured onto crushed ice and aqueous HCl and extracted three times with ethyl acetate. The combined extracts were washed with water and brine, and dried over anhydrous sodium sulfate. Evaporation of the solvent under reduced pressure gave 680 mg (2.26 mmol, quant.) of the title compound as off-white crystals.
MS: 301.0 (M+H)⁺.

### d] 2-[2-Methyl-5-(4-trifluoromethoxy-phenyl)-2H-pyrazol-3-yl]-ethanol

A solution of [2-methyl-5-(4-trifluoromethoxy-phenyl)-2H-pyrazol-3-yl]-acetic acid (680 mg, 2.7 mmol) in tetrahydrofuran (9.5 ml) was treated at 0 °C with a 1 M solution of BH₃*THF in tetrahydrofuran (5.7 ml, 5.7 mmol). The cooling bath was removed and the reaction mixture stirred at ambient temperature for 16 h. Careful quenching with MeOH and ice water, twofold extraction with AcOEt, washing with ice water/brine 1/1, drying over magnesium sulfate, and evaporation of the solvent left a crude product which was refluxed for 30 min in MeOH to liberate quantitatively the free alcohol. The solvent was evaporated in vacuo to yield a colorless oil which was purified by column chromatography (silica gel, n-heptane/AcOEt) to give 346 mg (1.2 mmol, 53 %) of the title compound as colorless crystals.
MS: 287.0 (M+H)⁺.

### e] 2-Methyl-2-(2-methyl-4-{2-[2-methyl-5-(4-trifluoromethoxy-phenyl)-2H-pyrazol-3-yl]-ethoxy}-phenoxy)-propionic acid ethyl ester

In analogy to the procedure described for example 1 e], 2-(4-hydroxy-2-methylphenoxy)-2-methyl-propionic acid ethyl ester (PCT Int. Appl. (2002), WO 2002092590 A1) was reacted with 2-[2-methyl-5-(4-trifluoromethoxy-phenyl)-2H-pyrazol-3-yl]-ethanol in the presence of di-tert-butyl azodicarboxylate and triphenylphosphine to give 2-methyl-2-(2-methyl-4-{2-[2-methyl-5-(4-trifluoromethoxy-phenyl)-2H-pyrazol-3-yl]-ethoxy}-phenoxy)-propionic acid ethyl ester as colorless oil.
MS: 507.5 (M+H)⁺.

### f] 2-Methyl-2-(2-methyl-4-{2-[2-methyl-5-(4-trifluoromethoxy-phenyl)-2H-pyrazol-3-yl]-ethoxy}-phenoxy)-propionic acid

In analogy to the procedure described in example 1 f], 2-methyl-2-(2-methyl-4-{2-[2-methyl-5-(4-trifluoromethoxy-phenyl)-2H-pyrazol-3-yl]-ethoxy}-phenoxy)-propionic acid ethyl ester was treated with LiOH to obtain 2-methyl-2-(2-methyl-4-{2-[2-methyl-5-(4-trifluoromethoxy-phenyl)-2H-pyrazol-3-yl]-ethoxy}-phenoxy)-propionic acid as yellow crystals.
MS: 479.4 (M+H)⁺.

### Example 3

### a] 2-Methyl-2-(3-{2-[2-methyl-5-(4-trifluoromethoxy-phenyl)-2H-pyrazol-3-yl] - ethoxy}-phenoxy)-propionic acid ethyl ester

In analogy to the procedure described for example 1 e], 2-(3-hydroxy-phenoxy)-2-methyl-propionic acid ethyl ester (PCT Int. Appl. (2001), WO 20010161120 A1) was reacted with 2-[2-methyl-5-(4-trifluoromethoxy-phenyl)-2H-pyrazol-3-yl]-ethanol (example 2 d]) in the presence of di-tert-butyl azodicarboxylate and triphenylphosphine to give 2-methyl-2-(3-{2-[2-methyl-5-(4-trifluoromethoxy-phenyl)-2H-pyrazol-3-yl] - ethoxy}-phenoxy)-propionic acid ethyl ester as colorless oil.
MS: 493.5 (M+H)⁺.

### b] 2-Methyl-2-(3-{2-[2-methyl-5-(4-trifluoromethoxy-phenyl)-2H-pyrazol-3-yl]-ethoxy}-phenoxy)-propionic acid

In analogy to the procedure described in example 1f], 2-methyl-2-(3-{2-[2-methyl-5-(4-trifluoromethoxy-phenyl)-2H-pyrazol-3-yl]-ethoxy}-phenoxy)-propionic acid ethyl ester was treated with LiOH to obtain 2-methyl-2-(3-{2-[2-methyl-5-(4-trifluoromethoxy-phenyl)-2H-pyrazol-3-yl]-ethoxy}-phenoxy)-propionic acid as colorless foam.
MS: 465.3 (M+H)⁺.

### Example 4

### a] 3-{2-Methoxy-4-[2-methyl-5-(4-trifluoromethoxy-phenyl)-2H-pyrazol-3-ylmethoxy]-phenyl}-propionic acid ethyl ester

In analogy to the procedure described for example 1 e], 3-(4-hydroxy-2-methoxyphenyl)-propionic acid ethyl ester (PCT Int. Appl. (2003), WO 2004000315 A1) was reacted with [2-methyl-5-(4-trifluoromethoxy-phenyl)-2H-pyrazol-3-yl] -methanol (example 1 d]) in the presence of N,N,N',N'-tetramethyl azodicarboxamide and tributylphosphine to give 3-{2-methoxy-4-[2-methyl-5-(4-trifluoromethoxy-phenyl)-2H-pyrazol-3-ylmethoxy]-phenyl}-propionic acid ethyl ester as colorless liquid.
MS: 479.4 (M+H)⁺.

### b] 3-{2-Methoxy-4-[2-methyl-5-(4-trifluoromethoxy-phenyl)-2H-pyrazol-3-ylmethoxy]-phenyl}-propionic acid

In analogy to the procedure described for example 1 f], 3-{2-methoxy-4-[2-methyl-5-(4-trifluoromethoxy-phenyl)-2H-pyrazol-3-ylmethoxy]-phenyl}-propionic acid ethyl ester was treated with LiOH to obtain 3-{2-methoxy-4-[2-methyl-5-(4-trifluoromethoxyphenyl)-2H-pyrazol-3-ylmethoxy]-phenyl}-propionic acid as colorless foam.
MS: 451.1 (M+H)⁺.

### Example 5

### a] 2-(4-Hydroxy-2,3-dimethyl-phenoxy)-2-methyl-propionic acid ethyl ester

A suspension of 8.0 g (57.9 mmol) 2,3-dimethylhydroquinone and 39.6 g (121.6 mmol) cesium carbonate in 100 ml DMF was treated with 9.45 ml (63.7 mmol) ethyl 2-bromo-2-methylpropionate and stirred for 2 days at RT. The reaction was poured on a mixture of saturated NH₄Cl-solution and ice and extracted with AcOEt (3x). The organic phase was washed with aqueous 10 % NaCl, dried (Na₂SO₄) and evaporated. The crude product was purified by flash chromatography.over silica gel with heptane/AcOEt 9:1, to give 5.4 g of the title compound as dark brown oil.
MS: 252.1 (M⁺).

### b] 2-{2,3-Dimethyl-4-[2-methyl-5-(4-trifluoromethoxy-phenyl)-2H-pyrazol-3-ylmethoxy]-phenoxy}-2-methyl-propionic acid ethyl ester

In analogy to the procedure described for example 1 e], 2-(4-hydroxy-2,3-dimethylphenoxy)-2-methyl-propionic acid ethyl ester was reacted with [2-methyl-5-(4-trifluoromethoxy-phenyl)-2H-pyrazol-3-yl] -methanol (example 1 d]) in the presence of N,N,N',N'-tetramethyl azodicarboxamide and tributylphosphine to give 2-{2,3-dimethyl-4-[2-methyl-5-(4-trifluoromethoxy-phenyl)-2H-pyrazol-3-ylmethoxy]-phenoxy}-2-methyl-propionic acid ethyl ester as colorless liquid.
MS: 507.5 (M+H)⁺.

### c] 2-{2,3-Dimethyl-4-[2-methyl-5-(4-trifluoromethoxy-phenyl)-2H-pyrazol-3-ylmethoxy]-phenoxy}-2-methyl-propionic acid

In analogy to the procedure described for example 1 f], 2-{2,3-dimethyl-4-[2-methyl-5-(4-trifluoromethoxy-phenyl)-2H-pyrazol-3-ylmethoxy] -phenoxy}-2-methyl-propionic acid ethyl ester was treated with LiOH to obtain 2-{2,3-dimethyl-4-[2-methyl-5-(4-trifluoromethoxy-phenyl)-2H-pyrazol-3-ylmethoxy]-phenoxy}-2-methyl-propionic acid as colorless foam.
MS: 479.4 (M+H)⁺.

### Example 6

### a] 2-(2,2,2-Trifluoro-ethyl)-5-(4-trifluoromethoxy-phenyl)-2H-pyrazole-3-carboxylic acid ethyl ester

Sodium hydride (55 % dispersion in mineral oil, 96 mg, 2 mmol) was added to an ice cooled solution of 5-(4-trifluoromethoxy-phenyl)-1H -pyrazole-3-carboxylic acid ethyl ester (500 mg, 2 mmol; example 1 b]) in DMF (30 ml) under an argon atmosphere. The solution was stirred for 10 min at 0 °C and for 40 min at ambient temperature. Trifluoroethyltriflate (506 mg, 2 mmol) was added and the mixture was stirred for 3 h at ambient temperature. The solution was cooled to 0 °C, 1 N HCl/ice water 1/2 and dichloromethane were added. The layers were separated, the aqueous layer was extracted two times with dichloromethane, the combined extracts were washed with brine/ice water 1/1 and dried over sodium sulfate. Evaporation of the solvent under reduced pressure gave a yellow oil which was purified by column chromatography (silica gel, n-heptane/AcOEt) to yield 553 mg (1.5 mmol, 87 %) of the title compound as colorless crystals.
MS: 382.1 (M)⁺.

### b] [2-(2,2,2-Trifluoro-ethyl)-5-(4-trifluoromethoxy-phenyl)-2H-pyrazol-3-yl]-methanol

In analogy to the procedure described for example 1 d], 2-(2,2,2-trifluoro-ethyl)-5-(4-trifluoromethoxy-phenyl)-2H-pyrazole-3-carboxylic acid ethyl ester was reduced with lithium aluminium hydride to give [2-(2,2,2-trifluoro-ethyl)-5-(4-trifluoromethoxyphenyl)-2H-pyrazol-3-yl]-methanol as colorless crystals.
MS: 341.0 (M+H)⁺.

### c] 2-Methyl-2-{2-methyl-4-[2-(2,2,2-trifluoro-ethyl)-5-(4-trifluoromethoxy-phenyl)-2H-pyrazol-3-ylmethoxy]-phenoxy}-propionic acid ethyl ester

In analogy to the procedure described for example 1e], 2-(4-hydroxy-2-methylphenoxy)-2-methyl-propionic acid ethyl ester (PCT Int. Appl. (2002), WO 2002092590 A1) was reacted with [2-(2,2,2-trifluoro-ethyl)-5-(4-trifluoromethoxy-phenyl)-2H-pyrazol-3-yl]-methanol in the presence of N,N,N',N'-tetramethyl azodicarboxamide and tributylphosphine to give 2-methyl-2-{2-methyl-4-[2-(2,2,2-trifluoro-ethyl)-5-(4-trifluoromethoxy-phenyl)-2H-pyrazol-3-ylmethoxy] -phenoxy}-propionic acid ethyl ester as yellow liquid.
MS: 561.3 (M+H)⁺.

### d] 2-Methyl-2-{2-methyl-4-[(2,2,2-trifluoro-ethyl)-5-(4-trifluoromethoxy-phenyl)-2H-pyrazol-3-ylmethoxy]-phenoxy}-propionic acid

In analogy to the procedure described for example 1 f], 2-methyl-2-{2-methyl-4-[2-(2,2,2-trifluoro-ethyl)-5-(4-trifluoromethoxy-phenyl)-2H-pyrazol-3-ylmethoxy] - phenoxy}-propionic acid ethyl ester was treated with LiOH to obtain 2-methyl-2-{2-methyl-4- [2-(2,2,2-trifluoro-ethyl)-5-(4-trifluoromethoxy-phenyl)-2H-pyrazol-3-ylmethoxy]-phenoxy}-propionic acid as yellow solid.
MS: 533.5 (M+H)⁺.

### Example 7

### a] [1-Methyl-5-(4-trifluoromethoxy-phenyl)-1H-pyrazol-3-yl]-methanol

In analogy to the procedure described for example 1 d], 1-methyl-5-(4-trifluoromethoxy-phenyl)-1H-pyrazole-3-carboxylic acid ethyl ester (example 1 c]) was reduced with lithium aluminium hydride to give [1-methyl-5-(4-trifluoromethoxyphenyl)-1H-pyrazol-3-yl]-methanol as brown solid.
MS: 273.0 (M+H)⁺.

### b] 2-Methyl-2-{2-methyl-4-[1-methyl-5-(4-trifluoromethoxy-phenyl)-1H-pyrazol-3-ylmethoxy]-phenoxy}-propionic acid ethyl ester

In analogy to the procedure described for example 1 e], 2-(4-hydroxy-2-methylphenoxy)-2-methyl-propionic acid ethyl ester (PCT Int. Appl. (2002), WO 2002092590 A1) was reacted with [1-methyl-5-(4-trifluoromethoxy-phenyl)-1H-pyrazol-3-yl]-methanol in the presence of N,N,N',N'-tetramethyl azodicarboxamide and tributylphosphine to give 2-methyl-2-{2-methyl-4-[1-methyl-5-(4-trifluoromethoxyphenyl)-1H-pyrazol-3-ylmethoxy]-phenoxy}-propionic acid ethyl ester as colorless oil.
MS: 493.5 (M+H)⁺.

### c] 2-Methyl-2-{2-methyl-4-[1-methyl-5-(4-trifluoromethoxy-phenyl)-1H-pyrazol-3-ylmethoxy]-phenoxy}-propionic acid

In analogy to the procedure described for example 1f], 2-methyl-2-{2-methyl-4-[1-methyl-5-(4-trifluoromethoxy-phenyl)-1H-pyrazol-3-ylmethoxy]-phenoxy}-propionic acid ethyl ester was treated with LiOH to obtain 2-methyl-2-{2-methyl-4-[1-methyl-5-(4-trifluoromethoxy-phenyl)-1H-pyrazol-3-ylmethoxy]-phenoxyl}-propionic acid as yellow oil.
MS: 465.3 (M+H)⁺.

### Example 8

### a] Benzoic acid 2,5-dichloro-4-(1-ethoxycarbonyl-1-methyl-ethoxy)-phenyl ester

To a suspension of benzoic acid 2,5-dichloro-4-hydroxy-phenyl ester (1.4 g, 5 mmol; D. Koike, Gunma Daigaku Kyoyobu Kiyo 1968, 2, 13-28), cesium carbonate (2.6 g, 7.9 mmol) and a trace of potassium iodide in acetonitrile (80 ml) under an argon atmosphere was added bromo-acetic acid ethyl ester (1.1 ml, 7.4 mmol). The mixture was stirred for 14 h at ambient temperature, poured onto 1 N HCl/ice water 1/1 and extracted two times with ethyl acetate. The combined organic layers were washed with brine/water 1/1 and dried over sodium sulfate. The solvent was removed under reduced pressure and the residue purified by column chromatography (silica gel, heptane/AcOEt) to give 0.5 g (1.3 mmol, 25 %) of the title compound as colorless oil.
MS: 396.1 (M)⁺.

### b] 2-(2,5-Dichloro-4-hydroxy-phenoxy)-2-methyl-propionic acid methyl ester

To an ice cold solution of benzoic acid 2,5-dichloro-4-(1-ethoxycarbonyl-1-methylethoxy)-phenyl ester (500 mg, 1.3 mmol) in methanol (11.5 ml) was added a solution of sodium (145 mg, 6.3 mmol) in methanol (11.5 ml) within 5 min under an argon atmosphere. The solution was stirred for 4 h at ambient temperature, cooled to 0 °C and carefully neutralized with 1 NHCl. The solvent was removed under reduced pressure and the residue was dissolved in ethyl acetate and brine/ice water 1/1. The layers were separated and the aqueous layer was extracted two times with ethyl acetate. The combined organic layers were washed with brine/ice water 1/1 and dried over sodium sulfate. The solvent was removed under reduced pressure and the residue purified by column chromatography (silica gel, heptane/AcOEt) to give 219 mg (0.8 mmol, 62 %) of the title compound as colorless oil.
MS: 279.1 (M+H)⁺.

### c] 2-{2,5-Dichloro-4-[2-methyl-5-(4-trifluoromethoxy-phenyl)-2H-pyrazol-3-ylmethoxy]-phenoxy}-2-methyl-propionic acid methyl ester

In analogy to the procedure described for example 1 e], 2-(2,5-dichloro-4-hydroxyphenoxy)-2-methyl-propionic acid methyl ester was reacted with [2-methyl-5-(4-trifluoromethoxy-phenyl)-2H-pyrazol-3-yl]-methanol (example 1 d]) in the presence of N,N,N',N'-tetramethyl azodicarboxamide and tributylphosphine to give 2-{2,5-dichloro-4- [2-methyl-5-(4-trifluoromethoxy-phenyl)-2H-pyrazol-3-ylmethoxy]-phenoxy}-2-methyl-propionic acid methyl ester as colorless oil.
MS: 533.3 (M+H)⁺.

### d] 2-{2,5-Dichloro-4-[2-methyl-5-(4-trifluoromethoxy-phenyl)-2H-pyrazol-3-ylmethoxy]-phenoxyl-2-methyl-propionic acid

In analogy to the procedure described for example 1 f], 2-{2,5-dichloro-4-[2-methyl-5-(4-trifluoromethoxy-phenyl)-2H-pyrazol-3-ylmethoxy]-phenoxy}-2-methyl-propionic acid methyl ester was treated with LiOH to obtain 2-{2,5-dichloro-4-[2-methyl-5-(4-trifluoromethoxy-phenyl)-2H-pyrazol-3-ylmethoxy]-phenoxy}-2-methyl-propionic acid as colorless solid.
MS: 517.1 (M-H)⁻.

### Example 9

### a] 2-Difluoromethyl-5-(4-trifluoromethoxy-phenyl)-2H-pyrazole-3-carboxylic acid ethyl ester and 1-Difluoromethyl-5-(4-trifluoromethoxy-phenyl-1H-pyrazole-3-carboxylic acid ethyl ester

Chlorodifluoromethane (28.6 g, 331 mmol) was introduced to a suspension of 5-(4-trifluoromethoxy-phenyl)-1H-pyrazole-3-carboxylic acid ethyl ester (2 g, 7 mmol; example 1 b]) and anhydrous potassium carbonate (2.76 g, 20 mmol) in dry N,N-dimethylformamide (120 ml). The reaction mixture was stirred at 90°C for 2 h. After cooling, the mixture was poured into ice water (400 ml) and extracted four times with dichloromethane. The combined extracts were washed two times with ice water/brine and dried over sodium sulfate. The solvent was removed under reduced pressure to give a yellow solid which was purified by column chromatography (silica gel, heptane/AcOEt) to give 281 mg (0.8 mmol, 12 %) 2-difluoromethyl-5-(4-trifluoromethoxy-phenyl)-2H-pyrazole-3-carboxylic acid ethyl ester as white solid and 1.29 g (3.7 mmol, 55 %) 1-difluoromethyl-5-(4-trifluoromethoxy-phenyl)-1H-pyrazole-3-carboxylic acid ethyl ester as white solid.

2-difluoromethyl-5-(4-trifluoromethoxy-phenyl)-2H-pyrazole-3-carboxylic acid ethyl ester: MS: 351.3 (M+H)⁺.

1-difluoromethyl-5-(4-trifluoromethoxy-phenyl)-1H-pyrazole-3-carboxylic acid ethyl ester: MS: 351.3 (M+H)⁺.

### b] [1-Difluoromethyl-5-(4-trifluoromethoxy-phenyl)-1H-pyrazol-3-yl]-methanol

In analogy to the procedure described for example 1 d], 1-difluoromethyl-5-(4-trifluoromethoxy-phenyl)-1H-pyrazole-3-carboxylic acid ethyl ester was reduced with lithium aluminium hydride to give [1-difluoromethyl-5-(4-trifluoromethoxy-phenyl)-1H-pyrazol-3-yl]-methanol as yellow oil.

### c] 2-{4-[1-Difluoromethyl-5-(4-trifluoromethoxy-phenyl)-1H-pyrazol-3-ylmethoxyl-2-methyl-phenoxy}-2-methyl-propionic acid ethyl ester

In analogy to the procedure described for example 1 e], 2-(4-hydroxy-2-methylphenoxy)-2-methyl-propionic acid ethyl ester (PCT Int. Appl. (2002), WO 2002092590 A1) was reacted with [1-difluoromethyl-5-(4-trifluoromethoxy-phenyl)-1H-pyrazol-3-yl]-methanol in the presence of N,N,N',N'-tetramethyl azodicarboxamide and tributylphosphine to give 2-{4-[1-difluoromethyl-5-(4-trifluoromethoxy-phenyl)-1H-pyrazol-3-ylmethoxy]-2-methyl-phenoxy}-2-methyl-propionic acid ethyl ester as colorless oil.
MS: 529.2 (M+H)⁺_{.}

### d] 2-{4-[1-Difluoromethyl-5-(4-trifluoromethoxy-phenyl)-1H-pyrazol-3-ylmethoxy]-2-methyl-phenoxy}-2-methyl-propionic acid

In analogy to the procedure described for example 1 f], 2-{4-[1-difluoromethyl-5-(4-trifluoromethoxy-phenyl)-1H-pyrazol-3-ylmethoxy] -2-methyl-phenoxy}-2-methyl-propionic acid ethyl ester was treated with LiOH to obtain 2-{4-[1-difluoromethyl-5-(4-trifluoromethoxy-phenyl)-1H-pyrazol-3-ylmethoxy]-2-methyl-phenoxyl-2-methylpropionic acid as orange oil.
MS: 501.1 (M+H)⁺.

### Example 10

### a] 5-Iodomethyl-1-methyl-3-(4-trifluoromethoxy-phenyl)-1H-pyrazole

A suspension of 5-chloromethyl-1-methyl-3-(4-trifluoromethoxy-phenyl)-1H-pyrazole (3.2 g, 11 mmol; example 2 a]) and sodium iodide (8.25 g, 55 mmol) in acetone (56 ml) was heated under reflux conditions for 30 min. Tert butyl methyl ether was added, the solid was filtered off and the filtrate was brought to dryness under reduced pressure. The residue was dissolved in tert butyl methyl ether, washed with ice water/brine 1/1 and the aqueous layer was extracted two times with tert butyl methyl ether. The combined extracts were washed with aqueous sodium thiosulfate solution and brine and dried over sodium sulfate. The solvent was removed under reduced pressure to give the title compound as yellow oil which was used in the next step without further purification.

### b] 3-[2-Methyl-5-(4-trifluoromethoxy-phenyl)-2H-pyrazol-3-yl]-propionic acid ethyl ester

A solution of lithium diisopropylamide (16.5 ml of a 2 M solution in tetrahydrofuran/heptane/ethylbenzol, 33 mmol) in tetrahydrofuran (25 ml) was cooled to -78°C. Within 30 min a solution of ethyl acetate (3.77 ml, 38 mmol) in tetrahydrofuran (10 ml) was added. The solution was stirred for 45 min at -78°C, DMPU (6.63 ml, 55 mmol) was added within 20 min and the mixture was kept for additional 30 min at -78°C. Within 20 min a solution of 5-iodomethyl-1-methyl-3-(4-trifluoromethoxy-phenyl)-1H-pyrazole (4.2 g,11 mmol) in tetrahydrofuran (25 ml) was added. The solution was stirred for 40 min at -78 °C, the cooling bath was removed and stirring was continued for 1 h. The reaction mixture was poored onto aqueous NH₄Cl solution/ice water and extracted two times with ethyl acetate. The combined extracts were washed three times with ice water/brine and dried over sodium sulfate. The solvent was removed under reduced pressure to give an orange oil which was purified by column chromatography (silica gel, heptane/AcOEt) to give 1.5 g (4.4 mmol, 40 %) of the title compound as yellow oil.
MS: 343.1 (M+H)⁺.

### c] 3-[2-Methyl-5-(4-trifluoromethoxy-phenyl)-2H-pyrazol-3-yl]-propan-1-ol

In analogy to the procedure described for example 1 d], 3-[2-methyl-5-(4-trifluoromethoxy-phenyl)-2H-pyrazol-3-yl]-propionic acid ethyl ester was reduced with lithium aluminium hydride in diethyl ether to give 3-[2-methyl-5-(4-trifluoromethoxyphenyl)-2H-pyrazol-3-yl]-propan-1-ol as yellow oil.
MS: 300.2 (M)⁺.

### d] 2-Methyl-2-(2-methyl-4-{3-[2-methyl-5-(4-trifluoromethoxy-phenyl)-2H-pyrazol-3-yl]-propoxy}-phenoxy)-propionic acid ethyl ester

In analogy to the procedure described for example 1 e], 2-(4-hydroxy-2-methylphenoxy)-2-methyl-propionic acid ethyl ester (PCT Int. Appl. (2002), WO 2002092590 A1) was reacted with 3-[2-methyl-5-(4-trifluoromethoxy-phenyl)-2H-pyrazol-3-yl]-propan-1-ol in the presence of N,N,N',N'-tetramethyl azodicarboxamide and tributylphosphine to give 2-methyl-2-(2-methyl-4-{3-[2-methyl-5-(4-trifluoromethoxyphenyl)-2H-pyrazol-3-yl]-propoxy}-phenoxy)-propionic acid ethyl ester as colorless oil.
MS: 521.5 (M+H)⁺.

### e] 2-Methyl-2-(2-methyl-4-{3-[2-methyl-5-(4-trifluoromethoxy-phenyl)-2H-pyrazol-3-yl]-propoxy}-phenoxy)-propionic acid

In analogy to the procedure described for example 1 f], 2-methyl-2-(2-methyl-4-{3-[2-methyl-5-(4-trifluoromethoxy-phenyl)-2H-pyrazol-3-yl]-propoxy}-phenoxy)-propionic acid ethyl ester was treated with LiOH to obtain 2-methyl-2-(2-methyl-4-{3-[2-methyl-5-(4-trifluoromethoxy-phenyl)-2H-pyrazol-3-yl]-propoxy}-phenoxy)-propionic acid as colorless solid.
MS: 493.5 (M+H)⁺.

### Example 11

### a] 2-(2,5-Dichloro-4-{2-[2-methyl-5-(4-trifluoromethoxy-phenyl)-2H-pyrazol-3-yl]-ethoxy}-phenoxy)- 2-methyl-propionic acid methyl ester

In analogy to the procedure described for example e], 2-(2,5-dichloro-4-hydroxyphenoxy)-2-methyl-propionic acid methyl ester (example 8 b]) was reacted with 2-[2-methyl-5-(4-trifluoromethoxy-phenyl)-2H-pyrazol-3-yl]-ethanol in the presence of di-tert-butyl azodicarboxylate and triphenylphosphine to give 2-(2,5-dichloro-4-{2-[2-methyl-5-(4-trifluoromethoxy-phenyl)-2H-pyrazol-3-yl] -ethoxy}-phenoxy)-2-methyl-propionic acid methyl ester as colorless oil.
MS: 547.3 (M+H)⁺.

### b] 2-(2,5-Dichloro-4-{2-[2-methyl-5-(4-trifluoromethoxy-phenyl)-2H-pyrazol-3-yl]-ethoxy}-phenoxy)-2-methyl-propionic acid

In analogy to the procedure described in example 1 f], 2-(2,5-dichloro-4-{2-[2-methyl-5-(4-trifluoromethoxy-phenyl)-2H-pyrazol-3-yl]-ethoxy}-phenoxy)-2-methyl-propionic acid methyl ester was treated with LiOH to obtain 2-(2,5-dichloro-4-{2-[2-methyl-5-(4-trifluoromethoxy-phenyl)-2H-pyrazol-3-yl]-ethoxy}-phenoxy)-2-methyl-propionic acid as colorless solid.
MS: 533.3 (M+H)⁺.

### Example 12

### a] 5-Chloromethyl-1-(2,2,2-trifluoro-ethyl)-3-(4-trifluoromethoxy-phenyl)-1H-pyrazole

In analogy to the procedure described for example 2 a], [2-(2,2,2-trifluoro-ethyl)-5-(4-trifluoromethoxy-phenyl)-2H-pyrazol-3-yl]-methanol (example 6 b]) was reacted with thionyl chloride in chloroform to yield 5-chloromethyl-1-(2,2,2-trifluoro-ethyl)-3-(4-trifluoromethoxy-phenyl)-1H-pyrazole as colorless oil.
MS: 359.0 (M+H)⁺.

### b] [2-(2,2,2-Trifluoro-ethyl)-5-(4-trifluoromethoxy-phenyl)-2H-pyrazol-3-yl]-acetonitrile

In analogy to the procedure described for example 2 b], 5-chloromethyl-1-(2,2,2-trifluoro-ethyl)-3-(4-trifluoromethoxy-phenyl)-1H-pyrazole was reacted with tetrabutylammonium cyanide in acetonitrile to give [2-(2,2,2-trifluoro-ethyl)-5-(4-trifluoromethoxy-phenyl)-2H-pyrazol-3-yl]-acetonitrile as yellow oil.
MS: 350.3 (M+NH₄)⁺.

### c] [2-(2,2,2-Trifluoro-ethyl)-5-(4-trifluoromethoxy-phenyl)-2H-pyrazol-3-yl]-acetic acid

In analogy to the procedure described for example 2 c], [2-(2,2,2-trifluoro-ethyl)-5-(4-trifluoromethoxy-phenyl)-2H-pyrazol-3-yl]-acetonitrile was treated with sodium hydroxide in water/ethanol 1/1 at 85°C to give [2-(2,2,2-trifluoro-ethyl)-5-(4-trifluoromethoxy-phenyl)-2H-pyrazol-3-yl]-acetic acid as brown crystals.
MS: 369.1 (M+H)⁺.

### d] 2-[2,2,2-Trifluoro-ethyl)-5-(4-trifluoromethoxy-pheny)-2H-pyrazol-3-yl-ethanol

In analogy to the procedure described for example 2 d], [2-(2,2,2-trifluoro-ethyl)-5-(4-trifluoromethoxy-phenyl)-2H-pyrazol-3-yl]-acetic acid was reduced with a 1M solution of BH₃*THF in tetrahydrofuran to give 2-[2-(2,2,2-trifluoro-ethyl)-5-(4-trifluoromethoxy-phenyl)-2H-pyrazol-3-yl]-ethanol as colorless oil.
MS: 355.3 (M+H)⁺.

### e] 2-Methyl-2-(2-methyl-4-{2-[2-(2,2,2-trifluoro-ethyl)-5-(4-trifluoromethoxy-phenyl) 2H-pyrazol-3-yl]-ethoxy}-phenoxy)-propionic acid ethyl ester

In analogy to the procedure described for example 1 e], 2-(4-hydroxy-2-methylphenoxy)-2-methyl-propionic acid ethyl ester (PCT Int. Appl. (2002), WO 2002092590 A1) was reacted with 2-[2-(2,2,2-trifluoro-ethyl)-5-(4-trifluoromethoxy-phenyl)-2H-pyrazol-3-yl]-ethanol in the presence of di-tert-butyl azodicarboxylate and triphenylphosphine to give 2-methyl-2-(2-methyl-4-{2-[2-(2,2,2-trifluoro-ethyl)-5-(4-trifluoromethoxy-phenyl)-2H-pyrazol-3-yl]-ethoxy}-phenoxy)-propionic acid ethyl ester as colorless oil.
MS: 575.5 (M+NH₄)⁺.

### f] 2-Methyl-2-(2-methyl-4-{2-[2-(2,2,2-trifluoro-ethyl)-5-(4-trifluoromethoxy-phenyl)-2H-pyrazol-3-yl]-ethoxy}-phenoxy)-propionic acid

In analogy to the procedure described in example 1 f], 2-methyl-2-(2-methyl-4-{2-[2-(2,2,2-trifluoro-ethyl)-5-(4-trifluoromethoxy-phenyl)-2H-pyrazol-3-yl]-ethoxy}phenoxy)-propionic acid ethyl ester was treated with LiOH to obtain 2-methyl-2-(2-methyl-4-{2-[2-(2,2,2-trifluoro-ethyl)-5-(4-trifluoromethoxy-phenyl)-2H-pyrazol-3-yl] ethoxy}-phenoxy)-propionic acid as colorless oil.
MS: 547.3 (M+H)⁺.

### Example 13

### a] 1-(4-Hydroxy-2-methoxy-5-methyl-phenyl)-ethanone

Acetylchloride (1.16 ml, 16 mmol) was added within 5 min to a ice cooled suspension of AlCl₃ (2.4 g, 16.4 mmol) in 1,2-dichloroethane (5 ml) under an argon atmosphere. A solution of 5-methoxy-2-methyl-phenol (1.13 g, 8.2 mmol; PCT Int. Appl. (2003), WO 2003084916 A2) in 1,2-dichloroethane (2.4 ml) was added within 5 min. The mixture was naturally warmed to ambient temperature, poured after 4 h onto ice water and extracted twice with dichloromethane. The combined extracts were washed with ice water/0.5 M NaOH solution 1/1 and brine and dried over sodium sulfate. Removal of the solvent under reduced pressure gave a yellow oil which was dissolved in a mixture of 3.5 ml methanol, 7 ml THF and 7 ml 1 M LiOH solution. The solution was stirred for 30 min at ambient temperature and the solvent was partially removed under reduced pressure. Ice water/1 M HCl solution 1/1 was added and the solution was extracted twice with ethyl acetate. The combined extracts were washed with brine and dried over sodium sulfate. Evaporation of the solvent left a yellow solid which was crystallized from dichloromethane/methanol/heptane to yield 441 mg (2.45 mmol, 30 %) of the title compound as colorless crystals.
MS: 179.4 (M-H)⁻.

### b] 2-(4-Acetyl-5-methoxy-2-methyl-phenoxy)-2-methyl-propionic acid ethyl ester

A suspension of 1-(4-hydroxy-2-methoxy-5-methyl-phenyl)-ethanone (416 mg, 2.3 mmol), 2-bromo-2-methyl-propionic acid ethyl ester (0.69 ml, 4.6 mmol), cesium carbonate (1.58 g, 4.9 mmol) and a trace of potassium iodide in acetonitrile (25 ml) was heated under reflux conditions for 14 h. The mixture was poured onto 1 M HCl solution/ice water 1/1 and extracted two times with ethyl acetate. The combined extracts were washed with brine/ice water and dried over sodium sulfate. Removal of the solvent under reduced pressure gave a yellow oil which was purified by column chromatography (silica gel, heptane/AcOEt) to give 450 mg (1.5 mmol, 66 %) of the title compound as colorless oil.
MS: 295.5 (M+H)⁺.

### c] 2-(4-Acetoxy-5-methoxy-2-methyl-phenoxy)-2-methyl-propionic acid ethyl ester

A solution of 2-(4-acetyl-5-methoxy-2-methyl-phenoxy)-2-methyl-propionic acid ethyl ester (531 mg, 1.8 mmol), 3-chloroperbenzoic acid (545 mg, 3.2 mmol) and 4-toluenesulfonic acid (34 mg, 0.2 mmol) in dichloromethane (24 ml) was heated under reflux conditions for 72 h. The mixture was cooled to room temperature and washed two times with ice water/sodium iodide solution and two times with ice water/aqueous NaHSO₃ solution. The organic layer was dried over sodium sulfate, the solvent was removed under reduced pressure and the resulting brown solid was purified by column chromatography (silica gel, heptane/AcOEt) to give 235 mg (0.8 mmol, 42 %) of the title compound as yellow oil.
MS: 311.3 (M+H)⁺.

### d] 2-(4-Hydroxy-5-methoxy-2-methyl-phenoxy)-2-methyl-propionic acid methyl ester

A freshly prepared solution of sodium (91 mg, 4 mmol) in methanol (5.4 ml) was added within 5 min to an ice cooled solution of 2-(4-acetoxy-5-methoxy-2-methyl-phenoxy)-2-methyl-propionic acid ethyl ester (235 mg, 0.8 mmol) in methanol (5.4 ml). The solution was naturally warmed to ambient temperature and after 5 h the solvent was removed under reduced pressure. Ice water/1 M HCl 1/1 was added and the mixture was extracted two times with dichloromethane. The combined extracts were dried over sodium sulfate and the solvent was removed under reduced pressure to yield 153 mg (0.6 mmol, 76 %) of the title compound as brown oil which was used in the next step without further purification.
MS: 254.2 (M)⁺.

### e] 2-{5-Methoxy-2-methyl-4-[2-methyl-5-(4-trifluoromethoxy-phenyl)-2H-pyrazol-3-ylmethoxy]-phenoxy)-2-methyl-propionic acid methyl ester

In analogy to the procedure described for example 1 e], 2-(4-hydroxy-5-methoxy-2-methyl-phenoxy)-2-methyl-propionic acid methyl ester was reacted with [2-methyl-5-(4-trifluoromethoxy-phenyl)-2H-pyrazol-3-yl]-methanol (example 1 d]) in the presence of N,N,N',N'-tetramethyl azodicarboxamide and tributylphosphine to give 2-{5-methoxy-2-methyl-4-[2-methyl-5-(4-trifluoromethoxy-phenyl)-2H-pyrazol-3-ylmethoxy]-phenoxy}-2-methyl-propionic acid methyl ester as colorless oil.
MS: 509.5 (M+H)⁺.

### e] 2-{5-Methoxy-2-methyl-4-[2-methyl-5-(4-trifluoromethoxy-phenyl)-2H-pyrazol-3-ylmethoxy]-phenoxy}-2-methyl-propionic acid

In analogy to the procedure described for example 1 f], 2-{5-methoxy-2-methyl-4-[2-methyl-5-(4-trifluoromethoxy-phenyl)-2H-pyrazol-3-ylmethoxyl]-phenoxy}-2-methyl-propionic acid methyl ester was treated with LiOH to obtain 2-{5-methoxy-2-methyl-4-[2-methyl-5-(4-trifluoromethoxy-phenyl)-2H-pyrazol-3-ylmethoxyl]-phenoxy}-2-methyl-propionic acid as colorless oil.
MS: 495.5 (M+H)⁺.

### Example 14

### a] 2-(5-Methoxy-2-methyl-4-{3-[2-methyl-5-(4-trifluoromethoxy-phenyl)-2H-pyrazol-3-yl]-propoxy}-phenoxy)-2-methyl-propionic acid methyl ester

In analogy to the procedure described for example 1 e], 2-(4-hydroxy-5-methoxy-2-methyl-phenoxy)-2-methyl-propionic acid methyl ester (example 13 d]) was reacted with 3-[2-methyl-5-(4-trifluoromethoxy-phenyl)-2H-pyrazol-3-yl]-propan-1-ol (example 10 c]) in the presence of N,N,N',N'-tetramethyl azodicarboxamide and tributylphosphine to give 2-(5-methoxy-2-methyl-4-{3-[2-methyl-5-(4-trifluoromethoxy-phenyl)-2H-pyrazol-3-yl] -propoxy}-phenoxy)-2-methyl-propionic acid methyl ester as colorless oil.
MS: 537.2 (M+H)⁺.

### b] 2-(5-Methoxy-2-methyl-4-{3-[2-methyl-5-(4-trifluoromethoxy-phenyl)-2H-pyrazol-3-yl]-propoxy}-phenoxy)-2-methyl-propionic acid

In analogy to the procedure described for example 1 f], 2-(5-methoxy-2-methyl-4-{3-[2-methyl-5-(4-trifluoromethoxy-phenyl)-2H-pyrazol-3-yl]-propoxyl-phenoxy)-2-methyl-propionic acid methyl ester was treated with LiOH to obtain 2-(5-methoxy-2-methyl-4-{3-[2-methyl-5-(4-trifluoromethoxy-phenyl)-2H-pyrazol-3-yl] -propoxy}-phenoxy)-2-methyl-propionic acid as colorless oil.
MS: 523.5 (M+H)⁺.

### Example 15

### a] [2-Difluoromethyl-5-(4-trifluoromethoxy-phenyl)-2H-pyrazol-3-yl]-methanol

In analogy to the procedure described for example 1 d], 2-difluoromethyl-5-(4-trifluoromethoxy-phenyl)-2H-pyrazole-3-carboxylic acid ethyl ester (example 9 a]) was reduced with lithium aluminium hydride to give [2-difluoromethyl-5-(4-trifluoromethoxy-phenyl)-2H-pyrazol-3-yl]-methanol as white solid.
MS: 309.4 (M+H)⁺.

### b] 2-{4-[2-Difluoromethyl-5-(4-trifluoromethoxy-phenyl)-2H-pyrazol-3-ylmethoxy]-2-methyl-phenoxy}-2-methyl-propionic acid ethyl ester

In analogy to the procedure described for example 1 e], 2-(4-hydroxy-2-methylphenoxy)-2-methyl-propionic acid ethyl ester (PCT Int. Appl. (2002), WO 2002092590 A1) was reacted with [2-difluoromethyl-5-(4-trifluoromethoxy-phenyl)-2H-pyrazol-3-yl]-methanol in the presence of N,N,N',N'-tetramethyl azodicarboxamide and tributylphosphine to give 2-{4-[2-difluoromethyl-5-(4-trifluoromethoxy-phenyl)-2H-pyrazol-3-ylmethoxy]-2-methyl-phenoxy}-2-methyl-propionic acid ethyl ester as yellow oil.
MS: 529.3 (M+H)⁺_{.}

### c] 2-{4-[2-Difluoromethyl-5-(4-trifluoromethoxy-phenyl)-2H-pyrazol-3-ylmethoxy]-2-methyl-phenoxy}-2-methyl-propionic acid

In analogy to the procedure described for example 1 f], 2-{4-[2-difluoromethyl-5-(4-trifluoromethoxy-phenyl)-2H-pyrazol-3-ylmethoxy] -2-methyl-phenoxy}-2-methyl-propionic acid ethyl ester was treated with LiOH to obtain 2-{4-[2-difluoromethyl-5-(4-trifluoromethoxy-phenyl)-2H-pyrazol-3-ylmethoxy] -2-methyl-phenoxy}-2-methyl-propionic acid as colorless oil.
MS: 501.4 (M+H)⁺.

### Example A

Film coated tablets containing the following ingredients can be manufactured in a conventional manner:

| Ingredients | Per tablet | |
|---|---|---|
| Kernel: | | |
| Compound of formula (I) | 10.0 mg | 200.0 mg |
| Microcrystalline cellulose | 23.5 mg | 43.5 mg |
| Lactose hydrous | 60.0 mg | 70.0 mg |
| Povidone K30 | 12.5 mg | 15.0 mg |
| Sodium starch glycolate | 12.5 mg | 17.0 mg |
| Magnesium stearate | 1.5 mg | 4.5 mg |
| (Kernel Weight) | 120.0 mg | 350.0 mg |

| Film Coat: | | |
|---|---|---|
| Hydroxypropyl methyl cellulose | 3.5 mg | 7.0 mg |
| Polyethylene glycol 6000 | 0.8 mg | 1.6 mg |
| Talc | 1.3 mg | 2.6 mg |
| Iron oxyde (yellow) | 0.8 mg | 1.6 mg |
| Titan dioxide | 0.8 mg | 1.6 mg |

The active ingredient is sieved and mixed with microcristalline cellulose and the mixture is granulated with a solution of polyvinylpyrrolidon in water. The granulate is mixed with sodium starch glycolate and magesiumstearate and compressed to yield kernels of 120 or 350 mg respectively. The kernels are lacquered with an aqueous solution / suspension of the above mentioned film coat.

### Example B

Capsules containing the following ingredients can be manufactured in a conventional manner:

| Ingredients | Per capsule |
|---|---|
| Compound of formula (I) | 25.0 mg |
| Lactose | 150.0 mg |
| Maize starch | 20.0 mg |
| Talc | 5.0 mg |

The components are sieved and mixed and filled into capsules of size 2.

### Example C

Injection solutions can have the following composition:

| | |
|---|---|
| Compound of formula (I) | 3.0 mg |
| Gelatine | 150.0 mg |
| Phenol | 4.7 mg |
| Sodium carbonate | to obtain a final pH of 7 |
| Water for injection solutions | ad 1.0 ml |

### Example D

Soft gelatin capsules containing the following ingredients can be manufactured in a conventional manner:

| **Capsule contents** | |
|---|---|
| Compound of formula (I) | 5.0 mg |
| Yellow wax | 8.0 mg |
| Hydrogenated Soya bean oil | 8.0 mg |
| Partially hydrogenated plant oils | 34.0 mg |
| Soya bean oil | 110.0 mg |
| Weight of capsule contents | 165.0 mg |

| **Gelatin capsule** | |
|---|---|
| Gelatin | 75.0 mg |
| Glycerol 85 % | 32.0 mg |
| Karion 83 | 8.0 mg (dry matter) |
| Titan dioxide | 0.4 mg |
| Iron oxide yellow | 1.1 mg |

The active ingredient is dissolved in a warm melting of the other ingredients and the mixture is filled into soft gelatin capsules of appropriate size. The filled soft gelatin capsules are treated according to the usual procedures.

### Example E

Sachets containing the following ingredients can be manufactured in a conventional manner:

| | |
|---|---|
| Compound of formula (I) | 50.0 mg |
| Lactose, fine powder | 1015.0 mg |
| Microcristalline cellulose (AVICEL PH 102) | 1400.0 mg |
| Sodium carboxymethyl cellulose | 14.0 mg |
| Polyvinylpyrrolidon K 30 | 10.0 mg |
| Magnesiumstearate | 10.0 mg |
| Flavoring additives | 1.0 mg |

The active ingredient is mixed with lactose, microcristalline cellulose and sodium carboxymethyl cellulose and granulated with a mixture of polyvinylpyrrolidon in water. The granulate is mixed with magnesiumstearate and the flavouring additives and filled into sachets.

## Claims

1. Compounds of the formula 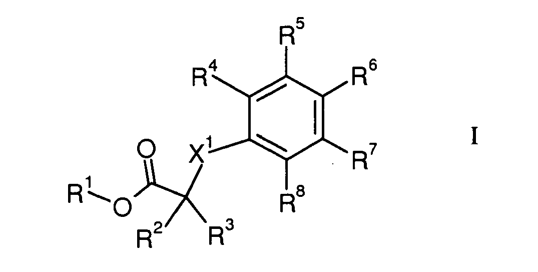 wherein
X¹ is selected from the group consisting of O, S and CH₂;
R¹ is hydrogen or C₁₋₇-alkyl;
R² is hydrogen or C₁₋₇-alkyl,
or, if X¹ is CH₂, R² is selected from the group consisting of hydrogen, C₁₋₇-alkyl and C₁₋₇-alkoxy;
R³ is hydrogen or C₁₋₇-alkyl;
R⁴ and R⁸ independently from each other are selected from the group consisting of hydrogen, C₁₋₇-alkyl, C₁₋₇-alkoxy, C₃₋₇-cycloalkyl, halogen, C₁₋₇-alkoxy- C₁₋₇-alkyl, C₂₋₇-alkenyl, C₂₋₇-alkinyl, fluoro-C₁₋₇-alkyl, fluoro-C₁₋₇-alkoxy, cyano-C₁₋₇-alkyl and cyano;
R⁵, R⁶ and R⁷ independently from each other are selected from the group consisting of hydrogen, C₁₋₇-alkyl, C₁₋₇-alkoxy, C₃₋₇-cycloalkyl, halogen, C₁₋₇-alkoxy- C₁₋₇-alkyl, C₂₋₇-alkenyl, C₂₋₇-alkinyl, fluoro-C₁₋₇-alkyl, fluoro-C₁₋₇-alkoxy, cyano-C₁₋₇-alkyl and cyano;
and one of R⁵, R⁶ and R⁷ is 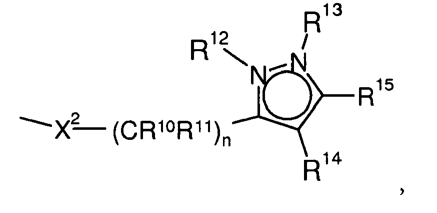 wherein
X² is S, O and NR⁹,
R⁹ is selected from the group consisting of hydrogen, C₁₋₇-alkyl, C₃₋₇-cycloalkyl, fluoro-C₁₋₇-alkyl, hydroxy-C₂₋₇-alkyl and C₁₋₇-alkoxy-C₂₋₇-alkyl;
R¹⁰ is selected from the group consisting of hydrogen, C₁₋₇-alkyl, C₃₋₇-cycloalkyl and fluoro-C₁₋₇-alkyl;
R¹¹ is selected from the group consisting of hydrogen, C₁₋₇-alkyl and C₁₋₇-alkoxy-C₁₋₇-alkyl;
one of R¹² or R¹³ is selected from the group consisting of hydrogen, C₁₋₇-alkyl, C₃₋₇-cycloalkyl, C₂₋₇-alkoxy-C₁₋₇-alkyl, C₂₋₇-alkenyl, C₂₋₇-alkinyl and fluoro-C₁₋₇-alkyl; and the other one is a lone pair;
R¹⁴ is hydrogen, C₁₋₇-alkyl, C₃₋₇-cycloalkyl, halogen, C₁₋₇-alkoxy-C₁₋₇-alkyl, C₂₋₇-alkenyl, C₂₋₇-alkinyl or fluoro-C₁₋₇-alkyl;
R¹⁵ is 4-trifluoromethoxyphenyl;
n is 1, 2 or 3; and
pharmaceutically acceptable salts thereof.

2. Compounds of formula I according to claim 1 having the formula 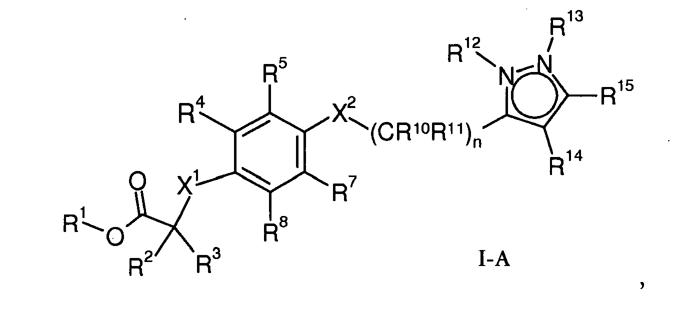 wherein
X¹, X², R¹ to R⁴, R⁸, R¹⁰ to R¹⁵ and n are as defined in claim 1;
R⁵ and R⁷ independently from each other are selected from the group consisting of hydrogen,C ₁₋₇-alkyl,C₁₋₇-alkoxy,C₃₋₇-cycloalkyl, halogen, C₁₋₇-alkoxy- C₁₋₇-alkyl, C₂₋₇-alkenyl, C₂₋₇-alkinyl, fluoro-C₁₋₇alkyl, fluoro-C₁₋₇-alkoxy, cyano-C₁₋₇-alkyl and cyano; and
and pharmaceutically acceptable salts thereof.

3. Compounds of formula I-A according to claim 2, wherein at least one of R⁴, R⁵, R⁷ and R⁸ is C₁₋₇-alkyl or C₁₋₇-alkoxy.

4. Compounds of formula I-A according to claim 3, wherein R⁴ is C₁₋₇-alkyl or C₁₋₇-₇-alkoxy.

5. Compounds of formula I according to claim 1 having the formula 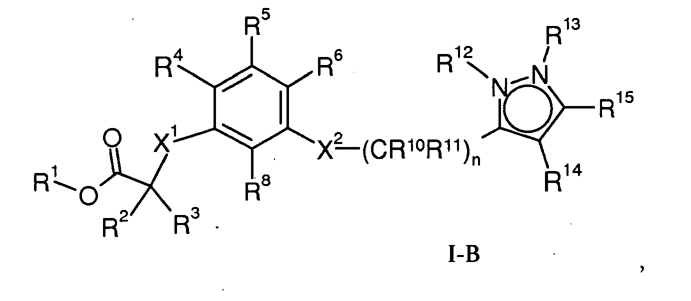 wherein
X¹, X², R¹ to R⁴, R⁸, R¹⁰ to R¹⁵ and n are as defined in claim 1;
R⁵ and R⁶ independently from each other are selected from the group consisting of hydrogen,C₁₋₇-alkyl,C₁₋₇-alkoxy,C₃₋₇-cycloalkyl, halogen,C₁₋₇-alkoxy- C₁₋₇-alkyl, C₂₋₇-alkenyl, C₂₋₇-alkinyl, fluoro-C₁₋₇-alkyl, fluoro-C₁₋₇-alkoxy, cyano-C₁₋₇-alkyl and cyano; and
pharmaceutically acceptable salts thereof.

6. Compounds of formula I-B according to claim 5, wherein at least one of R⁴, R⁵, R⁶ and R⁸ is C₁₋₇-alkyl or C₁₋₇-alkoxy.

7. Compounds of formula I according to claim 1 having the formula 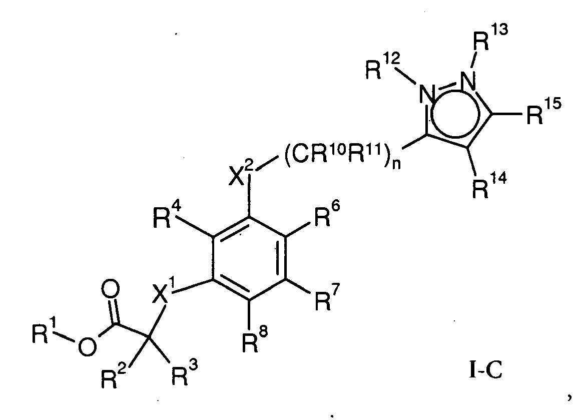 wherein
X¹, X², R¹ to R⁴, R⁸, R¹⁰ to R¹⁵ and n are as defined in claim 1;
R⁶ and R⁷ independently from each other are selected from the group consisting of hydrogen, C₁₋₇-alkyl, C₁₋₇-alkoxy, C₃₋₇-cycloalkyl, halogen, C₁₋₇-alkoxy- C₁₋₇-alkyl, C₂₋₇-alkenyl, C₂₋₇-alkinyl, fluoro-C₁₋₇-alkyl, fluoro-C₁₋₇-alkoxy, cyano-C₁₋₇-alkyl and cyano; and
pharmaceutically acceptable salts thereof.

8. Compounds of formula I-C according to claim 6, wherein at least one of R⁴, R⁶, R⁷ and R⁸ is C₁₋₇-alkyl or C₁₋₇-alkoxy.

9. Compounds of formula I according to any one of claims 1 to 8, wherein R¹ is hydrogen.

10. Compounds of formula I according to any one of claims 1 to 9, wherein X¹ is O.

11. Compounds of formula I according to claim 10, wherein R² and R³ are C₁₋₇-alkyl.

12. Compounds of formula I according to any one of claims 1 to 9, wherein X¹ is CH₂.

13. Compounds of formula I according to any one of claims 1 to 12, wherein X² is O.

14. Compounds of formula I according to any one of claims 1 to 13, wherein n is 1 or 2.

15. Compounds of formula I according to any one of claims 1 to 14, wherein n is 2.

16. Compounds of formula I according to any one of claims 1 to 13, wherein n is 3.

17. Compounds of formula I according to any one of claims 1 to 14, wherein one of R⁵, R⁶ and R⁷ is 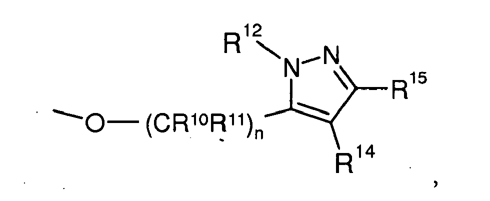 and R¹⁰ to R¹², R¹⁴, R¹⁵ and n are as defined in claim 1.

18. Compounds of formula I according to claim 15, wherein R¹² is C₁₋₇-alkyl or fluoro-C₁₋₇-alkyl.

19. Compounds of formula I according to claim 1, selected from the group consisting of
2-methyl-2-{2-methyl-4-[2-methyl-5-(4-trifluoromethoxy-phenyl)-2H-pyrazol-3-ylmethoxy]-phenoxy}-propionic acid,
2-methyl-2- (2-methyl-4-{2- [2-methyl-5-(4-trifluoromethoxy-phenyl) -2H-pyrazol-3-yl] - ethoxy}-phenoxy)-propionic acid,
2-methyl-2-(3-{2- [2-methyl-5-(4-trifluoromethoxy-phenyl)-2H-pyrazol-3-yl]-ethoxy}-phenoxy)-propionic acid,
3-{2-methoxy-4-[2-methyl-5-(4-trifluoromethoxy-phenyl)-2H-pyrazol-3-ylmethoxy]-phenyl}-propionic acid,
2-{2,3-dimethyl-4-[2-methyl-5-(4-trifluoromethoxy-phenyl)-2H-pyrazol-3-ylmethoxy]-phenoxy}-2-methyl-propionic acid,
2-methyl-2-{2-methyl-4-[2-(2,2,2-trifluoro-ethyl)-5-(4-trifluoromethoxy-phenyl)-2H-pyrazol-3-ylmethoxy]-phenoxy}-propionic acid,
2-methyl-2-{2-methyl-4-[1-methyl-5-(4-trifluoromethoxy-phenyl)-1H-pyrazol-3-ylmethoxy] -phenoxy}-propionic acid,
2-{2,5-dichloro-4- [2-methyl-5-(4-trifluoromethoxy-phenyl)-2H-pyrazol-3-ylmethoxy] - phenoxy}-2-methyl-propionic acid,
2-{4- [ 1-difluoromethyl-5-(4-trifluoromethoxy-phenyl)-1 H-pyrazol-3-ylmethoxy]-2-methyl-phenoxy}-2-methyl-propionic acid,
2-methyl-2-(2-methyl-4-{3-[2-methyl-5-(4-trifluoromethoxy-phenyl)-2H-pyrazol-3-yl]-propoxy}-phenoxy)-propionic acid,
2-(2,5-dichloro-4-{ 2- [2-methyl-5-(4-trifluoromethoxy-phenyl)-2H-pyrazol-3-yl] - ethoxy}-phenoxy)-2-methyl-propionic acid,
2-methyl-2-(2-methyl-4-{2-[2-(2,2,2-trifluoro-ethyl)-5-(4-trifluoromethoxy-phenyl)-2H-pyrazol-3-yl]-ethoxy}-phenoxy)-propionic acid,
2-{ 5-methoxy-2-methyl-4-[2-methyl-5-(4-trifluoromethoxy-phenyl)-2H-pyrazol-3-ylmethoxy]-phenoxy}-2-methyl-propionic acid,
2-(5-methoxy-2-methyl-4-{3-[2-methyl-5-(4-trifluoromethoxy-phenyl)-2H-pyrazol-3-yl]-propoxy}-phenoxy)-2-methyl-propionic acid,
2-{4- [2-difluoromethyl-5-(4-trifluoromethoxy-phenyl)-2H-pyrazol-3-ylmethoxy]-2-methyl-phenoxy}-2-methyl-propionic acid, and
pharmaceutically acceptable salts thereof.

20. Compounds of formula I according to claim 1, selected from the group consisting of
2-methyl-2-{2-methyl-4-[2-methyl-5-(4-trifluoromethoxy-phenyl)-2H-pyrazol-3-ylmethoxy] phenoxy}-propionic acid,
2-methyl-2-(2-methyl-4-{2-[2-methyl-5-(4-trifluoromethoxy-phenyl)-2H-pyrazol-3-yl]-ethoxy}-phenoxy)-propionic acid,
2-methyl-2-(2-methyl-4-{3-[2-methyl-5-(4-trifluoromethoxy-phenyl)-2H-pyrazol-3-yl]-propoxy}-phenoxy)-propionic acid,
2-(5-methoxy-2-methyl-4-{3-[2-methyl-5-(4-trifluoromethoxy-phenyl)-2H-pyrazol-3-yl]-propoxy}-phenoxy)-2-methyl-propionic acid, and
pharmaceutically acceptable salts thereof.

21. A process for the manufacture of compounds according to any one of claims 1 to 20, which process comprises
reacting a compound of formula 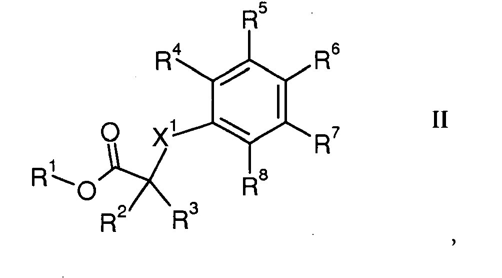 wherein R¹ is C₁₋₇-alkyl, R², R³, R⁴ and R⁸ are as defined as in claim 1 and R⁵, R⁶ and R⁷ are selected from hydrogen,C₁₋₇-alkyl,C₁₋₇-alkoxy,C₃₋₇-Cycloalkyl, halogen,C₁₋₇-alkoxy-C₁₋₇-alkyl, C₂₋₇-alkenyl, C₂₋₇-alkinyl, fluoro-C₁₋₇-alkyl, fluoro-C₁₋₇-alkoxy, cyano-C₁₋₇-alkyl, and cyano with the proviso that one of R⁵, R⁶ or R⁷ is -OH, -SH or -NHR⁹, wherein R⁹ is as defined in claim 1,
with a compound of formula 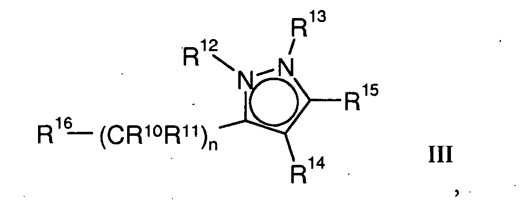 wherein R¹⁰ to R¹⁵ and n are as defined in claim 1 and R¹⁶ is -OH, -Cl, -Br, -I or another leaving group, to obtain a compound of formula 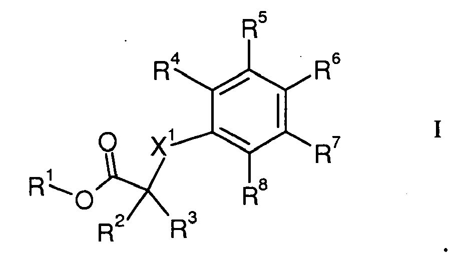 wherein R¹ is C₁₋₇-alkyl and X¹, R² to R⁸ are as defined in claim 1,
and optionally hydrolysing the ester group to obtain a compound of formula I, wherein R¹ is hydrogen.

22. Pharmaceutical compositions comprising a compound according to any one of claims 1 to 20 as well as a pharmaceutically acceptable carrier and/or adjuvant.

23. Pharmaceutical compositions according to claim 22 for the treatment and/or prevention of diseases which are modulated by PPARδ and/or PPARα agonists.

24. Compounds according to any one of claims 1 to 20 for use as therapeutically active substances.

25. Compounds according to any one of claims 1 to 20 for use as therapeutically active substances for the treatment and/or prevention of diseases which are modulated by PPARδ and/or PPARα agonists.

26. The use of compounds according to any one of claims 1 to 20 for the preparation of medicaments for the treatment and/or prevention of diseases which are modulated by PPARδ and/or PPARα agonists.

27. The use according to claim 26 for the treatment and/or prevention of diabetes, non-insulin dependent diabetes mellitus, increased lipid and cholesterol levels, particularly low HDL-cholesterol, high LDL-cholesterol, or high triglyceride levels, atherosclerotic diseases, metabolic syndrome, syndrome X, obesity, elevated blood pressure, endothelial dysfunction, procoagulant state, dyslipidemia, polycystic ovary syndrome, inflammatory diseases, and proliferative diseases.

28. The use according to claim 27 for the treatment and/or prevention of low HDL cholesterol levels, high LDL cholesterol levels, high triglyceride levels, metabolic syndrome and syndrome X.

## Patentansprüche

1. Verbindungen der Formel 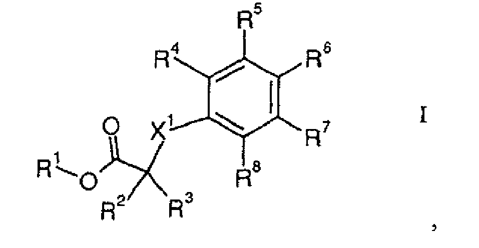 worin
X¹ ausgewählt ist aus der Gruppe, bestehend aus O, S und CH₂;
R¹ Wasserstoff oder C₁₋₇-Alkyl ist;
R² Wasserstoff oder C₁₋₇-Alkyl ist, oder,
wenn X¹ CH₂ ist, R² ausgewählt ist aus der Gruppe, bestehend aus Wasserstoff, C₁₋₇-Alkyl und C₁₋₇-Alkoxy,
R³ Wasserstoff oder C₁₋₇-Alkyl ist;
R⁴ und R⁸ unabhängig voneinander ausgewählt sind aus der Gruppe, bestehend aus Wasserstoff, C₁₋₇-Alkyl, C₁₋₇-Alkoxykoxy, C₃₋₇-Cycloalkyl, Halogen, C₁₋₇-Alkoxy-C₁₋₇-alkyl, C₂₋₇-Alkenyl, C₂₋₇-Alkinyl, Fluor-C₁₋₇-alkyl, Fluor-C₁₋₇-alkoxy, Cyano-C₁₋₇-alkyl und Cyano;
R⁵, R⁶ und R⁷ unabhängig voneinander ausgewählt sind aus der Gruppe, bestehend aus Wasserstoff, C₁₋₇-Alkyl, C₁₋₇-Alkoxy, C₃₋₇-Cycloalkyl, Halogen, C₁₋₇-Alkoxy-C₁₋₇-alkyl, C₂₋₇-Alkenyl, C₂₋₇-Alkinyl, Fluor-C₁₋₇-alkyl, Fluor-C₁₋₇-alkoxy, Cyano-C₁₋₇-alkyl und Cyano;
und einer von R⁵, R⁶ und R⁷ 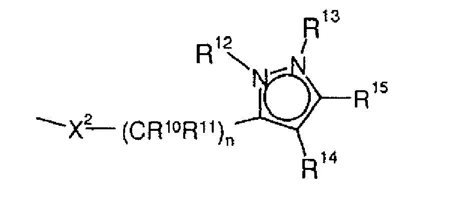 ist, worin
X² S, O und NR⁹ ist,
R⁹ ausgewählt ist aus der Gruppe, bestehend aus Wasserstoff, C₁₋₇-Alkyl, C₃₋₇-Cycloalkyl, Fluor-C₁₋₇-aklkyl, Hydroxy-C₂₋₇-alkyl und C₁₋₇-Alkoxy-C₂₋₇-alkyl,
R¹⁰ ausgewählt ist aus der Gruppe, bestehend aus Wasserstoff, C₁₋₇-Alkyl, C₃₋₇-Cycloalkyl und Fluor-C₁₋₇alkyl;
R¹¹ ausgewählt ist aus der Gruppe, bestehend aus Wasserstoff, C₁₋₇Alkyl und C₁₋₇-Alkoxy-C₁₋₇-alkyl;
einer von R¹² oder R¹³ ausgewählt ist aus der Gruppe, bestehend aus Wasserstoff, C₁₋₇-Alkyl, C₃₋₇-Cycloalkyl, C₂₋₇-Alkoxy-C₁₋₇-alkyl, C₂₋₇-Alkenyl, C₂₋₇-Alkinyl und Fluor-C₁₋₇-alkyl; und der andere ein freies Elektronenpaar ist;
R¹⁴ Wasserstoff, C₁₋₇-Alkyl, C₃₋₇-Cycloalkyl, Halogen, C₁₋₇-Alkoxy-C₁₋₇-alkyl, C₂₋₇-Alkenyl, C₂₋₇-Alkinyl oder Fluor-C₁₋₇-alkyl ist;
R¹⁵ 4-Trifluormethoxyphenyl ist;
n 1, 2 oder 3 ist;
und pharmazeutisch akzeptable Salze davon.

2. Verbindungen der Formel I nach Anspruch 1 mit der Formel 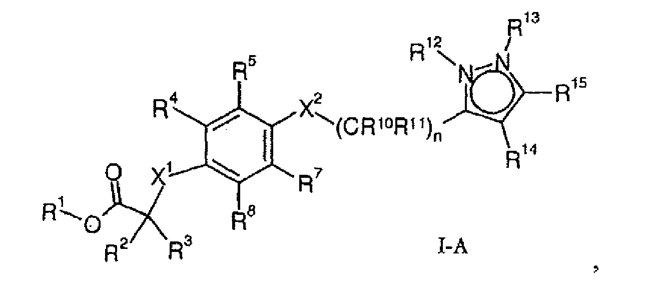 worin X¹, X², R¹ bis R⁴, R⁸, R¹⁰ bis R¹⁵ und n wie in Anspruch 1 definiert sind;
R⁵ und R⁷ unabhängig voneinander ausgewählt sind aus der Gruppe, bestehend aus Wasserstoff, C₁₋₇-Arkyl, C₁₋₇-Alkoxy, C₃₋₇-Cycloalkyl, Halogen, C₁₋₇-Alkoxy-C₁₋₇-alkyl, C₂₋₇-Alkenyl, C₂₋₇-Alkinyl, Fluor-C₁₋₇-alkyl, Fluor-C₁₋₇-alkoxy, Cyano-C₁₋₇-alkyl und Cyano;
und pharmazeutisch akzeptable Salze davon.

3. Verbindungen der Formel I-A nach Anspruch 2, worin mindestens einer von R⁴, R⁵, R⁷ und R⁸ C₁₋₇-Alkyl oder C₁₋₇-Alkoxy ist.

4. Verbindungen der Formel I-A nach Anspruch 3, worin R⁴ C₁₋₇-Alkyl oder C₁₋₇-Alkoxy ist.

5. Verbindungen der Formel I nach Anspruch 1 mit der Formel 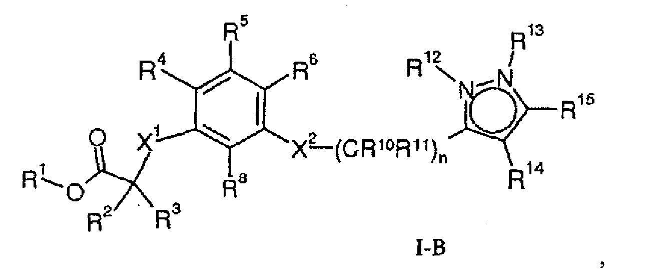 worin X¹, X², R¹ bis R⁴, R⁸, R¹⁰ bis R¹⁵ und n wie in Anspruch 1 definiert sind;
R⁵ und R⁶ unabhängig voneinander ausgewählt sind aus der Gruppe, bestehend aus Wasserstoff, C₁₋₇-Alkyl, C₁₋₇-Alkoxy, C₃₋₇-Cycloalkyl, Halogen, C₁₋₇-Alkoxy-C₁₋₇-alkyl, C₂₋₇-Alkenyl, C₂₋₇-Alkinyl, Fluor-C₁₋₇-alkyl, Fluor-C₁₋₇-alkoxy, Cyano-C₁₋₇-alkyl und Cyano;
und pharmazeutisch akzeptable Salze davon.

6. Verbindungen der Formel I-B nach Anspruch 5, worin mindestens einer von R⁴, R⁵, R⁶ und R⁸ C₁₋₇-Akyl oder C₁₋₇-Alkoxy ist.

7. Verbindungen der Formel I nach Anspruch 1 mit der Formel 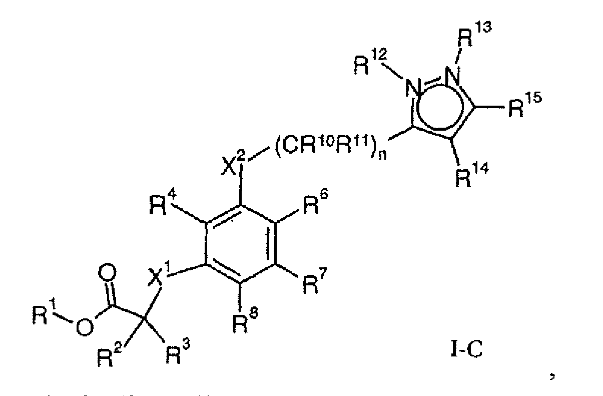 worin X¹, X², R¹ bis R⁴, R⁸, R¹⁰ bis R¹⁵ und n wie in Anspruch 1 definiert sind;
R⁶ und R⁷ unabhängig voneinander ausgewählt sind aus der Gruppe, bestehend aus Wasserstoff, C₁₋₇-Alkyl, C₁₋₇-Alkoxy, C₃₋₇-Cycloalkyl, Halogen, C₁₋₇-Alkoxy-C₁₋₇-alkyl, C₂₋₇-Alkenyl, C₂₋₇-Alkinyl, Fluor-C₁₋₇-alkyl, Fluor-C₁₋₇-alkoxy, Cyano-c₁₋₇-alkyl und Cyano;
und pharmazeutisch akzeptable Salze davon.

8. Verbindungen der Formel I-C nach Anspruch 6, worin mindestens einer von R⁴, R⁶, R⁷ und R⁸ C₁₋₇-Alkyl oder C₁₋₇-Alkoxy ist.

9. Verbindungen der Formel I nach einem der Ansprüche 1 bis 8, worin R¹ Wasserstoff ist.

10. Verbindungen der Formel I nach einem der Ansprüche 1 bis 9, worin X¹ O ist.

11. Verbindungen der Formel I nach Anspruch 10, worin R² und R³ C₁₋₇-Alkyl sind.

12. Verbindungen der Formel I nach einem der Ansprüche 1 bis 9, worin X¹ CH₂ ist.

13. Verbindungen der Formel I nach einem der Ansprüche 1 bis 12, worin X² O ist.

14. Verbindungen der Formel I nach einem der Ansprüche 1 bis 13, worin n 1 oder 2 ist.

15. Verbindungen der Formel I nach einem der Ansprüche 1 bis 14, worin n 2 ist.

16. Verbindungen der Formel I nach einem der Ansprüche 1 bis 13, worin n 3 ist.

17. Verbindungen der Formel I nach einem der Ansprüche 1 bis 14, worin einer von R⁵, R⁶ und R⁷ 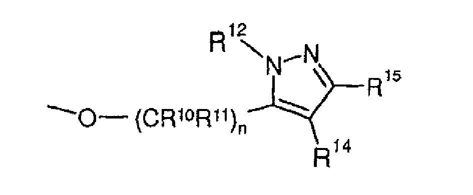 ist und
R¹⁰ bis R¹², R¹⁴, R¹⁵ und n wie in Anspruch 1 definiert sind.

18. Verbindungen der Formel I nach Anspruch 15, worin R¹² C₁₋₇-Alkyl oder Fluor-C₁₋₇-alkyl ist.

19. Verbindungen der Formel I nach Anspruch 1, ausgewählt aus der Gruppe, bestehend aus 2-Methyl-2-{2-methyl-4-[2-methyl-5-(4-trifluormethoxy-phenyl)-2H-pyrazol-3-ylmethoxy]-phenoxy}-propionsäure,
2-Methy-2-(2-methyl-4-{2-[2-methyl-5-(4-trifluormethoxy-phenyl)-2H-pyrazol-3-yl]ethoxy}-phenoxy)-propionsäure,
2-Methyl-2-(3-{2-[2-methyl-5-(4-trifluormethoxy-phenyl)-2H-pyrazol-3-yl]-ethoxy}-phenoxy)-propionsäure,
3-{2-Methoxy-4-[2-methyl-5-(4-trifluormethoxy-phenyl)-2H-pyrazol-3-ylmethoxy]-phenyl}-propionsäure,
2-{2,3-Dimethyl-4-[2-methyl-5-(4-trifluormethoxy-phenyl)-2H-pyrazol-3-ylmethoxy]-phenoxyl-2-methyl-propionsäure,
2-Methyl-2-{2-methyl-4-[2-(2,2,2-trifluor-ethyl)-5-(4-trifluormethoxy-phenyl)-2H-pyrazol-3-ylmethoxy]-phenoxy}-propionsäure,
2-ethyl-2-{2-methyl-4-[1-methyl-5-(4-trifluormethoxy-phenyl)-1H-pyrazol-3-ylmethoxy]-phenoxy}-propionsäure,
2-{2,5-Dichlor-4-[2-methyl-5-(4-trifluormethoxy-phenyl)-2H-pyrazol-3-ylmethoxy]-phenoxy}-2-methyl-propionsäure,
2-{4-[1-Difluormethyl-5-(4-trifluormethoxy-phenyl)-1H-pyrazol-3-ylmethoxy]-2-methylphenoxy}-2-methyl-propionsäure,
2-Methyl-2-(2-methyl-4-{3-[2-methyl-5-(4-trifluormethoxy-phenyl)-2H-pyrazol-3-yl]-propoxy}-phenoxy)-propionsäure,
2-(2,5-Dichlor-4-{2-[2-methyl-5-(4-trifluormethoxy-phenyl)-2H-pyrazol-3-yl]-ethoxy}-phenoxy)-2-methyl-propionsäure,
2-Methyl-2-(2-methyl-4-{2-[2-(2,2,2-trifluor-ethyl)-5-(4-trifluormethoxy-phenyl)-2H-pyrazol-3-yl]-ethoxy}-phenoxy)-propionsäure,
2-{5-Methoxy-2-methyl-4-[2-methyl-5-(4-trifluormethoxy-phenyl)-2H-pyrazol-3-ylmethoxy]-phenoxy}-2-methyl-propionsäure,
2-(5-Methoxy-2-methyl-4-{3-[2-methyl-5-(4-trifluormethoxy-phenyl)-2H-pyrazol-3-yl]-propoxy}-phenoxy)-2-methyl-propionsäure,
2-{4-[2-Difluormethyl-5-(4-trifluormethoxy-phenyl)-2H-pyrazol-3-ylmethoxy]-2-methylphenoxy}-2-methyl-propionsäure
und pharmazeutisch akzeptablen Salzen davon.

20. Verbindungen der Formel I nach Anspruch 1, ausgewählt aus der Gruppe, bestehend aus 2-Methyl-2-{2-methyl-4-[2-methyl-5-(4-trifluormethoxy-phenyl)-2H-pyrazol-3-ylmethoxy]-phenoxy}-propionsäure,
2-Methyl-2-(2-methyl-4-{2-[2-methyl-5-(4-trifluormethoxy-phenyl)-2H-pyrazol-3-yl]-ethoxy}-phenoxy)-propionsäure,
2-Methyl-2-(2-methyl-4-{3-[2-methyl-5-(4-trifluormethoxy-phenyl]-2H-pyrazol-3-yl]-propoxy}-phenoxy)-propionsäure,
2-(5-Methoxy-2-methyl-4-{3-[2-methyl-5-(4-trifluormethoxy-phenyl)-2H-pyrazol-3-yl]-propoxy}-phenoxy)-2-methyl-propionsäure
und pharmazeutisch akzeptablen Salzen davon.

21. Verfahren zur Herstellung von Verbindungen nach einem der Ansprüche 1 bis 20, wobei das Verfahren
das Umsetzen einer Verbindung der Formel 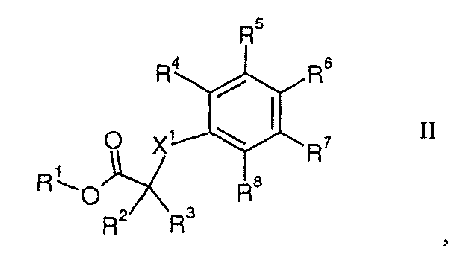 worin R¹ C₁₋₇-Alkyl ist, R², R³, R⁴ und R⁸ wie in Anspruch 1 definiert sind und R⁵, R⁶ und R⁷ aus Wasserstoff, C₁₋₇-Akyl, C₁₋₇-Alkoxy, C₃₋₇-Cycloalkyl, Halogen, C₁₋₇-Alkoxy-C₁₋₇-alkyl, C₁₋₇-Alkenyl, C₂₋₇-Alkinyl, Fluor-C₁₋₇-alkyl, Fluor-C₁₋₇-alkoxy, Cyano-C₁₋₇-alkyl und Cyano ausgewählt sind, mit der Maßgabe, daß einer von R⁵, R⁶ oder R⁷ -OH, -SH oder -NHR⁹ ist, wobei R⁹ wie in Anspruch 1 definiert ist,
mit einer Verbindung der Formel 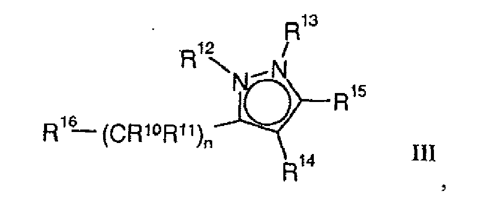 worin R¹⁰ bis R¹⁵ und n wie in Anspruch 1 definiert sind und R¹⁶ -OH, -Cl, -Br, -I odereine andere Abgangsgruppe ist,
unter Erhalt einer Verbindung der Formel 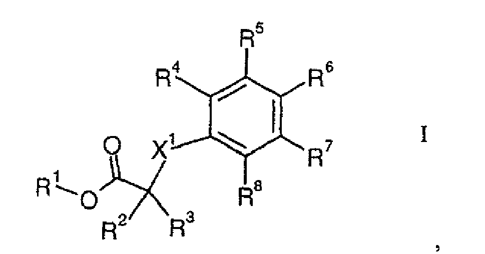 worin R¹ C₁₋₇-Alkyl ist und X¹, R² bis R⁸ wie in Anspruch 1 definiert sind, und gegebenenfalls das Hydrolysieren der Estergruppe unter Erhalt einer Verbindung der Formel I, worin R¹ Wasserstoff ist,
umfaßt.

22. Pharmazeutische Zusammensetzungen, umfassend eine Verbindung nach einem der Ansprüche 1 bis 20 sowie einen pharmazeutisch akzeptablen Träger und/oder ein pharmazeutisch akzeptables Adjuvans.

23. Pharmazeutische Zusammensetzungen nach Anspruch 22 zur Behandlung und/oder Vorbeugung von Krankheiten, die von PPARδ- und/oder PPARα-Agonisten moduliert werden.

24. Verbindungen nach einem der Ansprüche 1 bis 20 zur Verwendung als therapeutisch aktive Substanzen.

25. Verbindungen nach einem der Ansprüche 1 bis 20 zur Verwendung als therapeutisch aktive Substanzen zur Behandlung und/oder Vorbeugung von Krankheiten, die von PAARδ-und/oder PPARδ-Agonisten moduliert werden.

26. Verwendung von Verbindungen nach einem der Ansprüche 1 bis 20 zur Herstellung von Medikamenten zur Behandlung und/oder Vorbeugung von Krankheiten, die von PPARδ-und/oder PPARα-Agonisten moduliert werden.

27. Verwendung nach Anspruch 26 zur Behandlung und/oder Vorbeugung von Diabetes, nicht-insulinpflichtigem Diabetes mellitus, erhöhten Lipid- und Cholesterinspiegeln, insbesondere niedrigen HDL-Cholesterin-, hohen LDL-Cholesterin- oder hohen Triglyceridspiegeln, atherosklerotischen Krankheiten, des Stoffwechselsyndroms, Syndrom X, Fettleibigkeit, erhöhtem Blutdruck, Endotheldysfunktion, eines die Koagulation fördernden Zustandes, Dyslipidämie, des polyzystischen Ovarialsyndroms, Entzündungskrankheiten und proliferativen Krankheiten.

28. Verwendung nach Anspruch 27 zur Behandlung und/oder Vorbeugung von insbesondere niedrigen HDL-Cholesterinspiegeln, hohen LDL-Cholesterinspiegeln, hohen Triglyceridspiegeln, dem Stoffwechselsyndrom und Syndrom X.

## Revendications

1. Composés de formule 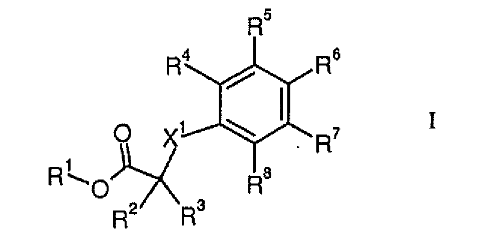 dans laquelle
X¹ est choisi dans le groupe consistant en O, S et un groupe CH₂ ;
R¹ représente un atome d'hydrogène ou un groupe alkyle en C₁ à C₇ ;
R² représente un atome d'hydrogène ou un groupe alkyle en C₁ à C₇,
ou, si X¹ représente un groupe CH₂, R² est choisi dans le groupe consistant en un atome d'hydrogène, des groupes alkyle en C₁ à C₇ et alkoxy en C₁ à C₇ ;
R³ représente un atome d'hydrogène ou un groupe alkyle en C₁ à C₇;
R⁴ et R⁸, indépendamment l'un de l'autre, sont choisis dans le groupe consistant en un atome d'hydrogène, des groupes alkyle en C₁ à C₇, alkoxy en C₁ à C₇, cycloalkyle en C₃ à C₇, halogéno, (alkoxy en C₁ à C₇) (alkyle en C₁ à C₇), alcényle en C₂ à C₇, alcynyle en C₂ à C₇, fluoro-alkyle en C₁ à C₇, fluoro-alkoxy en C₁ à C₇, cyano-alkyle en C₁ à C₇ et cyano ;
R⁵, R⁶ et R_{7,} indépendamment les uns des autres, sont choisis dans le groupe consistant en un atome d'hydrogène, des groupes alkyle en C₁ à C₇, alkoxy en C₁ à C_{7,} cycloalkyle en C₃ à C₇, halogéno, (alkoxy en C₁ à C₇) (alkyle en C₁ à C₇), alcényle en C₂ à C₇, alcynyle en C₂ à C₇, fluoro-alkyle en C₁ à C₇, fluoro-alkoxy en C₁ à C₇, cyano-alkyle en C₁ à C₇ et cyano ;
et un de R⁵, R⁶ et R⁷ représente un groupe 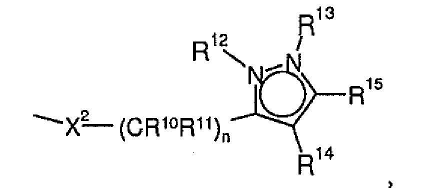 dans lequel
X² représente S, O ou un groupe NR⁹,
R⁹ est choisi dans le groupe consistant en un atome d'hydrogène, des groupes alkyle en C₁ à C₇, cycloalkyle en C₃ à C₇, fluoro-alkyle en C₁ à C₇, hydroxyalkyle en C₂ à C₇, et (alkoxy en C₁ à C₇) (alkyle en C₂ à C₇) ;
R¹⁰ est choisi dans le groupe consistant en un atome d'hydrogène, des groupes alkyle en C₁ à C₇, cycloalkyle en C₃ à C₇, et fluoro-alkyle en C₁ à C₇;
R¹¹ est choisi dans le groupe consistant en un atome d'hydrogène, des groupes alkyle en C₁ à C₇ et (alkoxy en C₁ à C₇) (alkyle en C₁ à C₇) ;
Un de R₁₂ et R¹³ est choisi dans le groupe consistant en un atome d'hydrogène, des groupes alkyle en C₁ à C₇, cycloalkyle en C₃ à C₇, (alkoxy en C₂ à C₇) (alkyle en C₁ à C₇), alcényle en C₂ à C₇, alcynyle en C₂ à C₇ et fluoro-alkyle en C₁ à C₇ ; et l'autre représente un doublet électronique libre ;
R¹⁴ représente un atome d'hydrogène, un groupe alkyle en C₁ à C₇, cycloalkyle en C₃ à C₇, halogéno, (alkoxy en C₁ à C₇) (alkyle en C₁ à C₇), alcényle en C₂ à C₇, alcynyle en C₂ à C₇ ou fluoro-alkyle en C₁ à C₇ ;
R¹⁵ représente un groupe 4-trifluorométhoxyphényle ;
n est égal à 1, 2 ou 3 ; et
leurs sels pharmaceutiquement acceptables.

2. Composés de formule I suivant la revendication 1, répondant à la formule 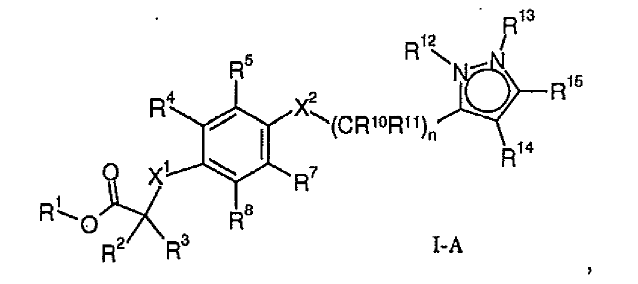 dans laquelle
X¹, X², R¹ à R⁴ , R⁸, R¹⁰ à R¹⁵ et n sont tels que définis dans la revendication 1 ;
R⁵ et R⁷, indépendamment l'un de l'autre, sont choisis dans le groupe consistant en un atome d'hydrogène, des groupes alkyle en C₁ à C₇, alkoxy en C₁ à C₇, cycloalkyle en C₃ à C₇, halogéno, (alkoxy en C₁ à C₇) (alkyle en C₁ à C₇), alcényle en C₂ à C₇, alcynyle en C₂ à C₇, fluoro-alkyle en C₁ à C₇, fluoro-alkoxy en C₁ à C₇, cyano-alkyle en C₁ à C₇ et cyano ; et
leurs sels pharmaceutiquement acceptables.

3. Composés de formule I-A suivant la revendication 2, dans lesquels au moins un de R⁴, R⁵ R⁷ et R⁸ représente un groupe alkyle en C₁ à C₇ ou alkoxy en C₁ à C₇.

4. Composés de formule I-A suivant la revendication 3, dans lesquels R⁴ représente un groupe alkyle en C₁ à C₇ ou alkoxy en C₁ à C₇.

5. Composés de formule I suivant la revendication 1, répondant à la formule 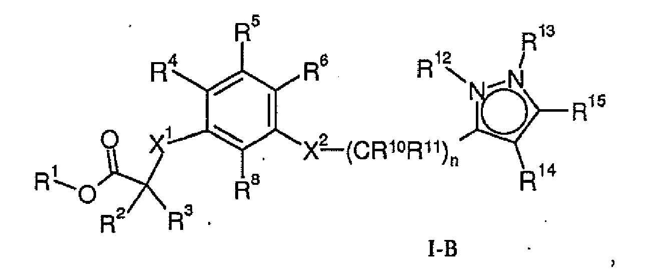 dans laquelle
X¹, X², R¹ à R⁴, R⁸, R¹⁰ à R¹⁵ et n sont tels que définis dans la revendication 1 ;
R⁵ et R⁶, indépendamment l'un de l'autre, sont choisis dans le groupe consistant en un atome d'hydrogène, des groupes alkyle en C₁ à C₇, alkoxy en C₁ à C₇, cycloalkyle en C₃ à C₇, halogéno, (alkoxy en C₁ à C₇) (alkyle en C₁ à C₇), alcényle en C₂ à C₇, alcynyle en C₂ à C₇, fluoro-alkyle en C₁ à C₇, fluoro-alkoxy en C₁ à C₇, cyano-alkyle en C₁ à C₇ et cyano ; et
leurs sels pharmaceutiquement acceptables.

6. Composés de formule I-B suivant la revendication 5, dans lesquels au moins un de R⁴, R⁵, R⁶ et R⁸ représente un groupe alkyle en C₁ à C₇ ou alkoxy en C₁ à C₇.

7. Composés de formule I suivant la revendication 1, répondant à la formule 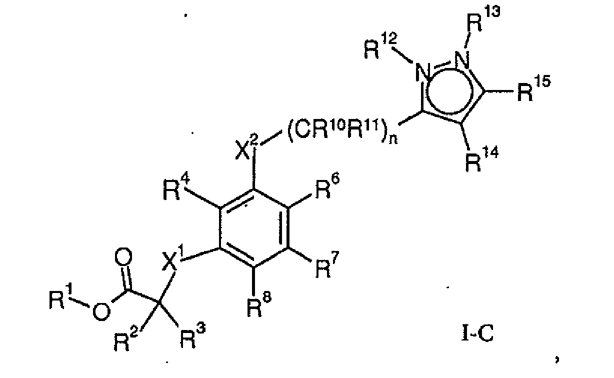 dans laquelle
X¹, X², R¹ à R⁴, R⁸, R¹⁰ à R¹⁵ et n sont tels que définis dans la revendication 1 ;
R⁶ et R⁷, indépendamment l'un de l'autre, sont choisis dans le groupe consistant en un atome d'hydrogène, des groupes alkyle en C₁ à C₇, alkoxy en C₁ à C₇, cycloalkyle en C₃ à C₇, halogéno, (alkoxy en C₁ à C₇) (alkyle en C₁ à C₇, alcényle en C₂ à C₇, alcynyle en C₂ à C₇, fluoro-alkyle en C₁ à C₇, fluoro-alkoxy en C₁ à C₇, cyano-alkyle en C₁ à C₇ et cyano ; et
leurs sels pharmaceutiquement acceptables.

8. Composés de formule I-C suivant la revendication 6, dans lesquels au moins un de R⁴, R⁶, R⁷ et R⁸ représente un groupe alkyle en C₁ à C₇ ou alkoxy en C₁ à C₇.

9. Composés de formule I suivant l'une quelconque des revendications 1 à 8, dans lesquels R¹ représente un atome d'hydrogène.

10. Composés de formule I suivant l'une quelconque des revendications 1 à 9, dans lesquels X¹ représente O.

11. Composés de formule I suivant la revendication 10, dans lesquels R² et R³ représentent des groupes alkyle en C₁ à C₇.

12. Composés de formule I suivant l'une quelconque des revendications 1 à 9, dans lesquels X¹ représente un groupe CH₂.

13. Composés de formule I suivant l'une quelconque des revendications 1 à 12, dans lesquels X² représente O.

14. Composés de formule I suivant l'une quelconque des revendications 1 à 13, dans lesquels n est égal à 1 ou 2.

15. Composés de formule I suivant l'une quelconque des revendications 1 à 14, dans lesquels n est égal à 2.

16. Composés de formule I suivant l'une quelconque des revendications 1 à 13, dans lesquels n est égal à 3.

17. Composés de formule I suivant l'une quelconque des revendications 1 à 14, dans lesquels un de R⁵, R⁶ et R⁷ représente un groupe 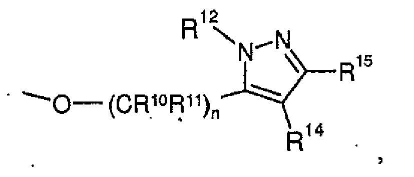 et R¹⁰ à R¹² R¹⁴, R¹⁵ et n sont tels que définis dans la revendication 1.

18. Composés de formule I suivant la revendication 15, dans lesquels R¹² représente un groupe alkyle en C₁ à C₇ ou fluoro-alkyle en C₁ à C₇.

19. Composés de formule I suivant la revendication 1, choisis dans le groupe consistant en :
l'acide 2-méthyl-2-{2-méthyl-4-[2-méthyl-5-(4-trifluorométhoxyphényl)-2H-pyrazole-3-ylméthoxy]phénoxy}-propionique,
l'acide 2-méthyl-2-(2-méthyl-4-{2-[2-méthyl-5-(4-trifluorométhoxyphényl)-2H-pyrazole-3-yl]éthoxy}phénoxy)-propionique,
l'acide 2-méthyl-2-(3-{2-[2-méthyl-5-(4-trifluorométhoxyphényl)-2H-pyrazole-3-yl]éthoxy}phénoxy)propionique,
l'acide 3-{2-méthoxy-4-[2-méthyl-5-(4-trifluorométhoxyphényl)-2H-pyrazole-3-ylméthoxy]phényl}propionique,
l'acide 2-{2,3-diméthyl-4-[2-méthyl-5-(4-trifluorométhoxyphényl)-2H-pyrazole-3-ylméthoxy]phénoxy}-2-méthylpropionique,
l'acide 2-méthyl-2-{2-méthyl-4-[2-(2,2,2-trifluoroéthyl)-5-(4-trifluorométhoxyphényl)-2H-pyrazole-3-ylméthoxy]-phénoxy}propionique,
l'acide 2-méthyl-2-{2-méthyl-4-[1-méthyl-5-(4-trifluorométhoxyphényl)-1H-pyrazole-3-ylméthoxy]phénoxy}-propionique,
l'acide 2-{2,5-dichloro-[2-méthyl-5-(4-trifluorométhoxyphényl)-2H-pyrazole-3-ylméthoxy]-phénoxy}-2-méthylpropionique,
l'acide 2-{4-[1-difluorométhyl-5-(4-trifluorométhoxyphényl)-1H-pyrazole-3-ylméthoxy]-2-méthylphénoxy}-2-méthylpropionique,
l'acide 2-méthyl-2-(2-méthyl-4-{3-[2-méthyl-5-(4-trifluorométhoxyphényl)-2H-pyrazole-3-yl]-propoxy}phénoxy)-propionique,
l'acide 2-(2,5-dichloro-4-{2-[2-méthyl-5-(4-trifluorométhoxyphényl)-2H-pyrazole-3-yl]-éthoxy}phénoxy)-2-méthylpropionique,
l'acide 2-méthyl-2-(2-méthyl-4-{2-(2,2,2-trifluoroéthyl)-5-(4-trifluorométhoxyphényl)-2H-pyrazole-3-yl]éthoxy}-phénoxy)propionique,
l'acide 2-{5-méthoxy-2-méthyl-4-[2-méthyl-5-(4-trifluoromëthoxyphényl)-2H-pyrazole-3-ylméthoxy]phénoxy}-2-méthylpropionique,
l'acide 2-(5-méthoxy-2-méthyl-4-{3-[2-méthyl-5-(4-trifluorométhoxyphényl)-2H-pyrazole-3-yl]propoxy}phénoxy)-2-méthylpropionique,
l'acide 2-{4-[2-difluorométhyl-5-(4-trifluorométhoxyphényl)-2H-pyrazole-3-ylméthoxy]-2-méthylphénoxy}-2-méthylpropionique, et
leurs sels pharmaceutiquement acceptables.

20. Composés de formule I suivant la revendication 1, choisis dans le groupe consistant en
l'acide 2-méthyl-2-{2-méthyl-4-[2-méthyl-5-(4-trifluoxométhoxyphényl)-2H-pyrazole-3-ylméthoxy]phénoxy}-propionique,
l'acide 2-méthyl-2-(2-méthyl-4-{2-[2-méthyl-5-(4-trifluorométhoxyphényl)-2H-pyrazole-3-yl]éthoxy}phénoxy)-propionique,
l'acide 2-méthyl-2-(2-méthyl-4-{3-[2-méthyl-5-(4-trifluorométhoxyphényl)-2H-pyrazole-3-yl]propoxy}phénoxy)-propionique,
l'acide 2-(5-méthoxy-2-méthyl-4-{3-[2-méthyl-5-(4-trifluorométhoxyphényl)-2H-pyrazole-3-yl]propoxy}phénoxy-2-méthylpropionique, et
leurs sels pharmaceutiquement acceptables.

21. Procédé pour la production de composés suivant l'une quelconque des revendications 1 à 20, procédé qui comprend
la réaction d'un composé de formule 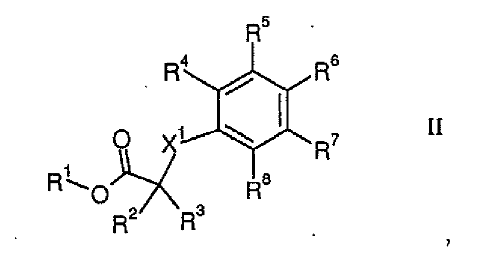 dans laquelle R¹ représente un groupe alkyle en C₁ à C₇, R² R³, R⁴ et R⁸ sont tels que définis dans la revendication 1 et R⁵, R⁶ et R⁷ sont choisis entre un atome d'hydrogène, des groupes alkyle en C₁ à C₇, alkoxy en C₁ à C₇, cycloalkyle en C₃ à C₇, halogéno, (alkoxy en C₁ à C₇) (alkyle en C₁ à C₇), alcényle en C₂ à C₇, alcynyle en C₂ à C₇, fluoro-alkyle en C₁ à C₇, fluoro-alkoxy en C₁ à C₇, cyano-alkyle en C₁ à C₇ et cyano, sous réserve qu'un de R⁵, R⁶ et R⁷ représente un groupe -OH, -SH ou -NHR⁹, dans lequel R⁹ est tel que défini dans la revendication 1,
avec un composé de formule 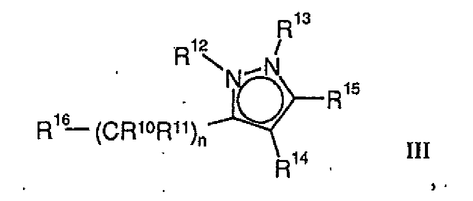 dans lequel R¹⁰ à R¹⁶ et n sont tels que définis dans la revendication 1 et R¹⁶ représente un groupe -OH, -Cl, - Br, -I ou un autre groupe partant, pour obtenir un composé de formule 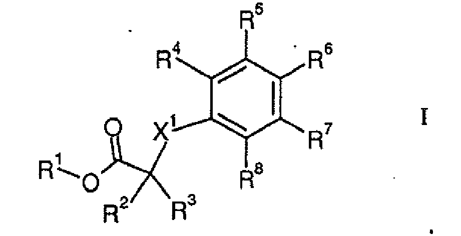 dans laquelle R¹ représente un groupe alkyle en C₁ à C₇ et X¹, R² à R⁸ sont tels que définis dans la revendication 1,
et facultativement, l'hydrolyse du groupe ester pour obtenir un composé de formule I dans laquelle R¹ représente un atome d'hydrogène.

22. Compositions pharmaceutiques comprenant un composé suivant l'une quelconque des revendications 1 à 20 ainsi qu'un support et/ou adjuvant pharmaceutiquement acceptables.

23. Compositions pharmaceutiques suivant la revendication 22, pour le traitement et/ou la prévention de maladies qui sont modulées par des agonistes de PPARδ et/ou PPARα.

24. Composés suivant l'une quelconque des revendications 1 à 20, destinés à être utilisés comme substances thérapeutiquement actives.

25. Composés suivant l'une quelconque des revendications 1 à 20, destinés à être utilisés comme substances thérapeutiquement actives pour le traitement et/ou la prévention de maladies qui sont modulées par des agonistes de PPARδ et/ou PPARα.

26. Utilisation de composés suivant l'une quelconque des revendications 1 à 20 pour la préparation de médicaments destinés au traitement et/ou à la prévention de maladies qui sont modulées par des agonistes de PPARδ et/ou PPARα.

27. Utilisation suivant la revendication 26 pour le traitement et/ou la prévention du diabète, du diabète sucré non insulino-dépendant, de taux élevés de lipides et de cholestérol, en particulier d'un taux bas de cholestérol-HDL, d'un taux élevé de cholestérol-LDL, ou de taux élevés de triglycérides, de maladies athérosclérotiques, du syndrome métabolique, du syndrome X, de l'obésité, d'une pression sanguine élevée, d'un dysfonctionnement endothélial, d'un état procoagulant, de la dyslipidémie, du syndrome d'ovaire polykystique, de maladies inflammatoires et de maladies prolifératives.

28. Utilisation suivant la revendication 27 pour le traitement et/ou la prévention de taux bas de cholestérol-HDL, de taux élevés de cholestérol-LDL, de taux élevés de triglycérides, du syndrome métabolique et du syndrome X.
